# EUROPEAN PATENT APPLICATION

(11) **EP 4 424 325 A1**
(43) Date of publication of application: **04.09.2024**
(21) Application number: 22895690.0
(22) Date of filing: 18.11.2022
(51) Int. Cl.: A61K 39/395

(54) **PEPTIDE HAVING AFFINITY FOR HUMAN TRANSFERRIN RECEPTOR**

(30) Priority: 19.11.2021 JP 2021189037; 18.02.2022 JP 2022023656; 18.03.2022 JP 2022043509
(71) Applicant: JCR Pharmaceuticals Co., Ltd., Ashiya-shi, Hyogo 659-0021 (JP)
(72) Inventor: ONOUCHI Takashi, Kobe-shi, Hyogo 651-2241 (JP); TAKAHASHI, Kenichi, Kobe-shi, Hyogo 651-2241 (JP)
(74) Representative: Boult Wade Tennant LLP
(86) International application number: PCT/JP2022/042785
(87) International publication number: WO 2023/090409

(57) **Abstract**

The present invention relates, in one embodiment thereof, to a peptide having affinity for human transferrin receptor, which can be used for binding to any of compounds (a protein, a nucleic acid, a low molecular weight compound, and the like) that should be caused to function in the central nervous system (CNS), in order to allow the compound to pass through the blood-brain barrier, and the use of the peptide. The peptide is, for example, a peptide having the amino acid sequence selected from the group consisting of amino acid sequences set forth in SEQ ID NOs:3 to 7 and 55, or a mutant thereof.

## Description

### Technical Field

The present invention relates, in one embodiment thereof, to a peptide having affinity for human transferrin receptor, the peptide being used by binding to a compound (a protein, a nucleic acid, a low molecular weight compound, or the like) that should be caused to function in the central nervous system (CNS), in order to allow the compound to pass through the blood-brain barrier, and a method of using the peptide.

### Background Art

In capillaries that supply blood to most tissues of the brain except for several regions including the circumventricular organs (pineal gland, pituitary gland, area postrema, and the like), endothelial cells that make up the endothelium of the capillaries are tightly held together by strong intercellular junctions, unlike capillaries present in other tissues such as muscles. Therefore, passive transport of substances from the blood to the brain is hindered, and although there are exceptions, it is difficult for substances other than highly lipid-soluble substances or substances that have a small molecular weight (200 to 500 daltons or less) and are electrically neutral near the physiological pH, to migrate from the capillaries to the brain parenchyma. Such a mechanism that restricts material exchange between the blood and the brain tissue fluid through the intracerebral capillary endothelium is called the blood brain barrier (BBB). Further, the blood brain barrier also restricts material exchange between the blood and the tissue fluids of the brain as well as the central nervous system including brain and spinal cord.

Due to the existence of the blood brain barrier, most of the cells in the central nervous system maintain their biochemical homeostasis without being affected by fluctuations in the concentration of substances such as hormones and cytokines in the blood. However, the existence of the blood brain barrier poses problems upon drug development. For example, with regard to mucopolysaccharidosis type II (Hunter syndrome), which is a genetic metabolic disease caused by a deficiency of iduronate-2-sulfatase, enzyme replacement therapy of intravenously administering recombinant iduronate-2-sulfatase is carried out; however, in connection with the notable abnormalities in the central nervous system (CNS) recognized in Hunter syndrome, the enzyme replacement therapy is less effective because iduronate-2-sulfatase cannot cross the blood brain barrier. In addition, for diseases in the central nervous system such as Alzheimer's disease and brain tumors, it is necessary to cause drugs to reach the central nervous system; however, drugs that cannot pass through the blood brain barrier cannot exert drug efficacy.

Various methods have been developed for allowing macromolecular substances such as proteins that should be caused to act on the central nervous system, to pass through the blood brain barrier. Regarding such methods, various methods for modifying a macromolecular substance so as to have affinity for membrane proteins present on the endothelial cells of the intracerebral capillaries have been reported. Examples of membrane proteins present on the endothelial cells of the intracerebral capillaries include receptors for compounds such as insulin, transferrin, insulin-like growth factors (IGF-I and IGF-II), LDL, and leptin. Among these, it has been reported that an antibody to transferrin receptor can pass through the blood brain barrier and migrate to the brain parenchyma, and by binding the antibody to a drug that should be caused to act on the central nervous system, the antibody can deliver the drug into the brain (Patent Literatures 1 to 8).

In addition, a Hunter syndrome therapeutic agent (IZCARGO (registered trademark)) in which iduronate-2-sulfatase is bound to an anti-transferrin receptor antibody, and which can pass through the blood brain barrier and exert drug efficacy in the central nervous system, was marketed in year 2021 (Non Patent Literature 1).

### Citation List

### Patent Literature

Patent Literature 1: US 2007/0082380
Patent Literature 2: US 2008/0152645
Patent Literature 3: US 2009/0053219
Patent Literature 4: US 2011/0110935
Patent Literature 5: US 2018/0171012
Patent Literature 6: US 2018/0179291
Patent Literature 7: US 2020/0317798
Patent Literature 8: US 2020/0384061

### Non Patent Literature

Non Patent Literature 1: Package insert "IZCARGO for Intravenous Drip Infusion 10 mg", March 2021 (1st Edition)

### Summary of Invention

### Technical Problem

Under the above-described circumstances, an object of the present invention is to provide a peptide having affinity for a human transferrin receptor, which can be used by binding to a compound (a protein, a nucleic acid, a low molecular weight compound, or the like) that should be caused to function in the central nervous system (CNS) so as to enable such a compound to pass through the blood brain barrier, and a method of using the peptide.

### Solution to Problem

In research aimed at the above-described object, the inventors of the present invention conducted extensive investigations, and as a result, the inventors found that a peptide having affinity for human transferrin receptor (hTfR), the peptide being obtained by a production method described in detail in the present specification and containing a variable region of an anti-hTfR heavy chain antibody that specifically recognizes the extracellular region of hTfR (anti-hTfR heavy chain antibody variable region peptide), efficiently passes through the blood brain barrier, thus completing the present invention. That is, the present invention includes the following. Incidentally, in the inventions described in the following 1 to 104, a human transferrin receptor affinity peptide can be replaced with a peptide containing a variable region of a heavy chain antibody, and a peptide can also be replaced with a protein.
1. A peptide having affinity for human transferrin receptor (human transferrin receptor affinity peptide), the peptide comprising a variable region of a heavy chain antibody having three complementarity-determining regions, CDR1, CDR2, and CDR3, and being selected from the group consisting of the following (1) to (5):
   (1) a peptide in which the amino acid sequence of CDR1 is the amino acid sequence of SEQ ID NO:8 or SEQ ID NO:9, the amino acid sequence of CDR2 is the amino acid sequence of SEQ ID NO: 10 or SEQ ID NO: 11, and the amino acid sequence of CDR3 is the amino acid sequence of SEQ ID NO: 12 or SEQ ID NO: 13;
   (2) a peptide in which the amino acid sequence of CDR1 is the amino acid sequence of SEQ ID NO: 14 or SEQ ID NO: 15, the amino acid sequence of CDR2 is the amino acid sequence of SEQ ID NO: 16 or SEQ ID NO: 17, and the amino acid sequence of CDR3 is the amino acid sequence of SEQ ID NO: 18 or SEQ ID NO: 19;
   (3) a peptide in which the amino acid sequence of CDR1 is the amino acid sequence of SEQ ID NO:20 or SEQ ID NO:21, the amino acid sequence of CDR2 is the amino acid sequence of SEQ ID NO:22 or SEQ ID NO:23, and the amino acid sequence of CDR3 is the amino acid sequence of SEQ ID NO:24 or SEQ ID NO:25;
   (4) a peptide in which the amino acid sequence of CDR1 is the amino acid sequence of SEQ ID NO:26 or SEQ ID NO:27, the amino acid sequence of CDR2 is the amino acid sequence of SEQ ID NO:28 or SEQ ID NO:29, and the amino acid sequence of CDR3 is the amino acid sequence of SEQ ID NO:30 or SEQ ID NO:31; and
   (5) a peptide in which the amino acid sequence of CDR1 is the amino acid sequence of SEQ ID NO:20 or SEQ ID NO:21, the amino acid sequence of CDR2 is the amino acid sequence of SEQ ID NO:56 or SEQ ID NO:57, and the amino acid sequence of CDR3 is the amino acid sequence of SEQ ID NO:24 or SEQ ID NO:25.
2. A human transferrin receptor affinity peptide selected from the group consisting of the following (1) to (6):
   (1) a peptide having the amino acid sequence identity of 80% or higher to the amino acid sequence of SEQ ID NO:3 and having three complementarity-determining regions, CDR1, CDR2, and CDR3, having the amino acid sequences shown in (1) of claim 1;
   (2) a peptide having the amino acid sequence identity of 80% or higher to the amino acid sequence of SEQ ID NO:4 and having three complementarity-determining regions, CDR1, CDR2, and CDR3, having the amino acid sequences shown in (2) of claim 1;
   (3) a peptide having the amino acid sequence identity of 80% or higher to the amino acid sequence of SEQ ID NO:5 and having three complementarity-determining regions, CDR1, CDR2, and CDR3, having the amino acid sequences shown in (3) of claim 1;
   (4) a peptide having the amino acid sequence identity of 80% or higher to the amino acid sequence of SEQ ID NO:6 and having three complementarity-determining regions, CDR1, CDR2, and CDR3, having the amino acid sequences shown in (4) of claim 1;
   (5) a peptide having the amino acid sequence identity of 80% or higher to the amino acid sequence of SEQ ID NO:7 and having three complementarity-determining regions, CDR1, CDR2, and CDR3, having the amino acid sequences shown in (3) of claim 1; and
   (6) a peptide having the amino acid sequence identity of 80% or higher to the amino acid sequence of SEQ ID NO:55 and having three complementarity-determining regions, CDR1, CDR2, and CDR3, having the amino acid sequences shown in (5) of claim 1.
3. The human transferrin receptor affinity peptide according to 2 above, wherein the amino acid sequence identity is 85% or higher.
4. The human transferrin receptor affinity peptide according to 2 above, wherein the amino acid sequence identity is 90% or higher.
5. The human transferrin receptor affinity peptide according to 2 above, wherein the amino acid sequence identity is 95% or higher.
6. The human transferrin receptor affinity peptide according to 1 above, wherein the human transferrin receptor affinity peptide contains the amino acid sequence selected from the group consisting of the following (1) to (6):
   (1) the amino acid sequence in which 1 to 10 amino acids are substituted, deleted, or added relative to the amino acid sequence of SEQ ID NO:3, in which the amino acid sequence of CDR1 is the amino acid sequence of SEQ ID NO:8 or SEQ ID NO:9, the amino acid sequence of CDR2 is the amino acid sequence of SEQ ID NO: 10 or SEQ ID NO: 11, and the amino acid sequence of CDR3 is the amino acid sequence of SEQ ID NO: 12 or SEQ ID NO: 13;
   (2) the amino acid sequence in which 1 to 10 amino acids are substituted, deleted, or added relative to the amino acid sequence of SEQ ID NO:4, in which the amino acid sequence of CDR1 is the amino acid sequence of SEQ ID NO: 14 or SEQ ID NO: 15, the amino acid sequence of CDR2 is the amino acid sequence of SEQ ID NO: 16 or SEQ ID NO: 17, and the amino acid sequence of CDR3 is the amino acid sequence of SEQ ID NO: 18 or SEQ ID NO: 19;
   (3) the amino acid sequence in which 1 to 10 amino acids are substituted, deleted, or added relative to the amino acid sequence of SEQ ID NO:5, in which the amino acid sequence of CDR1 is the amino acid sequence of SEQ ID NO:20 or SEQ ID NO:21, the amino acid sequence of CDR2 is the amino acid sequence of SEQ ID NO:22 or SEQ ID NO:23, and the amino acid sequence of CDR3 is the amino acid sequence of SEQ ID NO:24 or SEQ ID NO:25;
   (4) the amino acid sequence in which 1 to 10 amino acids are substituted, deleted, or added relative to the amino acid sequence of SEQ ID NO:6, in which the amino acid sequence of CDR1 is the amino acid sequence of SEQ ID NO:26 or SEQ ID NO:27, the amino acid sequence of CDR2 is the amino acid sequence of SEQ ID NO:28 or SEQ ID NO:29, and the amino acid sequence of CDR3 is the amino acid sequence of SEQ ID NO:30 or SEQ ID NO:31;
   (5) the amino acid sequence in which 1 to 10 amino acids are substituted, deleted, or added relative to the amino acid sequence of SEQ ID NO:7, in which the amino acid sequence of CDR1 is the amino acid sequence of SEQ ID NO:20 or SEQ ID NO:21, the amino acid sequence of CDR2 is the amino acid sequence of SEQ ID NO:22 or SEQ ID NO:23, and the amino acid sequence of CDR3 is the amino acid sequence of SEQ ID NO:24 or SEQ ID NO:25; and
   (6) the amino acid sequence in which 1 to 10 amino acids are substituted, deleted, or added relative to the amino acid sequence of SEQ ID NO:55, in which the amino acid sequence of CDR1 is the amino acid sequence of SEQ ID NO:20 or SEQ ID NO:21, the amino acid sequence of CDR2 is the amino acid sequence of SEQ ID NO:55 or SEQ ID NO:56, and the amino acid sequence of CDR3 is the amino acid sequence of SEQ ID NO:24 or SEQ ID NO:25.
7. The human transferrin receptor affinity peptide according to 5 above, wherein the number of substituted, deleted, or added amino acids is 1 to 5.
8. The human transferrin receptor affinity peptide according to 5 above, wherein the number of substituted, deleted, or added amino acids is 1 to 3.
9. The human transferrin receptor affinity peptide according to any one of 1 to 8 above, wherein the human transferrin receptor affinity peptide has affinity for both an extracellular region of human transferrin receptor and an extracellular region of monkey transferrin receptor.
10. The human transferrin receptor affinity peptide according to 9 above, wherein a dissociation constant with the extracellular region of human transferrin receptor is 5×10⁻⁹ to 1×10⁻⁷.
11. The human transferrin receptor affinity peptide according to 9 above, wherein a dissociation constant with the extracellular region of human transferrin receptor is 8×10⁻⁹ to 2×10⁻⁸.
12. The human transferrin receptor affinity peptide according to 9 above, wherein when the value of the dissociation constant with the extracellular region of human transferrin receptor is taken as 1, the value of a dissociation constant with monkey transferrin receptor is 0.5 to 2.5.
13. The human transferrin receptor affinity peptide according to 9 above, wherein when the value of the dissociation constant with the extracellular region of human transferrin receptor is taken as 1, the value of the dissociation constant with the monkey transferrin receptor is 0.7 to 1.2.
14. A heavy chain antibody variable region peptide in which a plurality of the human transferrin receptor affinity peptide according to any one of 1 to 13 above are bound, the heavy chain antibody variable region peptide being selected from the group consisting of the following (1) to (3):
   (1) a heavy chain antibody variable region peptide in which 2 to 10 of the human transferrin receptor affinity peptide according to 1 to 12 above are linked directly or via a linker;
   (2) a heavy chain antibody variable region peptide in which 2 or 3 of the human transferrin receptor affinity peptide according to 1 to 12 above are linked directly or via a linker; and
   (3) a heavy chain antibody variable region peptide in which two of the human transferrin receptor affinity peptide according to 1 to 12 above are linked directly or via a linker.
15. The human transferrin receptor affinity peptide according to 14 above, wherein the linker for linking a plurality of the human transferrin receptor affinity peptide is a peptide linker consisting of 1 to 50 amino acids.
16. The human transferrin receptor affinity peptide according to 14 above, wherein the amino acid sequence of the peptide linker for linking a plurality of the human transferrin receptor affinity peptide is selected from the group consisting of a single amino acid, the amino acid sequence Gly-Ser, the amino acid sequence Ser-Ser, the amino acid sequence Gly-Gly-Ser, the amino acid sequence Gly-Gly-Gly, and the amino acid sequences of SEQ ID NO:32, SEQ ID NO:33, SEQ ID NO:34, SEQ ID NO:35, SEQ ID NO:36, SEQ ID NO:58, and SEQ ID NO:69.
17. The human transferrin receptor affinity peptide according to 14 above, wherein the amino acid sequence of the peptide linker for linking the heavy chain antibody variable region peptides is the amino acid sequence in which 2 to 10 of the amino acid sequences selected from the group consisting of a single amino acid, the amino acid sequence Gly-Ser, the amino acid sequence Ser-Ser, the amino acid sequence Gly-Gly-Ser, the amino acid sequence Gly-Gly-Gly, and the amino acid sequence of SEQ ID NO:32, SEQ ID NO:33, SEQ ID NO:34, SEQ ID NO:58, and SEQ ID NO:69, are linked in tandem.
18. A human transferrin receptor affinity peptide-drug complex, wherein the human transferrin receptor affinity peptide according to any one of 1 to 17 above is linked to a drug.
19. The complex according to 18 above, wherein the drug is any one of a different protein (A), a nucleic acid, or a low molecular weight compound.
20. A fusion protein comprising:
   the human transferrin receptor affinity peptide according to any one of 1 to 17 above and
   a different protein (A).
21. The fusion protein according to 20 above, wherein the human transferrin receptor affinity peptide is bound to the C-terminus of the different protein (A) directly or via a linker.
22. The fusion protein according to 20 above, wherein the human transferrin receptor affinity peptide is bound to the N-terminus of the different protein (A) directly or via a linker.
23. The fusion protein according to 21 or 22 above, wherein the linker is a peptide linker consisting of 1 to 50 amino acids.
24. The fusion protein according to 23 above, wherein the amino acid sequence of the peptide linker is selected from the group consisting of a single amino acid, the amino acid sequence Gly-Ser, the amino acid sequence Ser-Ser, the amino acid sequence Gly-Gly-Ser, the amino acid sequence Gly-Gly-Gly, and the amino acid sequences of SEQ ID NO:32, SEQ ID NO:33, SEQ ID NO:34, SEQ ID NO:35, SEQ ID NO:36, SEQ ID NO:58, and SEQ ID NO:69.
25. The fusion protein according to 23 above, wherein the amino acid sequence of the peptide linker is the amino acid sequence in which 2 to 10 of the amino acid sequences selected from the group consisting of a single amino acid, the amino acid sequence Gly-Ser, the amino acid sequence Ser-Ser, the amino acid sequence Gly-Gly-Ser, the amino acid sequence Gly-Gly-Gly, each of the amino acid sequences of SEQ ID NO:32, SEQ ID NO:33, and SEQ ID NO:34, and the amino acid sequences of SEQ ID NO:58 and SEQ ID NO:69, are linked in tandem.
26. The fusion protein according to any one of 20 to 25 above, wherein the different protein (A) is a protein originating from human.
27. The fusion protein according to any one of 20 to 26 above, wherein the different protein (A) is a cytokine, a growth factor, or an antibody medicine.
28. The fusion protein according to any one of 20 to 26 above, wherein the different protein (A) is selected from the group consisting of a brain-derived neurotrophic factor (BDNF), a nerve growth factor (NGF), a lysosomal enzyme, a ciliary neurotrophic factor (CNTF), a glial cell-derived neurotrophic factor (GDNF), a neurotrophin 3, a neurotrophin 4/5, a neurotrophin 6, a neuregulin 1, an erythropoietin, a darbepoietin, an activin, a basic fibroblast growth factor (bFGF), a fibroblast growth factor 2 (FGF2), an epidermal growth factor (EGF), a vascular endothelial growth factor (VEGF), an interferon α, an interferon β, an interferon γ, an interleukin 6, a granulocyte-macrophage colony-stimulating factor (GM-CSF), a granulocyte-colony-stimulating factor (G-CSF), a macrophage colony-stimulating factor (M-CSF), a tumor necrosis factor-a receptor (TNF-α receptor), a PD-1 ligand, a PD-L1, a PD-L2, an enzyme having an activity of degrading beta-amyloid, an anti-beta-amyloid antibody, an anti-BACE antibody, an anti-EGFR antibody, an anti-PD-1 antibody, an anti-PD-L1 antibody, an anti-PD-L2 antibody, an anti-HER2 antibody, an anti-TNF-α antibody, and an anti-CTLA-4 antibody.
29. The fusion protein according to any one of 20 to 26 above, wherein the different protein (A) is a lysosomal enzyme, and the lysosomal enzyme is selected from the group consisting of α-L-iduronidase, iduronate-2-sulfatase, acidic α-glucosidase, glucocerebrosidase, β-galactosidase, GM2 activator protein, β-hexosaminidase A, β-hexosaminidase B, N-acetylglucosamine-1-phosphotransferase, α-mannosidase, β-mannosidase, galactosylceramidase, saposin C, allylsulfatase A, α-L-fucosidase, aspartylglucosaminidase, α-N-acetylgalactosaminidase, acidic sphingomyelinase, α-galactosidase A, β-glucuronidase, heparan-N-sulfatase, α-N-acetylglucosaminidase, acetyl CoA α-glucosaminide N-acetyltransferase, N-acetylglucosamine-6-sulfate sulfatase, acidic ceramidase, amylo-1,6-glucosidase, sialidase, palmitoyl protein thioesterase-1, tripeptidyl peptidase-1, hyaluronidase-1, CLN1, and CLN2.
30. The fusion protein according to any one of 20 to 29 above, wherein serum albumin is bound to the fusion protein.
31. The fusion protein according to any one of 20 to 29 above, wherein a human IgG Fc region or a portion thereof is bound to the fusion protein.
32. A nucleic acid encoding the human transferrin receptor affinity peptide according to any one of 1 to 17 above.
33. A nucleic acid encoding the fusion protein according to any one of 20 to 31 above.
34. An expression vector comprising the nucleic acid according to 32 or 33 above incorporated therein.
35. A cell transformed with the expression vector according to 34 above.
36. The cell according to 35 above, wherein the cell is derived from a mammal.
37. A human transferrin receptor affinity peptide comprising the amino acid sequence in which 1 to 10 amino acids are substituted, deleted, or added relative to the amino acid sequence selected from the group consisting of SEQ ID NO:3, SEQ ID NO:4, SEQ ID NO:5, SEQ ID NO:6, SEQ ID NO:7, and SEQ ID NO:55.
38. The human transferrin receptor affinity peptide according to 37 above, wherein the number of substituted, deleted, or added amino acids is 1 to 5.
39. The human transferrin receptor affinity peptide according to 37 above, wherein the number of substituted, deleted, or added amino acids is 1 to 3.
40. The human transferrin receptor affinity peptide according to 1 above, comprising the amino acid sequence selected from the group consisting of SEQ ID NO:3, SEQ ID NO:4, SEQ ID NO:5, SEQ ID NO:6, SEQ ID NO:8, and SEQ ID NO:55.
41. The human transferrin receptor affinity peptide according to 1 above, wherein the human transferrin receptor affinity peptide is selected from the group consisting of the following (1) to (6):
   (1) the amino acid sequence having the amino acid sequence identity of 80% or higher to the amino acid sequence of SEQ ID NO:3, in which the amino acid sequence of CDR1 is the amino acid sequence of SEQ ID NO:8 or SEQ ID NO:9, the amino acid sequence of CDR2 is the amino acid sequence of SEQ ID NO:10 or SEQ ID NO:11, and the amino acid sequence of CDR3 is the amino acid sequence of SEQ ID NO:12 or SEQ ID NO:13;
   (2) the amino acid sequence having the amino acid sequence identity of 80% or higher to the amino acid sequence of SEQ ID NO:4, in which the amino acid sequence of CDR1 is the amino acid sequence of SEQ ID NO:14 or SEQ ID NO:15, the amino acid sequence of CDR2 is the amino acid sequence of SEQ ID NO:16 or SEQ ID NO:17, and the amino acid sequence of CDR3 is the amino acid sequence of SEQ ID NO:18 or SEQ ID NO:19;
   (3) the amino acid sequence having the amino acid sequence identity of 80% or higher to the amino acid sequence of SEQ ID NO:5, in which the amino acid sequence of CDR1 is the amino acid sequence of SEQ ID NO:20 or SEQ ID NO:21, the amino acid sequence of CDR2 is the amino acid sequence of SEQ ID NO:22 or SEQ ID NO:23, and the amino acid sequence of CDR3 is the amino acid sequence of SEQ ID NO:24 or SEQ ID NO:25;
   (4) the amino acid sequence having the amino acid sequence identity of 80% or higher to the amino acid sequence of SEQ ID NO:6, in which the amino acid sequence of CDR1 is the amino acid sequence of SEQ ID NO:26 or SEQ ID NO:27, the amino acid sequence of CDR2 is the amino acid sequence of SEQ ID NO:28 or SEQ ID NO:29, and the amino acid sequence of CDR3 is the amino acid sequence of SEQ ID NO:30 or SEQ ID NO:31;
   (5) the amino acid sequence having the amino acid sequence identity of 80% or higher to the amino acid sequence of SEQ ID NO:7, in which the amino acid sequence of CDR1 is the amino acid sequence of SEQ ID NO:20 or SEQ ID NO:21, the amino acid sequence of CDR2 is the amino acid sequence of SEQ ID NO:22 or SEQ ID NO:23, and the amino acid sequence of CDR3 is the amino acid sequence of SEQ ID NO:24 or SEQ ID NO:25; and
   (6) the amino acid sequence having the amino acid sequence identity of 80% or higher to the amino acid sequence of SEQ ID NO:55, in which the amino acid sequence of CDR1 is the amino acid sequence of SEQ ID NO:20 or SEQ ID NO:21, the amino acid sequence of CDR2 is the amino acid sequence of SEQ ID NO:56 or SEQ ID NO:57, and the amino acid sequence of CDR3 is the amino acid sequence of SEQ ID NO:24 or SEQ ID NO:25.
42. The human transferrin receptor affinity peptide according to 41 above, wherein the amino acid sequence identity is 85% or higher.
43. The human transferrin receptor affinity peptide according to 41 above, wherein the amino acid sequence identity is 90% or higher.
44. The human transferrin receptor affinity peptide according to 41 above, wherein the amino acid sequence identity is 95% or higher.
45. A heavy chain antibody-drug complex, wherein a heavy chain antibody targeting a molecule present on the surface of a vascular endothelial cell as an antigen is linked to a drug.
46. The complex according to 45 above, wherein the drug is any one of a different protein (A), a nucleic acid, or a low molecular weight compound.
47. A fusion protein comprising:
   a heavy chain antibody targeting a molecule present on the surface of a vascular endothelial cell as an antigen; and
   a different protein (A),
   wherein the heavy chain antibody is bound to the C-terminus of the different protein (A) directly or via a linker, or the heavy chain antibody is bound to the N-terminus of the different protein (A) directly or via a linker.
48. The fusion protein according to 47 above, wherein the linker is a peptide linker consisting of 1 to 50 amino acids.
49. The fusion protein according to 47 above, wherein the amino acid sequence of the peptide linker is selected from the group consisting of a single amino acid, the amino acid sequence Gly-Ser, the amino acid sequence Ser-Ser, the amino acid sequence Gly-Gly-Ser, the amino acid sequence Gly-Gly-Gly, each of the amino acid sequences of SEQ ID NO: 11, SEQ ID NO: 12, SEQ ID NO: 13, SEQ ID NO: 14, and SEQ ID NO: 15, and the amino acid sequence consisting of three consecutive sequences of the amino acid sequence of SEQ ID NO: 11.
50. The fusion protein according to 47 above, wherein the amino acid sequence of the peptide linker is the amino acid sequence in which 2 to 10 of the amino acid sequence selected from the group consisting of a single amino acid, the amino acid sequence Gly-Ser, the amino acid sequence Ser-Ser, the amino acid sequence Gly-Gly-Ser, the amino acid sequence Gly-Gly-Gly, each of the amino acid sequences of SEQ ID NO:11, SEQ ID NO: 12, and SEQ ID NO:13, and the amino acid sequence consisting of three consecutive sequences of the amino acid sequence of SEQ ID NO: 11, are linked in tandem.
51. The complex or fusion protein according to any one of 46 to 50 above, wherein the different protein (A) is a protein originating from human.
52. The complex or fusion protein according to any one of 46 to 51 above, wherein the different protein (A) is a cytokine, a growth factor, or an antibody medicine.
53. The complex or fusion protein according to any one of 46 to 51 above, wherein the different protein (A) is selected from the group consisting of a brain-derived neurotrophic factor (BDNF), a nerve growth factor (NGF), a lysosomal enzyme, a ciliary neurotrophic factor (CNTF), a glial cell-derived neurotrophic factor (GDNF), a neurotrophin 3, a neurotrophin 4/5, a neurotrophin 6, a neuregulin 1, an erythropoietin, a darbepoietin, an activin, a basic fibroblast growth factor (bFGF), a fibroblast growth factor 2 (FGF2), an epidermal growth factor (EGF), a vascular endothelial growth factor (VEGF), an interferon α, an interferon β, an interferon γ, an interleukin 6, a granulocyte-macrophage colony-stimulating factor (GM-CSF), a granulocyte-colony-stimulating factor (G-CSF), a macrophage colony-stimulating factor (M-CSF), a tumor necrosis factor-a receptor (TNF-α receptor), a PD-1 ligand, a PD-L1, a PD-L2, an enzyme having an activity of degrading beta-amyloid, an anti-beta-amyloid antibody, an anti-BACE antibody, an anti-EGFR antibody, an anti-PD-1 antibody, an anti-PD-L1 antibody, an anti-PD-L2 antibody, an anti-HER2 antibody, an anti-TNF-α antibody, and an anti-CTLA-4 antibody.
54. The complex or fusion protein according to any one of 46 to 51 above, wherein the different protein (A) is a lysosomal enzyme, and the lysosomal enzyme is selected from the group consisting of α-L-iduronidase, iduronate-2-sulfatase, acidic α-glucosidase, glucocerebrosidase, β-galactosidase, GM2-activating protein, β-hexosaminidase A, β-hexosaminidase B, N-acetylglucosamine-1-phosphotransferase, α-mannosidase, β-mannosidase, galactosylceramidase, saposin C, arylsulfatase A, α-L-fucosidase, aspartylglucosaminidase, α-N-acetylgalactosaminidase, acidic sphingomyelinase, α-galactosidase A, β-glucuronidase, heparan-N-sulfatase, α-N-acetylglucosaminidase, acetyl CoA α-glucosaminide N-acetyltransferase, N-acetylglucosamine-6-sulfate sulfatase, acidic ceramidase, amylo-1,6-glucosidase, sialidase, palmitoyl protein thioesterase-1, tripeptidyl peptidase-1, hyaluronidase-1, CLN1, and CLN2.
55. The fusion protein according to any one of 46 to 54 above, wherein serum albumin is further bound to the fusion protein.
56. The fusion protein according to any one of 46 to 54 above, wherein a human IgG Fc region or a portion thereof is further bound to the fusion protein.
57. The complex or fusion protein according to any one of 45 to 56 above, wherein the vascular endothelial cell is a cerebrovascular endothelial cell.
58. The complex or fusion protein according to any one of 45 to 57 above, wherein the molecule present on the surface of the vascular endothelial cell is a molecule selected from the group consisting of transferrin receptor (TfR), insulin receptor, leptin receptor, a lipoprotein receptor, an IGF receptor, OATP-F, an organic anionic transporter, MCT-8, and a monocarboxylic acid transporter, and particularly the molecule is a molecule originating from human.
59. The complex or fusion protein according to any one of 45 to 57 above, wherein the molecule present on the surface of the vascular endothelial cell is transferrin receptor (TfR), and particularly the molecule is a transferrin receptor originating from human.
60. The complex according to 45 or 46 above, wherein the drug is bound to the heavy chain antibody via a linker selected from the group consisting of polyethylene glycol, polypropylene glycol, a copolymer of ethylene glycol and propylene glycol, polyoxyethylated polyol, polyvinyl alcohol, a polysaccharide, dextran, polyvinyl ether, a biodegradable polymer, a lipid polymer, a chitin, hyaluronic acid, biotin-streptavidin, and derivatives thereof. The antibody is bound to the human lysosomal enzyme via a linker selected from the group consisting of polyethylene glycol, polypropylene glycol, a copolymer of ethylene glycol and propylene glycol, polyoxyethylated polyol, polyvinyl alcohol, a polysaccharide, dextran, polyvinyl ether, a biodegradable polymer, a lipid polymer, a chitin, hyaluronic acid, biotin-streptavidin, and derivatives thereof.
61. The complex or fusion protein according to 45 to 60 above, wherein the heavy chain antibody is the entirety or a part of a heavy chain antibody derived from an animal of the family Camelidae or an immunoglobulin neoantigen receptor derived from cartilaginous fish.
62. The complex or fusion protein according to 61 above, wherein the heavy chain antibody is a single heavy chain antibody.
63. The complex or fusion protein according to 61 or 62 above, wherein the heavy chain antibody is selected from the group consisting of the following (1) to (4):
   (1) a heavy chain antibody composed only of the entirety or a part of a variable region of the heavy chain;
   (2) a heavy chain antibody composed of the entirety or a part of the variable region and a part of a hinge region of the heavy chain;
   (3) a heavy chain antibody composed of the entirety of the variable region and the hinge region of the heavy chain; and
   (4) a heavy chain antibody including a part of a constant region in addition to the variable region and the hinge region of the heavy chain.
64. A nucleic acid encoding the fusion protein according to any one of 47 to 59 and 61 to 63 above.
65. An expression vector comprising the nucleic acid according to 64 above incorporated therein.
66. A cell transformed with the expression vector according to 65 above.
67. The cell according to 66 above, wherein the cell is derived from a mammal.
68. A pharmaceutical composition comprising the complex or fusion protein according to 45 to 63 above as an active ingredient, particularly a pharmaceutical composition in which the active ingredient passes through the blood brain barrier and exerts drug efficacy on the central nerves.
69. A heavy chain antibody variable region peptide having affinity for human transferrin receptor, the heavy chain antibody variable region peptide having three complementarity-determining regions, CDR1, CDR2, and CDR3, and being selected from the group consisting of the following (1) to (6):
   (1) a heavy chain antibody variable region peptide in which the amino acid sequence of CDR1 is the amino acid sequence of SEQ ID NO:8 or SEQ ID NO:9, the amino acid sequence of CDR2 is the amino acid sequence of SEQ ID NO: 10 or SEQ ID NO: 11, and the amino acid sequence of CDR3 is the amino acid sequence of SEQ ID NO: 12 or SEQ ID NO: 13, where each of the amino acid sequences of CDR1, CDR2, and CDR3 may be any sequence; however, preferably, the amino acid sequence of CDR1 is the amino acid sequence of SEQ ID NO:8, the amino acid sequence of CDR2 is the amino acid sequence of SEQ ID NO: 10, and the amino acid sequence of CDR3 is the amino acid sequence of SEQ ID NO: 12, or the amino acid sequence of CDR1 is the amino acid sequence of SEQ ID NO:9, the amino acid sequence of CDR2 is the amino acid sequence of SEQ ID NO: 11, and the amino acid sequence of CDR3 is the amino acid sequence of SEQ ID NO: 13;
   (2) a heavy chain antibody variable region peptide in which the amino acid sequence of CDR1 is the amino acid sequence of SEQ ID NO: 14 or SEQ ID NO: 15, the amino acid sequence of CDR2 is the amino acid sequence of SEQ ID NO: 16 or SEQ ID NO: 17, and the amino acid sequence of CDR3 is the amino acid sequence of SEQ ID NO: 18 or SEQ ID NO: 19, where each of the amino acid sequences of CDR1, CDR2, and CDR3 may be any sequence; however, preferably, the amino acid sequence of CDR1 is the amino acid sequence of SEQ ID NO: 14, the amino acid sequence of CDR2 is the amino acid sequence of SEQ ID NO: 16, and the amino acid sequence of CDR3 is the amino acid sequence of SEQ ID NO: 18, or the amino acid sequence of CDR1 is the amino acid sequence of SEQ ID NO: 15 the amino acid sequence of CDR2 is the amino acid sequence of SEQ ID NO: 17, and the amino acid sequence of CDR3 is the amino acid sequence of SEQ ID NO: 19;
   (3) a heavy chain antibody variable region peptide in which the amino acid sequence of CDR1 is the amino acid sequence of SEQ ID NO:20 or SEQ ID NO:21, the amino acid sequence of CDR2 is the amino acid sequence of SEQ ID NO:22 or SEQ ID NO:23, and the amino acid sequence of CDR3 is the amino acid sequence of SEQ ID NO:24 or SEQ ID NO:25, where each of the amino acid sequences of CDR1, CDR2, and CDR3 may be any sequence; however, preferably, the amino acid sequence of CDR1 is the amino acid sequence of SEQ ID NO:20, the amino acid sequence of CDR2 is the amino acid sequence of SEQ ID NO:22, and the amino acid sequence of CDR3 is the amino acid sequence of SEQ ID NO:24, or the amino acid sequence of CDR1 is the amino acid sequence of SEQ ID NO:21 the amino acid sequence of CDR2 is the amino acid sequence of SEQ ID NO:23, and the amino acid sequence of CDR3 is the amino acid sequence of SEQ ID NO:25;
   (4) a heavy chain antibody variable region peptide in which the amino acid sequence of CDR1 is the amino acid sequence of SEQ ID NO:26 or SEQ ID NO:27, the amino acid sequence of CDR2 is the amino acid sequence of SEQ ID NO:28 or SEQ ID NO:29, and the amino acid sequence of CDR3 is the amino acid sequence of SEQ ID NO:30 or SEQ ID NO: 31, where each of the amino acid sequences of CDR1, CDR2, and CDR3 may be any sequence; however, preferably, the amino acid sequence of CDR1 is the amino acid sequence of SEQ ID NO:26, the amino acid sequence of CDR2 is the amino acid sequence of SEQ ID NO:28, and the amino acid sequence of CDR3 is the amino acid sequence of SEQ ID NO:30, or the amino acid sequence of CDR1 is the amino acid sequence of SEQ ID NO:27 the amino acid sequence of CDR2 is the amino acid sequence of SEQ ID NO:29, and the amino acid sequence of CDR3 is the amino acid sequence of SEQ ID NO:31;
   (5) a heavy chain antibody variable region peptide in which the amino acid sequence of CDR1 is the amino acid sequence of SEQ ID NO:20 or SEQ ID NO:21, the amino acid sequence of CDR2 is the amino acid sequence of SEQ ID NO:22 or SEQ ID NO:23, and the amino acid sequence of CDR3 is the amino acid sequence of SEQ ID NO:24 or SEQ ID NO:25, where each of the amino acid sequences of CDR1, CDR2, and CDR3 may be any sequence; however, preferably, the amino acid sequence of CDR1 is the amino acid sequence of SEQ ID NO:20, the amino acid sequence of CDR2 is the amino acid sequence of SEQ ID NO:22, and the amino acid sequence of CDR3 is the amino acid sequence of SEQ ID NO:24, or the amino acid sequence of CDR1 is the amino acid sequence of SEQ ID NO:21 the amino acid sequence of CDR2 is the amino acid sequence of SEQ ID NO:23, and the amino acid sequence of CDR3 is the amino acid sequence of SEQ ID NO:25; and
   (6) a heavy chain antibody variable region peptide in which the amino acid sequence of CDR1 is the amino acid sequence of SEQ ID NO:20 or SEQ ID NO:21, the amino acid sequence of CDR2 is the amino acid sequence of SEQ ID NO:56 or SEQ ID NO:57, and the amino acid sequence of CDR3 is the amino acid sequence of SEQ ID NO:24 or SEQ ID NO:25, where each of the amino acid sequences of CDR1, CDR2, and CDR3 may be any sequence; however, preferably, the amino acid sequence of CDR1 is the amino acid sequence of SEQ ID NO:20, the amino acid sequence of CDR2 is the amino acid sequence of SEQ ID NO:56, and the amino acid sequence of CDR3 is the amino acid sequence of SEQ ID NO:24, or the amino acid sequence of CDR1 is the amino acid sequence of SEQ ID NO:21 the amino acid sequence of CDR2 is the amino acid sequence of SEQ ID NO:57, and the amino acid sequence of CDR3 is the amino acid sequence of SEQ ID NO:25.
70. A heavy chain antibody variable region peptide having affinity for human transferrin receptor, the heavy chain antibody variable region peptide having three complementarity-determining regions, CDR1, CDR2, and CDR3, and being selected from the group consisting of the following (1) to (6):
   (1) a heavy chain antibody variable region peptide which contains the amino acid sequence having the amino acid sequence identity of 80% or higher to the amino acid sequence set forth in SEQ ID NO:3, and in which the amino acid sequences of CDR1, CDR1, and CDR3 are as shown in option (1) of 69 above;
   (2) a heavy chain antibody variable region peptide which contains the amino acid sequence having the amino acid sequence identity of 80% or higher to the amino acid sequence set forth in SEQ ID NO:4, and in which the amino acid sequences of CDR1, CDR1, and CDR3 are as shown in option (2) of 69 above;
   (3) a heavy chain antibody variable region peptide which contains the amino acid sequence having the amino acid sequence identity of 80% or higher to the amino acid sequence set forth in SEQ ID NO:5, and in which the amino acid sequences of CDR1, CDR1, and CDR3 are as shown in option (3) of 69 above;
   (4) a heavy chain antibody variable region peptide which contains the amino acid sequence having the amino acid sequence identity of 80% or higher to the amino acid sequence set forth in SEQ ID NO:6, and in which the amino acid sequences of CDR1, CDR1, and CDR3 are as shown in option (4) of 69 above;
   (5) a heavy chain antibody variable region peptide which contains the amino acid sequence having the amino acid sequence identity of 80% or higher to the amino acid sequence set forth in SEQ ID NO:7, and in which the amino acid sequences of CDR1, CDR1, and CDR3 are as shown in option (5) of 69 above; and
   (6) a heavy chain antibody variable region peptide which contains the amino acid sequence having the amino acid sequence identity of 80% or higher to the amino acid sequence set forth in SEQ ID NO:8, and in which the amino acid sequences of CDR1, CDR1, and CDR3 are as shown in option (6) of 69 above.
71. A heavy chain antibody variable region peptide selected from the group consisting of (1) to (6) of 70 above, wherein the amino acid sequence identity is 85% or higher.
72. A heavy chain antibody variable region peptide selected from the group consisting of (1) to (6) of 70 above, wherein the amino acid sequence identity is 90% or higher.
73. A heavy chain antibody variable region peptide selected from the group consisting of (1) to (6) of 70 above, wherein the amino acid sequence identity is 95% or higher.
74. A heavy chain antibody variable region peptide having affinity for human transferrin receptor, the heavy chain antibody variable region peptide having three complementarity-determining regions, CDR1, CDR2, and CDR3, and being selected from the group consisting of the following (1) to (6):
   (1) a heavy chain antibody variable region peptide having the amino acid sequence in which 1 to 10 amino acids are substituted, deleted, or added relative to the amino acid sequence set forth in SEQ ID NO:3, in which the amino acid sequences of CDR1, CDR1, and CDR3 are as shown in option (1) of 69 above;
   (2) a heavy chain antibody variable region peptide having the amino acid sequence in which 1 to 10 amino acids are substituted, deleted, or added relative to the amino acid sequence set forth in SEQ ID NO:4, in which the amino acid sequences of CDR1, CDR1, and CDR3 are as shown in option (2) of 69 above;
   (3) a heavy chain antibody variable region peptide having the amino acid sequence in which 1 to 10 amino acids are substituted, deleted, or added relative to the amino acid sequence set forth in SEQ ID NO:5, in which the amino acid sequences of CDR1, CDR1, and CDR3 are as shown in option (3) of 69 above;
   (4) a heavy chain antibody variable region peptide having the amino acid sequence in which 1 to 10 amino acids are substituted, deleted, or added relative to amino acid sequence set forth in SEQ ID NO:6, in which the amino acid sequences of CDR1, CDR1, and CDR3 are as shown in option (4) of 69 above;
   (5) a heavy chain antibody variable region peptide having the amino acid sequence in which 1 to 10 amino acids are substituted, deleted, or added relative to the amino acid sequence set forth in SEQ ID NO:7, in which the amino acid sequences of CDR1, CDR1, and CDR3 are as shown in option (5) of 69 above; and
   (6) a heavy chain antibody variable region peptide having the amino acid sequence in which 1 to 10 amino acids are substituted, deleted, or added relative to the amino acid sequence set forth in SEQ ID NO:8, in which the amino acid sequences of CDR1, CDR1, and CDR3 are as shown in option (6) of 69 above.
75. A heavy chain antibody variable region peptide selected from the group consisting of (1) to (6) of 74 above, wherein the number of substituted, deleted, or added amino acids is 1 to 5.
76. A heavy chain antibody variable region peptide selected from the group consisting of (1) to (6) of 74 above, wherein the number of substituted, deleted, or added amino acids is 1 to 3.
77. The heavy chain antibody variable region peptide according to any one of 69 to 76 above, wherein the heavy chain antibody variable region peptide has affinity for both an extracellular region of human transferrin receptor and an extracellular region of monkey transferrin receptor.
78. The heavy chain antibody variable region peptide according to 77 above, wherein a dissociation constant with the extracellular region of human transferrin receptor is 5×10⁻⁹ to 1×10⁻⁷.
79. The heavy chain antibody variable region peptide according to 77 above, wherein the dissociation constant with the extracellular region of human transferrin receptor is 8×10⁻⁹ to 2×10⁻⁸.
80. The heavy chain antibody variable region peptide according to 77 above, wherein when the value of the dissociation constant with the extracellular region of human transferrin receptor is taken as 1, the value of the dissociation constant with monkey transferrin receptor is 0.5 to 2.5.
81. The heavy chain antibody variable region peptide according to 77 above, wherein when the value of the dissociation constant with the extracellular region of human transferrin receptor is taken as 1, the value of the dissociation constant with monkey transferrin receptor is 0.7 to 1.2.
82. A heavy chain antibody variable region peptide selected from the group consisting of the following (1) to (3), wherein a plurality of the heavy chain antibody variable region peptide according to 69 to 81 above are linked:
   (1) a heavy chain antibody variable region peptide in which 2 to 10 of the heavy chain antibody variable region peptide according to 41 to 53 above are linked directly or via a linker;
   (2) a heavy chain antibody variable region peptide in which 2 or 3 of the heavy chain antibody variable region peptide according to 41 to 53 above are linked directly or via a linker; and
   (3) a heavy chain antibody variable region peptide in which 2 of the heavy chain antibody variable region peptide according to 41 to 53 above are linked directly or via a linker.
83. The heavy chain antibody variable region peptide according to 82 above, wherein the linker for linking between the heavy chain antibody variable region peptides is a peptide linker consisting of 1 to 50 amino acids.
84. The heavy chain antibody variable region peptide according to 83 above, wherein the amino acid sequence of the peptide linker for linking between the heavy chain antibody variable region peptides is selected from the group consisting of a single amino acid, the amino acid sequence Gly-Ser, the amino acid sequence Ser-Ser, the amino acid sequence Gly-Gly-Ser, the amino acid sequence Gly-Gly-Gly, and the amino acid sequences of SEQ ID NO:32, SEQ ID NO:33, SEQ ID NO:34, SEQ ID NO:35, SEQ ID NO:36, SEQ ID NO:58, and SEQ ID NO:69.
85. A heavy chain antibody variable region peptide in which the amino acid sequence of a peptide linker for linking between the heavy chain antibody variable region peptides is the amino acid sequence in which 2 to 10 of the amino acid sequences selected from the group consisting of a single amino acid, the amino acid sequence Gly-Ser, the amino acid sequence Ser-Ser, the amino acid sequence Gly-Gly-Ser, the amino acid sequence Gly-Gly-Gly, and the amino acid sequences of SEQ ID NO:32, SEQ ID NO:33, SEQ ID NO:34, SEQ ID NO:35, SEQ ID NO:36, SEQ ID NO:58, and SEQ ID NO:69, are linked in tandem.
86. A heavy chain antibody variable region peptide-drug complex in which a heavy chain antibody variable region peptide is linked to a drug, wherein the heavy chain antibody variable region peptide is the heavy chain antibody variable region peptide according to any one of 69 to 85 above.
87. The complex according to 86 above, wherein the drug is any one of a different protein (A), a nucleic acid, or a low molecular weight compound.
88. A fusion protein of a heavy chain antibody variable region peptide and a different protein (A), wherein the heavy chain antibody variable region peptide is the heavy chain antibody variable region peptide according to any one of 69 to 85 above.
89. The fusion protein according to 88 above, wherein the heavy chain antibody variable region peptide is bound to the C-terminus of the different protein (A) directly or via a linker.
90. The fusion protein according to 88 above, wherein the heavy chain antibody variable region peptide is bound to the N-terminus of the different protein (A) directly or via a linker.
91. The fusion protein according to 89 or 90 above, wherein the linker is a peptide linker consisting of 1 to 50 amino acids.
92. The fusion protein according to 91 above, wherein the amino acid sequence of the peptide linker is selected from the group consisting of a single amino acid, the amino acid sequence Gly-Ser, the amino acid sequence Ser-Ser, the amino acid sequence Gly-Gly-Ser, the amino acid sequence Gly-Gly-Gly, and the amino acid sequences of SEQ ID NO:32, SEQ ID NO:33, SEQ ID NO:34, SEQ ID NO:35, SEQ ID NO:36, SEQ ID NO:58, and SEQ ID NO:69.
93. The fusion protein according to 91 above, wherein the amino acid sequence of the peptide linker is the amino acid sequence in which 2 to 10 of the amino acid sequence selected from the group consisting of a single amino acid, the amino acid sequence Gly-Ser, the amino acid sequence Ser-Ser, the amino acid sequence Gly-Gly-Ser, the amino acid sequence Gly-Gly-Gly, and the amino acid sequences of SEQ ID NO:32, SEQ ID NO:33, SEQ ID NO:34, SEQ ID NO:35, SEQ ID NO:36, SEQ ID NO:58, and SEQ ID NO:69, are linked in tandem.
94. The complex or fusion protein according to any one of 87 to 93 above, wherein the different protein (A) is a protein originating from human.
95. The complex or fusion protein according to any one of 87 to 94 above, wherein the different protein (A) is a cytokine, a growth factor, or an antibody medicine.
96. The complex or fusion protein according to any one of 87 to 94 above, wherein the different protein (A) is selected from those shown in 35 above.
97. The complex or fusion protein according to any one of 87 to 94 above, wherein the different protein (A) is a lysosomal enzyme, and the lysosomal enzyme is selected from those shown in 36 above.
98. The complex or fusion protein according to any one of 87 to 94 above, wherein serum albumin is bound to the complex or the fusion protein.
99. The complex or fusion protein according to any one of 87 to 94 above, wherein a human IgG Fc region or a portion thereof is bound to the complex or the fusion protein.
100. A nucleic acid encoding the heavy chain antibody variable region peptide according to any one of 69 to 85 above.
101. A nucleic acid encoding the fusion protein according to any one of 88 to 99 above.
102. An expression vector comprising the nucleic acid according to 100 or 101 above incorporated therein.
103. A cell transformed with the expression vector according to 102 above.
104. The cell according to 103 above, wherein the cell is derived from a mammal.

### Advantageous Effects of Invention

The peptide having affinity for human transferrin receptor (hTfR affinity peptide), which is an embodiment of the present invention, is such that when bound to a substance that can hardly or not at all pass through the blood brain barrier, the peptide can cause the substance to pass through the blood brain barrier and reach the central nervous system, and therefore, the function of the substance can be exhibited in the central nervous system.

### Brief Description of Drawings

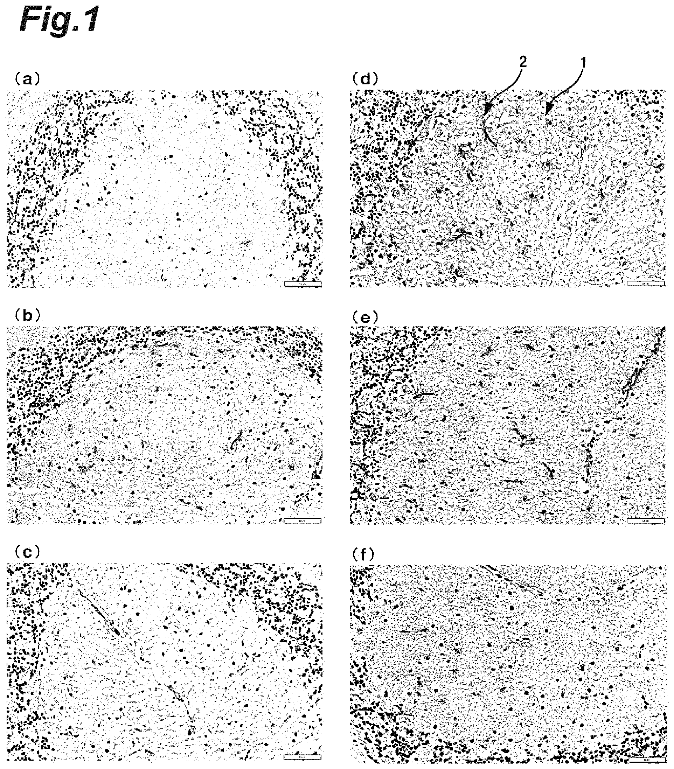

FIG. 1 is drawing-substituting photographs showing the results of immunohistochemical staining for anti-hTfR antibody in mouse cerebellum 6 hours after the administration of hTfR affinity peptide-trastuzumab. (a) shows a result of administering trastuzumab, (b) shows a result of administering hTfR affinity peptide-trastuzumab 1, (c) shows a result of administering hTfR affinity peptide-trastuzumab 2, (d) shows a result of administering hTfR affinity peptide-trastuzumab 3, (e) shows a result of administering hTfR affinity peptide-trastuzumab 4, and (f) shows a result of administering hTfR affinity peptide-trastuzumab 5. The bar at the lower right corner of each photograph indicates a 50-µm gauge.

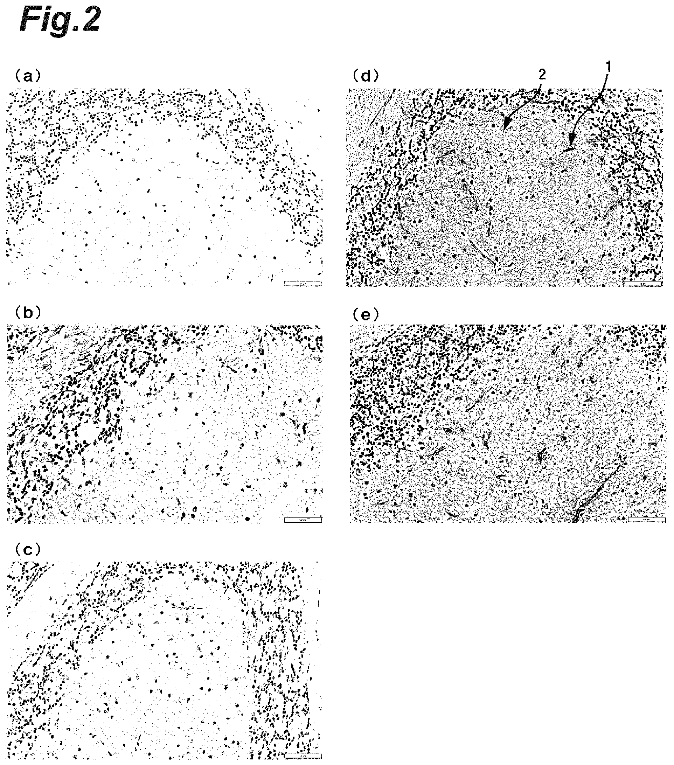

FIG. 2 is drawing-substituting photographs showing the results of immunohistochemical staining for anti-hTfR antibody in mouse cerebellum 24 hours after the administration of hTfR affinity peptide-trastuzumab. (a) shows a result of administering trastuzumab, (b) shows a result of administering hTfR affinity peptide-trastuzumab 1, (c) shows a result of administering hTfR affinity peptide-trastuzumab 2, (d) shows a result of administering hTfR affinity peptide-trastuzumab 3, and (e) shows a result of administering hTfR affinity peptide-trastuzumab 4. The bar at the lower right corner of each photograph indicates a 50-µm gauge.

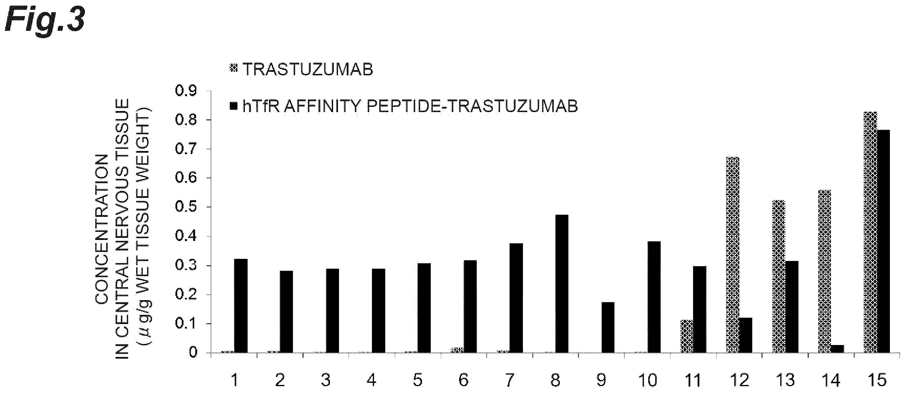

FIG. 3 is a graph showing the quantitative values of hTfR affinity peptide-trastuzumab 5 and trastuzumab included in various brain tissues of cynomolgus monkeys 8 hours after the administration of these substances. The graph shows the quantitative values of (1) cerebral cortex, (2) cerebellum, (3) hippocampus, (4) midbrain, (5) pons, (6) medulla oblongata, (7) caudate nucleus, (8) putamen, (9) globus pallidus, (10) thalamus, (11) hypothalamus, (12) cervical spinal cord, (13) thoracic spinal cord, (14) lumbar spinal cord, and (15) retina. The vertical axis represents the amounts (µg) of these substances included per wet weight (g). Shaded bars indicate the quantitative values of trastuzumab, and black bars indicate the quantitative values of hTfR affinity peptide-trastuzumab 5.

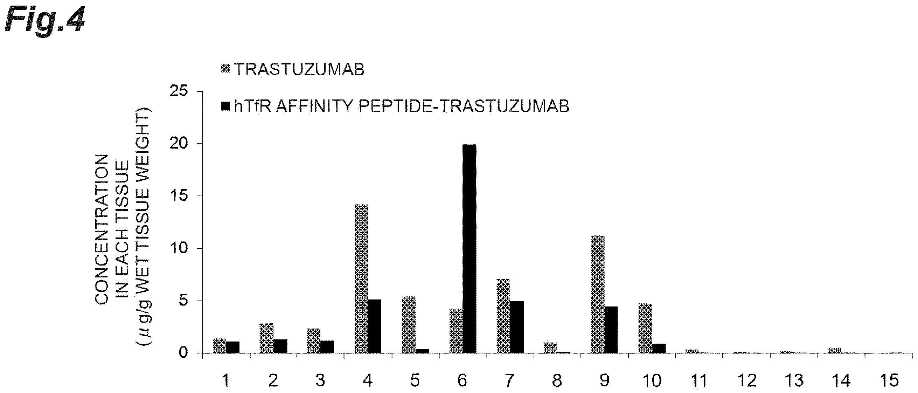

FIG. 4 is a graph showing the quantitative values of hTfR affinity peptide-trastuzumab 5 and trastuzumab included in various organs of cynomolgus monkeys 8 hours after the administration of these substances. The graph shows the quantitative values of (1) liver, (2) kidney, (3) heart (left ventricle), (4) heart (aortic valve), (5) lung, (6) bone marrow, (7) spleen, (8) thymus, (9) testis, (10) prostate, (11) rectus femoris muscle, (12) EDL, (13) soleus muscle, (14) triceps, and (15) diaphragm. The vertical axis represents the amounts (µg) of these substances included per wet weight (g). Shaded bars indicate the quantitative values of trastuzumab, and black bars indicate the quantitative values of hTfR affinity peptide-trastuzumab 5.

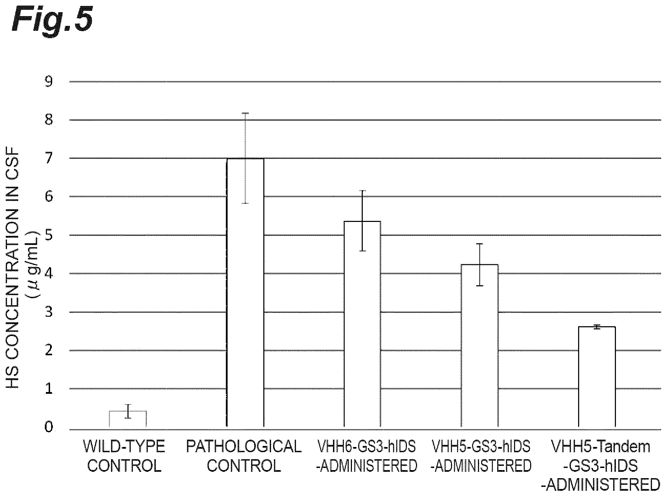

FIG. 5 is a graph showing the measured values of the concentration of heparan sulfate (HS) included in mouse cerebrospinal fluid (CSF) after the administration of a fusion protein of hTfR affinity peptide and human iduronate-2-sulfatase (hIDS). The vertical axis represents the concentration (µg/mL) of heparan sulfate (HS) included in the cerebrospinal fluid (CSF). Vertical bars indicate SD values.

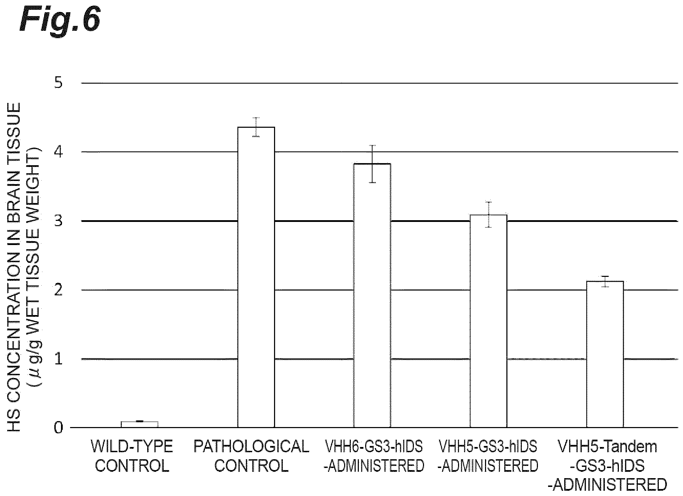

FIG. 6 is a graph showing the measured values of the concentration of heparan sulfate (HS) included in mouse brain tissue after administration of a fusion protein of hTfR affinity peptide and human iduronate-2-sulfatase (hIDS). The vertical axis represents the concentration (µg/mg) of heparan sulfate (HS) included per dry weight (mg) of the brain tissue. Vertical bars indicate SD values.

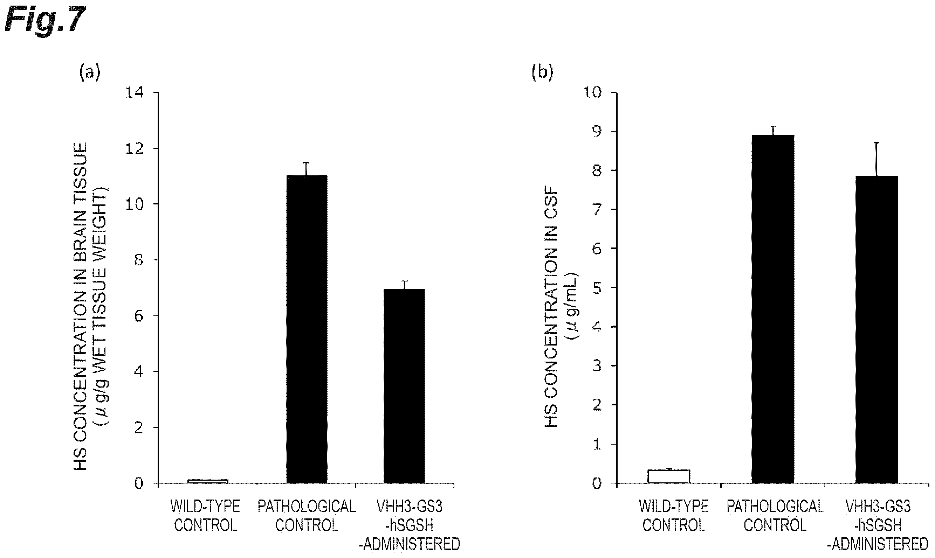

FIG. 7 is a graph showing the measured values of the concentration of heparan sulfate (HS) included in mouse cerebrospinal fluid (CSF) and brain tissue after administration of a fusion protein of hTfR affinity peptide and human heparan-N-sulfatase (hSGSH). (a) shows the measured value of heparan sulfate (HS) included in the brain tissue, and (b) shows the measured value in the cerebrospinal fluid (CSF). In (a), the vertical axis represents the concentration (µg/mg) of heparan sulfate (HS) included per dry weight (mg) of the brain tissue, and vertical bars indicate SD values. In (b), the vertical axis represents the concentration (µg/mL) of heparan sulfate (HS) included in the cerebrospinal fluid (CSF), and vertical bars indicate SD values.

### Description of Embodiments

In many mammals including human and mouse, antibody monomers are formed as heterotetramers in which two H chains and two L chains are covalently bonded by disulfide bonds. At the N-terminus of each chain, there is a region in which the amino acid sequence differs among different antibodies, and this region is referred to as a variable region. In the variable region of the H chain, there are three complementarity-determining regions that significantly contribute to specific binding to antigens. These three complementarity-determining regions are referred to as CDR1, CDR2, and CDR3 in order from the N-terminus. Similarly, in the L chain as well, there are three complementarity-determining regions that significantly contribute to specific binding to antigens, and these three complementarity-determining regions are referred to as CDR1, CDR2, and CDR3 in order from the N-terminus.

One H chain and one L chain form a pair and specifically bind to an antigen. That is, specificity to an antigen is roughly determined by the amino acid sequences of a total of six sites of CDR, including three sites of CDR in the H chain and three sites of CDR in the L chain. Since monomers of many mammalian antibodies have two pairs of an H chain and an L chain, one antibody molecule has two sites of a region that can bind to antigens.

Regions other than the CDR in the variable region are called framework regions (FR). Each of the H chain and the L chain has four sites of FR, and they are referred to as FR1, FR2, FR3, and FR4 in order from the N-terminus. A CDR is sandwiched between FRs in the variable region.

FR1 is a region extending from the N-terminus of the variable region of the heavy chain antibody to the amino acid adjacent to the N-terminus of CDR1. FR2 is a region between CDR1 and CDR2. FR3 is a region between CDR2 and CDR3. FR4 is a region extending from the amino acid adjacent to the C-terminus of CDR3 to the C-terminus of the variable region of the heavy chain antibody.

In contrast to the above-described monomers of many mammalian antibodies, there are antibodies containing only heavy chains with no L chains (heavy chain antibodies). Examples thereof include a heavy chain antibody (hcIg) produced by the Camelidae animals, and an immunoglobulin new antigen receptor (IgNAR) produced by many cartilaginous fishes, including sharks. The heavy chain antibody (hcIg) of the Camelidae animals is a homodimer in which two heavy chains are covalently bonded by disulfide bonds. Each of the heavy chains has a variable region at the N-terminus, and in this variable region, there are three sites of the complementarity-determining region (CDRs) that significantly contribute to specific binding to antigens. These three sites of the complementarity-determining region are referred to as CDR1, CDR2, and CDR3 in order from the N-terminus. Since each heavy chain constituting hcIg can specifically bind to an antigen even in a monomeric form, one hcIg has two sites of the region that can bind to an antigen in the molecule.

Similarly to hcIg, IgNAR is also a homodimer in which two heavy chains are covalently bonded by disulfide bonds. Each of the heavy chains has a variable region at the N-terminus, and this variable region has three sites of the complementarity-determining region (CDR) that significantly contributes to specific binding to an antigen. These three sites of the complementarity-determining region are referred to as CDR1, CDR2, and CDR3 in order from the N-terminus. Since each heavy chain constituting IgNAR can specifically bind to an antigen even in a monomeric form, one IgNAR has two sites of the region that can bind to an antigen in the molecule.

The framework region in the variable region of hcIg and IgNAR can be identified from public DNA databases and the like that include germline antibody gene sequences. For example, germline DNA sequences and amino acid sequences of human heavy chain variable region genes can be selected from the "VBase" human germline sequence database (available on the Internet at www.mrccpe.cam.ac.uk/vbase). The germline DNA sequences and amino acid sequences can also be identified from DNA sequences and amino acid sequences described in other published literature, for example, "Kabat EA, Sequences of Proteins of Immunological Interest, 5th edition, U.S. Department of Health and Human Services, NIH Publication No. 91-3242 (1991)", "Tomlinson IM, J. fol. Biol. 227, 776-98 (1992)", and "Cox JPL, Eur. J. Immunol. 24:827-836 (1994)". For example, since the variable region gene of the human heavy chain is homologous to the variable region of hcIg, it is also possible to identify the framework region of hcIg based on information on the variable region gene of the human heavy chain.

However, without being limited thereto, for each FR region, (1) a region containing 1 to 5 amino acids from the N-terminus thereof and/or 1 to 5 amino acids at the C-terminus, (2) a region containing 1 to 3 amino acids from the N-terminus thereof and/or 1 to 3 amino acids at the C-terminus, or (3) a region including 1 or 2 amino acids from the N-terminus thereof and/or 1 or 2 amino acids at the C-terminus, may be included in the CDR region without being included in the FR region. Even when a region is generally considered as FR, the region can be included in the CDR in the present invention as long as the region is involved in maintaining the structure of CDR or binding to an antigen and has a function of substantially determining the complementarity of the antibody.

At the C-terminus of the variable region of the heavy chain antibody, a constant region is located, with a hinge region interposed therebetween. A constant region is located at the C-terminus of the variable region of IgNAR as well. These constant regions almost do not contribute to specific binding of the heavy chain antibody to antigens.

The heavy chain antibody is a homodimer covalently bonded by disulfide bonds at the hinge region. However, the heavy chain antibody does not have to be a homodimer in order to bind to an antigen, and each of the heavy chains constituting the heavy chain antibody can bind to an antigen by itself.

A monomer of the heavy chain constituting the heavy chain antibody can be obtained by deleting cysteine residues that form a disulfide bond in the amino acid sequence of the heavy chain, or substituting the cysteine residues with other amino acids. Such a monomer of the heavy chain can be produced as a recombinant protein using a genetic engineering technology. In the present specification, such a heavy chain is referred to as a "single heavy chain antibody". A single heavy chain antibody is included in the heavy chain antibody as long as it can bind to the antigen of the heavy chain antibody from which the single heavy chain antibody was derived. Single heavy chain antibodies can be connected in tandem. Each of the single heavy chain antibodies is linked by linking the C-terminus of one to the N-terminus of another, directly or via a peptide linker. The number of the single heavy chain antibodies to be connected is 2 to 10, and the number is, for example, 2. Regarding the sequence of the peptide linker, those described in the present specification can be applied. An antibody obtained by connecting a plurality of such single heavy chain antibodies is also included in the single heavy chain antibody.

The variable region of each of the heavy chains constituting the heavy chain antibody can bind to an antigen even in the absence of a hinge region or/and a constant region. A protein composed of such a variable region can be produced as a recombinant protein by, for example, constructing a gene encoding only the amino acid sequence of the variable region of the heavy chain of a heavy chain antibody, incorporating this gene into an expression vector, and introducing the expression vector into a host cell.

In the present specification, for convenience, any antibodies that contain a part or the entirety of the variable region of the heavy chain constituting a heavy chain antibody and have affinity for antigens are all considered to be included in the heavy chain antibody. Furthermore, any antibody including: (1) a part or the entirety of the hinge region, and (2) the hinge region as well as a part of the constant region of the heavy chain, in addition to a part or the entirety of the variable region of the heavy chains constituting a heavy chain antibody, is also included in the heavy chain antibody. Heavy chain antibodies include those derived from hcIg and those derived from IgNAR.

Heavy chains derived from hcIg include: (1h) those composed only of the entirety or a part of the variable region of the heavy chain, (2h) those composed of the entirety or a part of the variable region and a part of the hinge region of the heavy chain, (3h) those composed of the entirety of the variable region and the hinge region of the heavy chain, and (4h) those including a part of the constant region in addition to the variable region and the hinge region of the heavy chain. Here, a portion of the constant region included in the heavy chain antibody is, for example, a portion corresponding to a part or the entirety of C_{H}2 of the heavy chain. Heavy chain antibodies derived from IgNAR include: (1I) those composed only of the entirety or a part of the variable region of the heavy chain, (2I) those composed of the variable region and a part of the hinge region of the heavy chain, (3I) those composed of the entirety of the variable region and the hinge region of the heavy chain, and (4I) those including a part of the constant region in addition to the variable region and the hinge region of the heavy chain. Here, a part of the constant region included in the heavy chain antibody is, for example, a portion corresponding to a part or the entirety of C_{H}1 of the heavy chain. In the present specification, the proteins of (1h) to (4h) containing the variable region of the heavy chain antibody derived from hcIg, and the proteins of (1I) to (4I) containing the variable region of the heavy chain antibody derived from IgNAR are collectively referred to as heavy chain antibody variable region peptides. Therefore, the heavy chain antibody variable region peptide includes those having two antigen-binding sites in which two peptide chains are linked by disulfide bonds, and those having one antigen-binding site composed of one peptide chain.

In the present specification, the heavy chain antibody recognizes a molecule present on the surface of a vascular endothelial cell, particularly a cerebrovascular endothelial cell, as an antigen. There is no particular limitation on the species of the vascular endothelial cell and the cerebrovascular endothelial cell; however, human cells are preferred. The molecule present on the surface of these cells is preferably transferrin receptor (TfR), insulin receptor (IR), leptin receptor, a lipoprotein receptor, an IGF receptor, OATP-F, an organic anion transporter, MCT-8, or a monocarboxylate transporter; more preferably TfR or insulin receptor; and even more preferably TfR.

A heavy chain antibody variable region peptide having affinity for a molecule present on the surface of a vascular endothelial cell, particularly a cerebrovascular endothelial cell, is referred to as a blood brain barrier heavy chain antibody variable region peptide (BBB heavy chain antibody variable region peptide). Furthermore, the BBB heavy chain antibody variable region peptide and all peptides having affinity for molecules present on the surface of vascular endothelial cells, the peptides being derived from the BBB heavy chain antibody variable region peptide, are referred to as BBB affinity peptides. In the present specification, the protein of the above-described (1h) or (2h) containing the variable region of a heavy chain antibody derived from hcIg is referred to as an immunoglobulin single variable domain peptide. The immunoglobulin single variable domain peptide included in the BBB heavy chain antibody variable region peptide may be particularly referred to as a BBB immunoglobulin single variable domain peptide. In the present invention, each of the terms VHH, VHH domain, VHH antibody, NANOBODY (trademark of Ablynx N.Y.), and NANOBODY (trademark of Ablynx N.V.) has the same meaning as the immunoglobulin single variable domain peptide and is used interchangeably. Therefore, for example, VHH having affinity for TfR is included in the BBB immunoglobulin single variable domain peptide.

A heavy chain antibody variable region peptide having affinity for anti-hTfR is referred to as anti-hTfR heavy chain antibody variable region peptide. Furthermore, the anti-hTfR heavy chain antibody variable region peptide, and all peptides having affinity for anti-hTfR, which are derived from the anti-hTfR heavy chain antibody variable region peptide, are referred to as hTfR affinity peptides. A VHH having affinity for hTfR is particularly included in the hTfR affinity peptide. The same also applies to other VHHs having affinity for the molecules present on the surface of cells, and for example, a VHH having affinity for hIR is included in the hIR affinity peptide. There is no particular limitation on the number of amino acids constituting a BBB affinity peptide; however, the BBB affinity peptide is usually a peptide composed of 80 to 200 amino acids, preferably 80 to 150 amino acids, more preferably 100 to 130 amino acids, and even more preferably 110 to 125 amino acids. For example, a BBB affinity peptide is composed of 113, 115, 119, or 120 amino acids. In the present specification, the term "peptide" has the same meaning as "polypeptide" or "protein", and can be replaced with these terms. In the following description, a heavy chain antibody variable region peptide having affinity for human transferrin receptor (hTfR) will be taken as an example, and characteristics and the like thereof will be described in detail; however, these descriptions can also be generally applied to other heavy chain antibody variable region peptides having affinity for molecules present on the surface of cells.

While an antibody is usually composed of light chains and heavy chains, a VHH is composed of one heavy chain, and therefore, the process in the case of producing a fusion protein in which the VHH is bound to a different protein (A) as an hTfR affinity peptide can be simplified. For example, in a case where a conventional antibody is used as the hTfR affinity peptide, it is necessary to express two proteins; however, when using a VHH, it is sufficient to express one protein.

A heavy chain antibody variable region peptide having affinity for human transferrin receptor is particularly referred to as anti-hTfR heavy chain antibody variable region peptide. In the present specification, the above-described protein of (1h) or (2h) containing a heavy chain antibody variable region derived from hcIg is referred to as an immunoglobulin single variable domain peptide. An immunoglobulin single variable domain peptide having affinity for human transferrin receptor (hTfR) is particularly referred to as hTfR immunoglobulin single variable domain peptide or human transferrin receptor affinity peptide (hTfR affinity peptide). The hTfR affinity peptide is a peptide composed of preferably 100 to 130 amino acids, and more preferably 110 to 125 amino acids. For example, the hTfR affinity peptide is composed of 113, 115, or 119 amino acids.

In addition to the peptides described as (1h) to (4h) and (1I) to (4I), the heavy chain antibody variable region peptides also include peptides in which mutations such as substitution, deletion, and addition have been applied to the above-described peptides, as long as the peptides have affinity for the antigens for which the original peptides have affinity. Furthermore, in addition to the peptide described as (1h) or (2h), immunoglobulin single variable domain peptides also include peptides in which mutations such as substitution, deletion, and addition have been applied to the above-described peptide, as long as the peptide has affinity for the antigen for which the original peptide has affinity. Mutations to the hTfR immunoglobulin single variable domain peptides will be described in more detail below.

According to the present invention, the term "human transferrin receptor" or "hTfR" refers to a membrane protein having the amino acid sequence set forth in SEQ ID NO: 1. According to an embodiment, the anti-hTfR antibody of the present invention specifically binds to a portion of the amino acid sequence set forth in SEQ ID NO:1, the portion including from the 89^{th} cysteine residue from the N-terminus to phenylalanine at the C-terminus (extracellular region of hTfR); however, the embodiment is not limited to this. Furthermore, the term "monkey transferrin receptor" or "monkey TfR" according to the present invention particularly refers to a membrane protein having the amino acid sequence set forth in SEQ ID NO:2 derived from cynomolgus monkey (Macaca fascicularis). According to an embodiment, the anti-hTfR antibody of the present invention also binds to a portion of the amino acid sequence set forth in SEQ ID NO:2, the portion including from the 89^{th} cysteine residue from the N-terminus to phenylalanine at the C-terminus (extracellular region of monkey TfR); however, the embodiment is not limited to this.

The hTfR affinity peptide can also be obtained as a recombinant protein by a similar technique.
(1a) A recombinant human transferrin receptor (rhTfR) is produced using cells into which an expression vector incorporating hTfR gene has been introduced.
(2a) Camelidae animals capable of producing hcIg, or fishes, particularly cartilaginous fish, capable of producing IgNAR such as shark, are immunized using rhTfR.
(3a) Antibody-producing cells are taken out from the organisms immunized using rhTfR. Here, since the antibody-producing cells are present particularly in peripheral blood, bone marrow, spleen, and the like, cells in peripheral blood, bone marrow-derived cells, spleen cells, and the like are collected as the antibody-producing cells.
(4a) cDNA is synthesized from mRNA extracted from the antibody-producing cells by a reverse transcription reaction.
(5a) A PCR reaction is performed using cDNA as a template, and a DNA fragment containing a gene encoding a variable region of a heavy chain antibody is amplified.
(6a) The DNA fragment encoding a variable region of a heavy chain antibody is incorporated into an expression vector, and host cells are transformed using this expression vector to obtain cells expressing the variable region of a heavy chain antibody.
(7a) From the cells expressing the variable region of a heavy chain antibody, cells expressing the variable region of a heavy chain antibody which recognizes hTfR (cells expressing the variable region of anti-hTfR heavy chain) are selected. In is also possible that from the cells selected here, the gene encoding the variable region of a heavy chain antibody which recognizes hTfR is amplified by performing a PCR reaction and isolated.
(8a) The cells expressing the variable region of anti-hTfR heavy chain antibody are cultured to express a recombinant anti-hTfR heavy chain antibody variable region peptide in the culture fluid or in the cells.
(9a) The expressed recombinant anti-hTfR heavy chain antibody variable region peptide is purified.

The cells expressing the variable region of the heavy chain antibody obtained in the above-described step (6a) for acquiring an anti-hTfR heavy chain antibody which recognizes hTfR, do not necessarily express the variable region of the heavy chain antibody which recognizes hTfR. Therefore, a step of selecting cells that produce the variable region of a heavy chain antibody having desired characteristics (affinity for hTfR) from the cells expressing the variable region of a heavy chain antibody obtained in the above-described step (6a), that is, the above-described step (7a), is required. As a method for selecting cells that produce an anti-hTfR heavy chain antibody variable region peptide, the method that will be described in detail below is effective.

The cells expressing the variable region of a heavy chain antibody obtained in the above-described step (6a) are seeded and cultured on a 96-well plate such that approximately one well contains one cell, and then the culture supernatant is collected from each well. Recombinant hTfR is added to the plate and retained thereon, subsequently the collected culture supernatant is added thereto, and the variable region of the anti-hTfR heavy chain antibody contained in the culture supernatant is let bound to the recombinant hTfR. Next, the culture supernatant is removed from the plate, and the plate is further washed to remove any antibodies that are not bound to the recombinant hTfR. Next, the quantity of antibodies retained on the plate is measured. According to this method, as the affinity for hTfR of the antibody contained in the culture supernatant of the antibody-producing cells added onto the plate is higher, the quantity of the antibodies retained on the plate becomes larger. Therefore, the quantity of the antibodies retained on the plate is measured, and cells corresponding to the plate on which more antibodies are retained can be selected as the cell line producing the anti-hTfR heavy chain antibody variable region peptide having relatively higher affinity for hTfR. It is also possible to perform a PCR reaction using cDNA obtained by reverse transcription of the mRNA extracted from the cell line selected in this manner as a template, and to amplify and isolate a DNA fragment containing a gene encoding the anti-hTfR heavy chain antibody variable region peptide.

As another method for obtaining the anti-hTfR heavy chain antibody as a recombinant protein, a method including the following step is available. This method includes a step of displaying the variable region of a heavy chain antibody encoded by a gene encoding the variable region of a heavy chain antibody on phages, and selecting the phages that display the variable region of the heavy chain antibody having desired properties. Such a process is called a phage display method and is a known technology described in PCT International Publications (WO 1997/09436 and WO 1995/11317) and the like. A method of applying the phage display method to obtain an anti-hTfR heavy chain antibody which recognizes hTfR as a recombinant protein includes, for example, the following steps (1b) to (9b). An hTfR affinity peptide can also be obtained as a recombinant protein by a similar technique.
(1b) A recombinant human transferrin receptor (rhTfR) is produced by using cells into which an expression vector incorporating hTfR gene has been introduced.
(2b) Camelidae animals capable of producing hcIg, or fishes, particularly cartilaginous fish, capable of producing IgNAR such as shark, are immunized using rhTfR.
(3b) Antibody-producing cells are taken out from the organisms immunized using rhTfR. Here, since the antibody-producing cells are present particularly in peripheral blood, bone marrow, spleen, and the like, cells in peripheral blood, bone marrow-derived cells, spleen cells, and the like are collected as the antibody-producing cells.
(4b) cDNA is synthesized from mRNA extracted from the antibody-producing cells by a reverse transcription reaction.
(5b) A PCR reaction is performed using cDNA as a template, and a DNA fragment containing a gene encoding a variable region of a heavy chain antibody is amplified.
(6b) The DNA fragment encoding a variable region of a heavy chain antibody is incorporated into a phagemid, this phagemid is introduced into host cells, and then the host cells are cultured to release phages having the variable region of the heavy chain antibody on the capsid surface into the culture medium.
(7b) A solution containing collected phages is loaded into a column packed with a carrier retaining rhTfR, and the phages having the variable region of a heavy chain antibody having affinity for rhTfR on the capsid surface are allowed to bind to the column. After washing the column, the phages bound to hTfR are eluted from the column. Alternatively, the solution containing collected phages is added onto a plate retaining rhTfR, and the phages having the variable region of a heavy chain antibody having affinity for rhTfR on the capsid surface are allowed to bind to the plate.
(8b) Only the phages that display the variable region of the heavy chain antibody which recognizes hTfR retained on the column or plate are collected, and a gene encoding the variable region of the heavy chain antibody which recognizes hTfR is amplified by a PCR reaction using the phagemid contained in those phages as a template.
(9b) The amplified gene is incorporated into an expression vector, and host cells are transformed using this expression vector to obtain cells expressing the variable region of the heavy chain antibody which recognizes hTfR.
(10b) The cells expressing the anti-hTfR heavy chain antibody variable region peptide are cultured to allow the cells to express a recombinant anti-hTfR heavy chain antibody variable region peptide in the culture fluid or in the cells.
(11b) The expressed recombinant anti-hTfR heavy chain antibody variable region peptide is purified.

In the above-described steps (1b) to (3b) of the phage display method, it is necessary to take out antibody-producing cells from an organism immunized with rhTfR. Instead of this, mRNA obtained in advance from antibody-producing cells of an organism capable of expressing a heavy chain antibody, or a DNA fragment synthesized using this mRNA as a template, can be pooled, and a DNA fragment containing a gene encoding the variable region of a heavy chain antibody can be amplified using the above-described mRNA or DNA fragment as a template. According to the phage display method, for example, since 1×10¹⁰ phages can be screened all at once, there is a possibility that phages displaying the variable region of a heavy chain antibody which recognizes hTfR may be selected even from non-immunized cells. In this case, it is not necessary to immunize an organism, and it is not necessary to take out antibody-producing cells from the organism.

The above-described step (7b) is intended for selecting phages that display the variable region of a heavy chain antibody which recognizes rhTfR. Here, the affinity of the selected phages for rhTfR can be adjusted by adjusting the conditions for causing the phages to bind to the column (or plate), and/or the conditions for washing the column (or plate). Furthermore, by repeatedly carrying out the step (7b) while making the conditions increasingly rigorous, phages having higher affinity for rhTfR are selected each time the step is repeated, and therefore, only the phages displaying the variable region of a heavy chain antibody having high affinity for rhTfR can be efficiently acquired.

Furthermore, during the process in which the phage genome is replicated, mutations may be introduced into the genome. Since this changes the affinity for rhTfR of the variable region of the heavy chain antibody encoded by the phage genome, when the above-described step (7) is repeatedly carried out, there is a possibility that mutations may be randomly introduced into the genome, and an antibody having higher affinity for rhTfR may be obtained.

The process in the case of repeating the above-described step (7b) is, for example, as shown in the following (7b').

(7b') A culture fluid containing collected phages is loaded into a column packed with a carrier retaining rhTfR, and phages displaying the variable region of a heavy chain antibody which recognizes rhTfR are allowed to bind to the column. Alternatively, the solution containing collected phages is applied onto a plate retaining rhTfR, and phages having the variable region of a heavy chain antibody having affinity for rhTfR on the capsid surface are allowed to bind to the plate. After washing the column or plate, phages displaying the variable region of a heavy chain antibody that recognizes hTfR are eluted from the column or plate. The eluted phages are used to infect host cells, subsequently the host cells are cultured, and phages displaying the variable region of the heavy chain antibody on the surface in the culture fluid are collected. This culture fluid containing the collected phages is loaded into a column chromatograph packed with a carrier retaining rhTfR, or is added onto a plate retaining rhTfR.

In the above-described step (7b'), a mutation can be actively introduced into the phage genome by bringing the phages into contact with a mutagen, or by adding a mutagen into the culture fluid of the host cells infected with the phages. A mutation can be actively introduced into the phage genome by irradiating the phages or the host cells infected with the phages, with electromagnetic waves such as ultraviolet radiation or gamma rays.

All of the above-described steps (1a) to (9a) and the above-described steps (1b) to (11b) can include a step of isolating a gene encoding the variable region of an anti-hTfR heavy chain antibody. It is also possible to translate the amino acid sequence containing the variable region of the anti-hTfR heavy chain antibody from the nucleotide sequence of the isolated gene encoding the anti-hTfR heavy chain antibody, and artificially synthesize a DNA fragment encoding this amino acid sequence. When artificially synthesizing a DNA fragment, it is possible to synthesized it by selecting appropriate codons for expressing the gene in host cells. By introducing an expression vector incorporating such a DNA fragment into host cells, the expression level of the anti-hTfR heavy chain antibody variable region peptide in the host cells can be increased. The expression level of the rhTfR affinity peptide can also be increased in the same manner.

Furthermore, the cells used as the host cells in the above-described steps are not particularly limited regardless of being prokaryotic cells or eukaryotic cells, as long as they are cells capable of expressing a peptide containing the variable region of a heavy chain antibody by introducing an expression vector in which a gene encoding the variable region of a heavy chain antibody has been incorporated, into the cells; however, Escherichia coli, CHO cells derived from Chinese hamster ovary, or NS/0 cells derived from mouse myeloma are particularly preferred. Furthermore, regarding the expression vector used for incorporating and expressing a gene encoding the variable region of a heavy chain antibody, any expression vector capable of expressing a peptide containing the variable region of the heavy chain antibody when introduced into host cells, can be used without particular limitation. The gene incorporated into the expression vector is disposed downstream of a DNA sequence capable of regulating the frequency of gene transcription (gene expression control site) in a host cell. Examples of a gene expression control site that can be used in the present invention include a promoter derived from cytomegalovirus, SV40 early promoter, human elongation factor 1α (EF-1α) promoter, and human ubiquitin C promoter.

The amino acid sequence of the variable region in the anti-hTfR heavy chain antibody variable region peptide obtained by the above-described steps includes four FRs and three CDRs, such as, from the N-terminus, the amino acid sequences of FR1, CDR1, FR2, CDR1, FR3, CDR1, and FR4. However, without being limited to this, as long as the amino acid sequence can specifically bind to hTfR, the amino acid sequence of the variable region may be one in which a part or the entirety of FR1 is deleted, one in which a part or the entirety of FR4 is deleted, or one in which a part or the entirety of FR1 is deleted, and a part or the entirety of FR4 is deleted. These peptides in which parts of the FR regions are deleted, are also included in the variable regions of anti-hTfR heavy chain antibodies. The same also applies to hTfR affinity peptides.

Suitable anti-hTfR heavy chain antibody variable region peptides according to an embodiment of the present invention are peptides having the amino acid sequences of hTfR affinity peptides 1 to 5. hTfR affinity peptides 1 to 6 have amino acid sequences set forth in SEQ ID NO:3, 4, 5, 6, 7, and 55, respectively. The amino acid sequences set forth in SEQ ID NO:3, 4, 5, 6, 7, and 55 are all amino acid sequences of the variable region. The hTfR affinity peptides 1 to 6 contain the amino acid sequences of four FRs and three CDRs, such as FR1, CDR1, FR2, CDR1, FR3, CDR1, and FR4, in order from the N-terminus. The hTfR affinity peptides 1 to 6 are all included in the hTfR affinity peptides.

CDR1 of the hTfR affinity peptide 1 is a region having the amino acid sequence of SEQ ID NO:8 or 9, CDR2 thereof is a region having the amino acid sequence of SEQ ID NO: 10 or 11, and CDR3 thereof is a region having the amino acid sequence of SEQ ID NO: 12 or 13. In the hTfR affinity peptide 1, the amino acid sequence of each of the CDRs can be arbitrarily combined. For example, the hTfR affinity peptide 1 is such that CDR1 is a region having the amino acid sequence of SEQ ID NO: 8, CDR2 is a region having the amino acid sequence of SEQ ID NO: 10, and CDR3 is a region having the amino acid sequence of SEQ ID NO: 12, or such that CDR1 is a region having the amino acid sequence of SEQ ID NO:9, CDR2 is a region having the amino acid sequence of SEQ ID NO: 11, and CDR3 is a region having the amino acid sequence of SEQ ID NO: 13. Furthermore, any of the CDRs may consist of these amino acid sequences.

CDR1 of the hTfR affinity peptide 2 is a region having the amino acid sequence of SEQ ID NO: 14 or 15, CDR2 thereof is a region having the amino acid sequence of SEQ ID NO: 16 or 17, and CDR3 thereof is a region having the amino acid sequence of SEQ ID NO: 18 or 19. In the hTfR affinity peptide 2, the amino acid sequence of each CDR can be arbitrarily combined. For example, the hTfR affinity peptide 2 is such that CDR1 is a region having the amino acid sequence of SEQ ID NO: 14, CDR2 is a region having the amino acid sequence of SEQ ID NO: 16, and CDR3 is a region having the amino acid sequence of SEQ ID NO: 18, or such that CDR1 is a region having the amino acid sequence of SEQ ID NO:15, CDR2 is a region having the amino acid sequence of SEQ ID NO: 17, and CDR3 is a region having the amino acid sequence of SEQ ID NO: 19. Furthermore, any of the CDRs may consist of these amino acid sequences.

CDR1 of the hTfR affinity peptide 3 is a region having the amino acid sequence of SEQ ID NO:20 or 21, CDR2 thereof is a region having the amino acid sequence of SEQ ID NO:22 or 23, and CDR3 thereof is a region having the amino acid sequence of SEQ ID NO:24 or 25. In the hTfR affinity peptide 3, the amino acid sequence of each of the CDRs can be arbitrarily combined. For example, the hTfR affinity peptide 3 is such that CDR1 is a region having the amino acid sequence of SEQ ID NO:20, CDR2 is a region having the amino acid sequence of SEQ ID NO:22, and CDR3 is a region having the amino acid sequence of SEQ ID NO:24, or such that CDR1 is a region having the amino acid sequence of SEQ ID NO:21, CDR2 is a region having the amino acid sequence of SEQ ID NO:23, and CDR3 is a region having the amino acid sequence of SEQ ID NO:25. Furthermore, any of the CDRs may consist of these amino acid sequences.

CDR1 of the hTfR affinity peptide 4 is a region having the amino acid sequence of SEQ ID NO:26 or 27, CDR2 thereof is a region having the amino acid sequence of SEQ ID NO:28 or 29, and CDR3 thereof is a region having the amino acid sequence of SEQ ID NO:30 or 31. In the hTfR affinity peptide 4, the amino acid sequence of each of the CDRs can be arbitrarily combined. For example, the hTfR affinity peptide 4 is such that CDR1 is a region having the amino acid sequence of SEQ ID NO:26, CDR2 is a region having the amino acid sequence of SEQ ID NO:28, and CDR3 is a region having the amino acid sequence of SEQ ID NO:30, or such that CDR1 is a region having the amino acid sequence of SEQ ID NO:27, CDR2 is a region having the amino acid sequence of SEQ ID NO:29, and CDR3 is a region having the amino acid sequence of SEQ ID NO:31. Furthermore, any of the CDRs may consist of these amino acid sequences.

CDR1 of the hTfR affinity peptide 5 is a region having the amino acid sequence of SEQ ID NO:20 or 21, CDR2 thereof is a region having the amino acid sequence of SEQ ID NO:22 or 23, and CDR3 thereof is a region having the amino acid sequence of SEQ ID NO:24 or 25. In the hTfR affinity peptide 5, the amino acid sequence of each of the CDRs can be arbitrarily combined. For example, the hTfR affinity peptide 5 is such that CDR1 is a region having the amino acid sequence of SEQ ID NO:20, CDR2 is a region having the amino acid sequence of SEQ ID NO:22, and CDR3 is a region having the amino acid sequence of SEQ ID NO:24, or such that CDR1 is a region having the amino acid sequence of SEQ ID NO:21, CDR2 is a region having the amino acid sequence of SEQ ID NO:23, and CDR3 is a region having the amino acid sequence of SEQ ID NO:25. Furthermore, any of the CDRs may consist of these amino acid sequences.

CDR1 of the hTfR affinity peptide 6 is a region having the amino acid sequence of SEQ ID NO:20 or 21, CDR2 thereof is a region having the amino acid sequence of SEQ ID NO: 56 or 57, and CDR3 thereof is a region having the amino acid sequence of SEQ ID NO:24 or 25. In the hTfR affinity peptide 6, the amino acid sequence of each of the CDRs can be arbitrarily combined. For example, the hTfR affinity peptide 6 is such that CDR1 is a region having the amino acid sequence of SEQ ID NO:20, CDR2 is a region having the amino acid sequence of SEQ ID NO: 56, and CDR3 is a region having the amino acid sequence of SEQ ID NO:24, or such that CDR1 is a region having the amino acid sequence of SEQ ID NO:21, CDR2 is a region having the amino acid sequence of SEQ ID NO: 57, and CDR3 is a region having the amino acid sequence of SEQ ID NO:25. Furthermore, any of the CDRs may consist of these amino acid sequences.

Furthermore, the amino acid sequence of the variable region of the above-described hTfR affinity peptide may have mutation applied thereto, as long as the variable region can specifically bind to hTfR. In a case where such mutation is applied, the amino acid sequence of the variable region of the hTfR affinity peptide after introducing the mutation preferably has the amino acid sequence identity of 80% or higher, more preferably the amino acid sequence identity of 85% or higher, even more preferably the amino acid sequence identity of 90% or higher, and still more preferably the amino acid sequence identity of 95% or higher, and has the amino acid sequence identity of, for example, 98% or higher to the original amino acid sequence.

Furthermore, when mutation is applied to the amino acid sequence of the variable region, mutation may be applied only to the FR region without having mutation applied to the amino acid sequences of CDR1, CDR2, and CDR3. When such mutation is applied, the amino acid sequence of the variable region after introducing the mutation preferably has the amino acid sequence identity of 85% or higher, more preferably the amino acid sequence identity of 90% or higher, and even more preferably the amino acid sequence identity of 95% or higher, and has the amino acid sequence identity of, for example, 98% or higher to the original amino acid sequence. Furthermore, when mutation is applied to the amino acid sequence of the variable region, mutation may be applied only to the CDR regions without having mutation applied to the amino acid sequence of the FR region. When such mutation is applied, the amino acid sequence of the variable region after introducing the mutation preferably has the amino acid sequence identity of 90% or higher, and more preferably the amino acid sequence identity of 95% or higher, and has the amino acid sequence identity of 98% or higher to the original amino acid sequence.

The amino acid sequence identity between the amino acid sequence of the original variable region and the variable region to which mutation has been applied can be easily calculated using well-known homology calculation algorithms. Examples of such algorithms include BLAST (Altschul SF, J Mol. Biol. 215, 403-10, (1990)), Pearson and Lipman's Similarity Search Method (Proc. Natl. Acad. Sci. USA. 85.2444 (1988)), and Smith and Waterman's Local Homology Algorithm (Adv. Appl. Math. 2. 482-9 (1981)). Furthermore, the term the amino acid sequence identity as used through the present specification refers to the amino acid sequence identity calculated by these algorithms. In the present specification, the terms homology of the amino acid sequence and the amino acid sequence identity are used interchangeably.

A case in which mutations such as substitution, deletion, and addition are applied to the amino acid sequence of the variable region of the hTfR affinity peptide, will be described in detail below.

In a case where amino acids in the amino acid sequence of the variable region of the hTfR affinity peptide are substituted with other amino acids, the number of amino acids to be substituted is preferably 1 to 20, more preferably 1 to 15, even more preferably 1 to 10, and still more preferably 1 to 5, and the number is, for example, 1, 2, or 3. In a case where amino acids in the amino acid sequence of the variable region are deleted, the number of amino acids to be deleted is preferably 1 to 20, more preferably 1 to 15, even more preferably 1 to 10, and still more preferably 1 to 5, and the number is, for example, 1, 2, or 3. Furthermore, it is also possible to apply mutations combining these substitutions and deletions of amino acids. In a case where amino acids are added to the variable region, preferably 1 to 20, more preferably 1 to 15, even more preferably 1 to 10, and still more preferably 1 to 5 amino acids, for example, 1, 2, or 3 amino acids, are added in the amino acid sequence of the variable region or at the N-terminus or the C-terminus thereof. It is also possible to apply mutations combining these additions, substitutions, and deletions of amino acids.

When mutations such as substitution, deletion, and addition are applied to the amino acid sequence of the variable region of the hTfR affinity peptide, mutations can be applied only to the FR region without applying mutations to the amino acid sequences of the CDR regions (CDR1, CDR2, and CDR3). The number of amino acids to be substituted when applying substitution only to the FR region is preferably 1 to 12, more preferably 1 to 10, even more preferably 1 to 8, and still more preferably 1 to 4, and the number is, for example, 1, 2, or 3. Furthermore, when deleting amino acids only from the amino acid sequence of the FR region, the number of amino acids to be deleted is preferably 1 to 8, more preferably 1 to 4, even more preferably 1 to 3, and still more preferably 1 to 2, and the number is, for example, 1 or 2. It is also possible to apply mutations combining these substitutions and deletions of amino acids. Furthermore, when amino acids are added only to the amino acid sequence of the FR region, preferably 1 to 8, more preferably 1 to 4, even more preferably 1 to 3, and still more preferably 1 to 2 amino acids, for example, 1 or 2 amino acids, are added in the amino acid sequence of the variable region or at the N-terminus or the C-terminus thereof. It is also possible to apply mutations combining these additions, substitutions, and deletions of amino acids.

When mutations are applied only to the FR region, a method of substituting the amino acid residues of the FR region of the hTfR affinity peptide with the amino acid residues corresponding to the FR region in the variable region of a human antibody of IgG type, is known. In the present specification, this method is referred to as humanization of hTfR affinity peptide. Such a method is disclosed in, for example, Vincle C., et. Al, J Biol Chem. 284. 3273-84 (2009). When the original antibody is an antibody of alpaca, there is a possibility that the antibody may be recognized as an antigen when administered into a human. A humanized antibody is expected to have low antigenicity as compared with the original antibody. When mutation is applied only to the FR region, humanization is one of preferred embodiments. According to the present specification, humanized hTfR affinity peptides are also to be included in the hTfR affinity peptide.

When mutations such as substitution, deletion, and addition are applied to the amino acid sequence of the variable region of the hTfR affinity peptide, mutations may be applied only to the CDR regions without applying mutations to the amino acid sequence of the FR region. The number of amino acids to be substituted when substitution is applied only to the CDR regions is preferably 1 to 4, more preferably 1 to 3, and even more preferably 1 to 2, and the number is, for example, 1 or 2. Furthermore, when amino acids are deleted only from the amino acid sequence of the CDR regions, the number of amino acids to be deleted is preferably 1 to 4, more preferably 1 to 3, and even more preferably 1 to 2, and the number is, for example, 1 or 2. It is also possible to apply mutations combining these substitutions and deletions of amino acids. Furthermore, when amino acids are added only to the amino acid sequence of the FR region, preferably 1 to 4, more preferably 1 to 3, and even more preferably 1 to 2 amino acids, for example, 1 or 2 amino acids, are added in the amino acid sequence of the variable region or at the N-terminus or the C-terminus thereof. It is also possible to apply mutations combining these additions, substitutions, and deletions of amino acids.

Substitution of an amino acid in the amino acid sequence of the above-described variable region with another amino acid takes place, for example, within a family of amino acids that are related to each other in their side chains and the chemical properties. It is predicted that such substitution within an amino acid family does not bring about significant change in the function of the anti-hTfR heavy chain antibody (that is, conservative amino acid substitution). Such amino acid families include, for example, the following:
(1) aspartic acid and glutamic acid, which are acidic amino acids,
(2) histidine, lysine, and arginine, which are basic amino acids,
(3) phenylalanine, tyrosine, and tryptophan, which are aromatic amino acids,
(4) serine and threonine, which are amino acids having a hydroxyl group (hydroxyamino acids),
(6) cysteine, serine, threonine, asparagine, and glutamine, which are neutral hydrophilic amino acids,
(7) glycine and proline, which are amino acids affecting the orientation of peptide chains,
(8) asparagine and glutamine, which are amide-type amino acids (polar amino acids)
(9) alanine, leucine, isoleucine, and valine, which are aliphatic amino acids,
(10) alanine, glycine, serine, and threonine, which are amino acids having a small side chain,
(11) alanine and glycine, which are amino acids having particularly a small side chain,
(12) valine, leucine, and isoleucine, which are amino acids having a branched chain.

The above-described conservative amino acid substitution is also applied to the case where an amnio acid in the amino acid sequence of another protein such as human serum albumin (HSA) with another amino acid.

With regard to the case of applying a mutation to the variable region and adding an amino acid at the C-terminus or the N-terminus, in a case where the hTfR affinity peptide is fused with different protein (A), and the added amino acid is positioned between the hTfR affinity peptide and the different protein (A), the added amino acid is considered to constitute a part of the hTfR affinity peptide. With regard to the fusion protein of the hTfR affinity peptide and the different protein (A), a linker located between the hTfR affinity peptide and the different protein (A) will be described in detail later. In the present specification, the term "different protein (A)" refers to a protein other than the hTfR affinity peptide, which needs to be administered to a human in a form of being bound to the hTfR affinity peptide and exert physiological activity in the human body.

The hTfR affinity peptide selected by the above-described steps and the like has certain affinity for hTfR. By introducing mutation such as substitution, deletion, or addition into the amino acid sequence of the variable region of this hTfR affinity peptide, the variable region can be modified into the variable region of an anti-hTfR heavy chain antibody having desired characteristics. Introduction of mutation into the amino acid sequence of the variable region of an anti-hTfR heavy chain antibody is carried out by applying mutation to a gene corresponding to the amino acid sequence.

In the variable region of the hTfR affinity peptide, the affinity for hTfR can be appropriately adjusted by applying mutation such as substitution, deletion, or addition to the amino acid sequence constituting the variable region. For example, in a case where the hTfR affinity peptide has high affinity for an antigen thereof and has a notably low dissociation constant in an aqueous liquid, when this is administered into a living body, there is a possibility that the variable region may not dissociate from the antigen, and as a result, functional problems may occur. In such a case, by introducing mutations to the variable region, the dissociation constant can be adjusted stepwise to 2 to 5 times, 5 to 10 times, or 10 to 100 times, compared to the dissociation constant of the original variable region, and the most preferable hTfR affinity peptide suited to the purpose can be acquired. On the contrary, the dissociation constant can also be adjusted stepwise to 1/2 to 1/5 times, 1/5 to 1/10 times, or 1/10 to 1/100 times compared to the dissociation constant of the original variable region by introducing the mutations.

The binding affinity of the hTfR affinity peptide to transferrin receptor, that is, the dissociation constant, can be measured using, for example, a method using biolayer interferometry (BLI), specifically an Octet system. The entirety or a part of the transferrin receptor derived from an appropriate animal is immobilized on a sensor, the binding rate constant (Kon) and the dissociation rate constant (Koff) are measured using a solution containing the variable region of the hTfR affinity peptide as a sample, and the dissociation constant can be calculated from the measurement results. Similarly, a method using surface plasmon resonance (SPR) technology, specifically, a method of using Biacore, can also be used.

Introduction of mutations such as substitution, deletion, and addition into the amino acid sequence of the variable region of the hTfR affinity peptide can be carried by, for example, using a gene encoding the variable region of the anti-hTfR heavy chain antibody as a template, and introducing mutations to specific sites of the nucleotide sequence of a gene by methods such as PCR, or randomly introducing mutations.

Introduction of mutation into the amino acid sequence of the hTfR affinity peptide for the purpose of adjusting the affinity of the hTfR affinity peptide to hTfR can be carried out by, for example, incorporating a gene encoding the hTfR affinity peptide into a phagemid, producing phages that express the hTfR affinity peptide on the capsid surface using this phagemid, and allowing a mutagen or the like to act on the phages. The phages can be expanded while mutation is introduced into the gene encoding the hTfR affinity peptide using a mutagen, and from the expanded phages, phages expressing a single-chain antibody having a desired dissociation constant can be selected by the above-described method.

The hTfR affinity peptide having relatively high affinity for hTfR, which is obtained by the above-described method, is preferably such that the dissociation constant (K_{D}) with hTfR as measured by the method described in Example 7 is preferably 5×10⁻⁸ M or less, more preferably 2×10⁻⁸ M or less, and for example, 1×10⁻⁸ M or less, 5×10⁻⁹ M or less, or 1×10⁻⁹ M or less. Examples of a suitable hTfR affinity peptide include those having a dissociation constant of 5×10⁻¹¹ M to 1×10⁻⁸ M, those having a dissociation constant of 2×10⁻¹¹ M to 1×10⁻⁸ M, and those having a dissociation constant of 1×10⁻¹⁰ M to 1×10⁻⁸ M.

By selecting an hTfR affinity peptide having affinity for monkey TfR from the hTfR affinity peptides obtained as described above, a peptide having affinity for both human TfR and monkey TfR can be obtained. An hTfR affinity peptide having affinity for monkey TfR can be selected by, for example, an ELISA method using a recombinant monkey TfR produced using genetic recombination technology. In this ELISA method, a recombinant monkey TfR is solid-phased on a plate, hTfR affinity peptides are brought into contact with the TfR, subsequently those peptides that are not bound to the recombinant monkey TfR are removed from the plate, and the amount of the hTfR affinity peptides retained on the plate is measured. As the amount of hTfR affinity peptides retained on the plate increases when the affinity for the recombinant monkey TfR is higher, therefore, hTfR affinity peptides corresponding to the plate on which a larger amount of hTfR affinity peptides is retained, can be selected as hTfR affinity peptides having affinity for monkey TfR. The term "monkey" as used herein preferably refers to a monkey classified as an anthropoid monkey except for human, more preferably a monkey classified under the Cercopithecidae family, and even more preferably a monkey classified under the genus Macaca, and the monkey is, for example, a cynomolgus monkey or a rhesus monkey, and particularly a cynomolgus monkey. Cynomolgus monkeys are convenient for evaluating the efficacy of a drug that utilizes an hTfR affinity peptide. By carrying out similar steps for the anti-hTfR heavy chain antibody variable region peptide, an hTfR affinity peptide having affinity for both human TfR and monkey TfR can be obtained.

An hTfR affinity peptide having affinity for both human hTfR and monkey hTfR has an advantageous effect that the pharmacokinetics to be obtained when the hTfR affinity peptide be administered in the human body can be observed using monkeys. For example, when a medicine is developed by utilizing the hTfR affinity peptide of an embodiment of the present invention, since some of the non-clinical trials such as pharmacokinetic tests of the medicine can be carried out using monkeys, development of the medicine can be significantly promoted. The same can also be said for the anti-hTfR heavy chain antibody variable region peptide.

According to the present invention, an hTfR affinity peptide having relatively high affinity for hTfR and also having affinity for monkey TfR has the following dissociation constants with human TfR and monkey TfR, particularly when measured by the method described in Example 7:
(a) an affinity peptide whose dissociation constants with hTfR and monkey TfR are both 5×10⁻⁹ M to 1×10⁻⁷ M,
(b) an affinity peptide whose dissociation constants with hTfR and monkey TfR are both 5×10⁻⁹ M to 5×10⁻⁸ M,
(d) an affinity peptide whose dissociation constants with hTfR and monkey TfR are both 1×10⁻¹ M to 3×10⁻¹ M,
(e) an affinity peptide whose dissociation constants with hTfR and monkey TfR are both 1×10⁻⁹ M to 2×10⁻⁷ M,
(f) an affinity peptide whose dissociation constants with hTfR and monkey TfR are both 5×10⁻⁹ M to 5×10⁻⁷ M.

Particularly, in the above items (a) to (f), the ratio of the dissociation constant with hTfR to the dissociation constant with monkey TfR is such that when the value of the dissociation constant with hTfR is taken as 1, the value of the dissociation constant with monkey TfR is preferably 0.3 to 3.0, and preferably, for example, 0.5 to 2.5, 0.8 to 2.5, 0.5 to 2.5, or 0.8 to 1.5.

With regard to the hTfR affinity peptides 1 to 5 according to an embodiment of the present invention, since the dissociation constants with human TfR and monkey TfR correspond to any one of the above-described items (a) to (e), it can be said that when these hTfR affinity peptides are administered to monkeys, the behavior thereof in the monkey bodies approximates the behavior when administered to humans. Therefore, for drugs that utilize the hTfR affinity peptides 1 to 5, the tests using monkey give results more closely reflecting the behavior of the drugs when administered to humans. Therefore, more useful results as a guidance for conducting clinical tests using humans may be obtained by the tests using monkeys. Particularly, the hTfR affinity peptides 1 and 3 to 5 are suitable because the ratio of the dissociation constant with hTfR to the dissociation constant with monkey TfR is such that when the value of the dissociation constant with hTfR is taken as 1, the value of the dissociation constant with monkey TfR is in the range of 0.5 to 1.5, and therefore, the behavior in the human bodies can be easily speculated from the behavior in the monkey bodies.

The hTfR affinity peptide according to an embodiment of the present invention can efficiently bind, when administered into a living body by intravenous injection or the like, to hTfR present on the endothelial cells of capillaries in the brain. The hTfR affinity peptide bound to hTfR passes through the blood brain barrier by a mechanism such as endocytosis, exocytosis, or transcytosis, and then is taken up into the brain. Therefore, by binding a protein, a low molecular weight compound, and the like, which need to be allowed to function in the brain, to hTfR affinity peptides, these substances can be efficiently allowed to pass through the blood brain barrier and reach the brain. Furthermore, the hTfR affinity peptide according to an embodiment of the present invention is predicted to reach the cerebral brain parenchyma, hippocampal neuronal-like cells, and cerebellar brain parenchyma, or at least any one of these, after passing through the blood brain barrier. Therefore, by binding drugs, specifically a protein, a low molecular weight compound, a nucleic acid, and the like, which need to be allowed to act on these tissues or cells, to the hTfR affinity peptides of the present invention, these drugs can be allowed to reach these tissues or cells. A product in which an hTfR affinity peptide and a drug are bound in this way is referred to an hTfR affinity peptide-drug complex in the present specification. The same can also be said for the anti-hTfR heavy chain antibody variable region peptide.

In regard to allowing substances (a protein, a low molecular weight compound, a nucleic acid, and the like) that are normally unable to pass through the blood brain barrier and can therefore hardly or not at all exert physiological or pharmacological action in the brain when intravenously administered, to reach the brain from the blood and exert its action there, forming complexes with the hTfR affinity peptide according to an embodiment of the present invention may be an effective means. Particularly, the hTfR affinity peptide of the present invention is predicted to reach the cerebral brain parenchyma, hippocampal neuronal-like cells, cerebellar brain parenchyma, and the like, or at least any one of these after passing through the blood brain barrier. Therefore, by administering those substances into the blood by intravenous administration or the like in a form of being bound to the hTfR affinity peptide of the present invention, it becomes possible for those substances to pass through the blood brain barrier, reach the central nervous tissues, and have their physiological activity exerted or enhanced in the tissues or cells of the central nervous system. The same can also be said for the anti-hTfR heavy chain antibody variable region peptide.

Regarding a method of binding hTfR affinity peptides with those substances (a protein, a low molecular weight, a nucleic acid, and the like), there is a method of binding by means of a non-peptide linker or a peptide linker. The same can also be said for the anti-hTfR heavy chain antibody variable region peptide. As the non-peptide linker, polyethylene glycol (PEG), polypropylene glycol, a copolymer of ethylene glycol and propylene glycol, polyoxyethylated polyol, polyvinyl alcohol, a polysaccharide, dextran, polyvinyl ether, a biodegradable polymer, a lipid polymer, a chitin, and hyaluronic acid, or derivatives thereof, or combinations these can be used. A peptide linker is a peptide chain consisting of 1 to 50 amino acids linked by peptide bonds, or a derivative thereof, and as the N-terminus and the C-terminus thereof each form a covalent bond with either an hTfR affinity peptide or a protein, a low molecular weight compound, a nucleic acid, or the like, resulting in that an anti-hTfR antibody is bound to the protein, low molecular weight compound, nucleic acid, or the like.

A product in which the hTfR affinity peptide of the present invention is bound to desired different protein (A) using PEG as a non-peptide linker is particularly referred to as an hTfR affinity peptide-PEG-protein. The hTfR affinity peptide-PEG-protein can be produced by binding an hTfR affinity peptide to PEG to produce hTfR affinity peptide-PEG, and then binding the hTfR affinity peptide-PEG to the different protein (A). Alternatively, the hTfR affinity peptide-PEG-protein can also be produced by binding the different protein (A) to PEG to produce protein-PEG, and then binding the protein-PEG to an hTfR affinity peptide. When binding PEG to the hTfR affinity peptide and the different protein (A), a PEG modified with a functional group such as carbonate, carbonylimidazole, an active ester of a carboxylic acid, azlactone, cyclic imide thione, isocyanate, isothiocyanate, imidate, or aldehyde, is used. As these functional groups introduced into PEG react mainly with amino groups in the molecules of the hTfR affinity peptide and the different protein (A), PEG is covalently bonded to the hTfR affinity peptide and the different protein (A). There are no particular limitations on the molecular weight and the shape of the PEG used in this case; however, the average molecular weight (MW) thereof is preferably 500 to 60000, and more preferably 500 to 20000. For example, a PEG having an average molecular weight of about 300, about 500, about 1000, about 2000, about 4000, about 10000, about 20000, or the like can be suitably used as the non-peptide linker. The same also applies to the case where an hTfR affinity peptide is bound to a desired low molecular weight compound, nucleic acid, or the like.

For example, the hTfR affinity peptide-PEG is obtained by mixing an hTfR affinity peptide and a polyethylene glycol having an aldehyde group as a functional group (ALD-PEG-ALD) such that the molar ratio of ALD-PEG-ALD with respect to the antibody is 1:11, 1:12.5, 1:15, 1:110, 1:120, or the like, adding a reducing agent such as NaCNBH₃ to this mixture, and reacting the mixture. Next, the hTfR affinity peptide-PEG is reacted with the different protein (A) in the presence of the reducing agent such as NaCNBH₃, to obtain hTfR affinity peptide-PEG-protein. In contrast, the hTfR affinity peptide-PEG-protein can be obtained by first binding the different protein (A) to the ALD-PEG-ALD to produce protein-PEG, and then binding the protein-PEG to an hTfR affinity peptide.

The affinity hTfR affinity peptide and the different protein (A) can also bound by linking the C-terminus or the N-terminus of the hTfR affinity peptide to the N-terminus or the C-terminus of the different protein (A), respectively, via a peptide linker or directly. A fusion protein obtained by binding such an hTfR affinity peptide to different protein (A) can be obtained as a recombinant protein by incorporating a DNA fragment in which a cDNA encoding the different protein (A) is disposed in frame at the 3'-terminus or the 5'-terminus of a cDNA encoding the hTfR affinity peptide, directly or by interposing a DNA fragment encoding the amino acid sequence of a peptide linker, into an expression vector for eukaryotic cells such as mammalian cells or yeast, or prokaryotic cells such as Escherichia coli, and culturing host cells into which this expression vector has been introduced. In the present specification, such a recombinant protein in which an hTfR affinity peptide and different protein (A) are bound , is referred to as an hTfR affinity peptide-protein (A) fusion protein. Similarly, a recombinant protein in which an immunoglobulin single variable domain peptide and different protein (A) are bound , is referred to as an immunoglobulin single variable domain peptide-protein (A) fusion protein. Furthermore, a recombinant protein in which a heavy chain antibody variable region peptide and different protein (A) are bound , is referred to as a heavy chain antibody variable region peptide-protein (A) fusion protein.

In this case, the peptide linker disposed between the hTfR affinity peptide and the different protein (A) is a peptide chain consisting of preferably 1 to 50, more preferably 1 to 17, even more preferably 1 to 10, and still more preferably 1 to 5 amino acids; however, the number of amino acids constituting the peptide linker can be appropriately adjusted to 1, 2, 3, 1 to 17, 1 to 10, 10 to 40, 20 to 34, 23 to 31, 25 to 29, 27, or the like, depending on the different protein (A) to be bound to the anti-hTfR antibody. Such a peptide linker is not limited in the amino acid sequence thereof as long as the hTfR affinity peptide linked thereby has affinity for hTfR, and the different protein (A) linked by the peptide linker can exhibit desired physiological activity under physiological conditions; however, preferably, the amino acid sequence is composed of glycine and serine.

Examples of a preferred peptide linker include peptide linkers consisting of one amino acid (for example, glycine or serine), and peptide linkers having the amino acid sequence Gly-Ser, the amino acid sequence Ser-Ser, the amino acid sequence Gly-Gly-Ser, the amino acid sequence of SEQ ID NO:32, an amnio acid sequence of SEQ ID NO:33, the amino acid sequence of SEQ ID NO:34, the amino acid sequence of SEQ ID NO:35, the amino acid sequences of SEQ ID NO:36, SEQ ID NO:58, and SEQ ID NO:69, and a sequence in which 2 to 10, or 2 to 5 of these amino acid sequences are consecutively linked. Such a peptide linker has a sequence consisting of 2 to 50 amino acids, or a sequence consisting of 2 to 17, 2 to 10, 10 to 40, 20 to 34, 23 to 31, 25 to 29, or 27 amino acids. For example, a peptide having the amino acid sequence Gly-Ser can be suitably used as a peptide linker. Furthermore, a peptide having a total of 25 amino acid sequences consisting of five amino acid sequences of SEQ ID NO:32 consecutively linked can also be suitably used as a peptide linker.

The hTfR affinity peptide can also be bound to the Fc region of human IgG (hIgG Fc region). By binding the hIgG Fc region to the hTfR affinity peptide, in vivo stability of the hTfR affinity peptide, for example, retention in blood, can be increased. Examples of such an hTfR affinity peptide having the hIgG Fc region bound thereto include a product obtained by binding the hIgG Fc region to the C-terminus of the hTfR affinity peptide directly or via a peptide linker, or a product obtained by binding the hIgG Fc region to the N-terminus of the hTfR affinity peptide directly or via a peptide linker. A conjugate obtained by binding an Fc region to the hTfR affinity peptide can be further bound to a substance (a protein, a low molecular weight compound, a nucleic acid, or the like) that is normally unable to pass through the blood brain barrier and can therefore hardly or not at all exert physiological and pharmacological action in the brain when administered intravenously, to form a complex. Such a substance can be bound to the conjugate of an Fc region and an hTfR affinity peptide, by the above-described technique, for example, a technique using PEG. This complex is predicted to be able to pass through the blood brain barrier when administered into the living body by intravenous injection or the like, and reach the cerebral brain parenchyma, hippocampal neuronal-like cells, cerebellar brain parenchyma, and the like. Therefore, by forming the conjugate, the physiological activity of this substance can be exerted in the tissues or cells of the central nervous system. Furthermore, since the stability in blood of the complex is increased as compared with the stability in blood of a complex lacking the hIgG Fc region, the complex can exert drug efficacy over a longer period of time when administered into the living body. A protein in which an hTfR affinity peptide is bound to the Fc region of human IgG can be obtained as a recombinant protein, similarly to the hTfR affinity peptide-protein (A) fusion protein.

The hTfR affinity peptide, different protein (A), and the hIgG Fc region can also be bound. By binding the hIgG Fc region, the in vivo stability of the conjugate, for example, retention in blood, can be increased. Regarding such a protein having an Fc region bound thereto, for example, there is a protein in which the hIgG Fc region is bound to the C-terminus of the different protein (A) directly or via a peptide linker, and the hTfR affinity peptide is further bound to the C-terminus of the hIgG Fc region directly or via a peptide linker. There is also a protein in which the hIgG Fc region is bound to the C-terminus of the hTfR affinity peptide directly or via a peptide linker, and the different protein (A) is further bound to the C-terminus of the hIgG Fc region directly or via a linker sequence. Furthermore, there is also a protein in which the different protein (A) is bound to the C-terminus of the hTfR affinity peptide directly or via a peptide linker, and the hIgG Fc region is further bound to the C-terminus of the different protein (A) directly or via a linker sequence. There is also a protein in which the hTfR affinity peptide is bound to the C-terminus of the different protein (A) directly or via a peptide linker, and the hIgG Fc region is further bound to the C-terminus of the hTfR affinity peptide directly or via a linker sequence. Furthermore, there is also a protein in which the hTfR affinity peptide is bound to the C-terminus of the hIgG Fc region directly or via a peptide linker, and the different protein (A) is further bound to the C-terminus of the hTfR affinity peptide directly or via a linker sequence. There is also a protein in which the different protein (A) is bound to the C-terminus of the hIgG Fc region directly or via a peptide linker, and the hTfR affinity peptide is further bound to the C-terminus of the different protein (A) directly or via a linker sequence. A protein in which the hTfR affinity peptide, the different protein (A), and the hIgG Fc region are bound can be obtained as a recombinant protein, similarly to the hTfR affinity peptide-protein(A) fusion protein.

The type of IgG of the hIgG Fc region to be bound is not particularly limited, and any of IgG1 to IgG5 may be used. The hIgG Fc region to be introduced may be the entire Fc region or may be a part thereof. A preferred embodiment of such an hIgG Fc region may be one having the amino acid sequence set forth in SEQ ID NO:37, which is the entire region of Fc of human IgG1.

The hTfR affinity peptide may also be bound to human serum albumin (HSA). By binding HSA to the hTfR affinity peptide, in vivo stability of the hTfR affinity peptide, for example, retention in blood, can be increased. Regarding such an hTfR affinity peptide having HSA bound thereto, for example, there is a product in which HSA is bound to the C-terminus of the hTfR affinity peptide directly or via a peptide linker, or a product in which HSA is bound to the N-terminus of the hTfR affinity peptide directly or via a peptide linker. A conjugate in which HSA is bound to the hTfR affinity peptide can be further bound to a substance (a protein, a low molecular weight compound, a nucleic acid, or the like) that is normally unable to pass through the blood brain barrier and can therefore hardly or not at all exert physiological and pharmacological action in the brain when administered intravenously, to form a complex. Such a substance can be bound to the conjugate of HSA and the hTfR affinity peptide by the above-described techniques, for example, a technique using PEG. This complex is predicted to be able to pass through the blood brain barrier when administered into the living body by intravenous injection or the like and reach the cerebral brain parenchyma, hippocampal neuronal-like cells, cerebellar brain parenchyma, and the like. Therefore, by forming the conjugate, the physiological activity of this substance can be exerted in the tissues or cells of the central nervous system. Furthermore, since the stability in blood of the complex is increased as compared with the stability in blood of a complex lacking HSA, the complex can exert drug efficacy over a longer period of time when administered into the living body. A protein in which an hTfR affinity peptide is bound to HSA can be obtained as a recombinant protein, similarly to the hTfR affinity peptide-protein (A) fusion protein.

HSA may be further bound to the hTfR affinity peptide-protein (A) fusion protein. Regarding such a protein having HSA bound thereto, for example, there is a protein in which HSA is bound to the C-terminus of the different protein (A) directly or via a peptide linker, and the hTfR affinity peptide is further bound to the C-terminus of the HSA directly or via a peptide linker. Furthermore, there is a protein in which HSA is bound to the C-terminus of the hTfR affinity peptide directly or via a peptide linker, and the different protein (A) is further bound to the C-terminus of the HSA directly or via a linker sequence. Furthermore, there is also a protein in which the different protein (A) is bound to the C-terminus of the hTfR affinity peptide directly or via a peptide linker, and HSA is further bound to the C-terminus of the different protein (A) directly or via a linker sequence. Furthermore, there is also a protein in which the hTfR affinity peptide is bound to the C-terminus of the different protein (A) directly or via a peptide linker, and HSA is further bound to the C-terminus of the hTfR affinity peptide directly or via a linker sequence. There is also protein in which the hTfR affinity peptide is bound to the C-terminus of HSA directly or via a peptide linker, and the different protein (A) is further bound to the C-terminus of the hTfR affinity peptide directly or via a linker sequence. Furthermore, there is also a protein in which the different protein (A) is bound to the C-terminus of HSA directly or via a peptide linker, and the hTfR affinity peptide is further bound to the C-terminus of the different protein (A) directly or via a linker sequence. A protein in which hTfR immunoglobulin single variable domain peptide, different protein (A), and HSA are bound can be obtained as a recombinant protein, similarly to the hTfR immunoglobulin single variable domain peptide-protein (A) fusion protein. A recombinant protein obtained in this manner is also included in the hTfR affinity peptide-protein (A) fusion protein.

The above-described HSA is preferably wild-type HSAhaving the amino acid sequence set forth in SEQ ID NO:38; however, without being limited to this, the HSA may also be a mutant-type HSA obtained by applying substitution, deletion, or addition into the amino acid sequence of wild-type HSA, as long as the stability in blood of a substance bound thereto can be improved.

Instead of HSA, a substance having affinity for albumin can also be used. Since a substance having affinity for albumin binds to albumin in blood, the half-life in blood of a fusion protein of an anti-hTfR heavy chain antibody and different protein (A) or the like that has been administered into the living body can be increased as in the case of using HSA. As the substance having affinity for albumin, for example, a peptide obtained by modifying the albumin-binding domain of a protein derived from Streptococcus strain G418, which has the amino acid sequence set forth in SEQ ID NO:39 (Alm T. Biotechnol J. 5. 605-17 (2010)), so as to exhibit alkali resistance, can be used.

The binding affinity for albumin of the hTfR affinity peptide-protein (A) fusion protein into which an albumin affinity peptide has been introduced, is preferably 1×10⁻⁷ M or less, more preferably 5×10⁻⁷ M or less, even more preferably 1×10⁻⁸ M or less, and still more preferably 1×10⁻⁹ M or less, when measured by the biolayer interferometry described in Example 7.

A plurality of hTfR affinity peptides may be linked directly or via peptide linkers. In this case, the plurality of hTfR affinity peptides to be bound may all have the same amino acid sequence or may have different amino acid sequences. There is no particular limitation on the number of hTfR affinity peptides to be bound; however, for example, the number is 2 to 10, 2 to 5, 2, or 3. By binding a plurality of hTfR affinity peptides, properties such as improved affinity for anti-hTfR can be imparted. In the present specification, a product obtained by linking a plurality of hTfR affinity peptides in this way is referred to as a tandem hTfR affinity peptide. The tandem hTfR affinity peptide is included in the hTfR affinity peptide. The same can also be said for the anti-hTfR heavy chain antibody variable region peptide.

A peptide linker used to link the above-described hTfR affinity peptide to the hIgG Fc region or HSA is preferably consisting of 1 to 50 amino acids. Here, the number of amino acids is appropriately adjusted to 1 to 17, 1 to 10, 10 to 40, 20 to 34, 23 to 31, 25 to 29, 27, or the like. There is no limitation on the amino acid sequence of such a peptide linker as long as the hTfR affinity peptide linked thereby has affinity for hTfR, and the hIgG Fc region or HSA linked by the peptide linker can exert desired functions under physiological conditions; however, preferably, the amino acid sequence is composed of glycine and serine. The same also applies to the peptide linker used to bind the hTfR affinity peptide, the different protein (A), and the hIgG Fc region. The same also applies to the peptide linker used to bind the hTfR affinity peptide, the different protein (A), and HSA. Furthermore, the same also applies to the peptide linker used to bind a plurality of hTfR affinity peptides.

Examples of a preferred peptide linker include a peptide linker consisting of one amino acid, either glycine or serine, and peptide linkers having the amino acid sequence Gly-Ser, the amino acid sequence Ser-Ser, the amino acid sequence Gly-Gly-Ser, the amino acid sequences of SEQ ID NO:32, SEQ ID NO:33, SEQ ID NO:34, SEQ ID NO:35, SEQ ID NO:36, SEQ ID NO:58, and SEQ ID NO:69, and a sequence having 2 to 10, or 2 to 5 of these amino acid sequences consecutively linked . Such a peptide linker has a sequence consisting of 2 to 50 amino acids, or a sequence consisting of 2 to 17, 2 to 10, 10 to 40, 20 to 34, 23 to 31, 25 to 29, or 27 amino acids. For example, a peptide linker having the amino acid sequence Gly-Ser can be suitably used as a peptide linker. Furthermore, a peptide linker having a total of 25 amino acid sequences consisting of five amino acid sequences of SEQ ID NO:32 consecutively linked can also be suitably used as a peptide linker.

The hTfR affinity peptide and the hTfR affinity peptide-protein (A) fusion protein can also be stabilized in blood by a method other than the above-described methods. For example, the stability in blood can be increased by modifying these proteins with PEG. Such a technique is generally implemented in the field of protein medicines, and PEGylated erythropoietin, interferon, and the like have been put to practical use as pharmaceutical products. Furthermore, the hTfR affinity peptide can also be stabilized by introducing a mutation thereinto.

There is no particular limitation on the different protein (A) to be bound to the hTfR affinity peptide; however, since the different protein (A) is a protein that can exert physiological activity in the living body, and particularly a protein that should be allowed to reach the brain to exert its function but nevertheless cannot be expected to function in the brain when administered intravenously because the protein cannot pass through the blood brain barrier as it is. Examples of such a protein include lysosomal enzymes such as iduronate-2-sulfatase (IDS), glucocerebrosidase (GBA), β-galactosidase, GM2 activator protein, β-hexosaminidase A, β-hexosaminidase B, N-acetylglucosamine-1-phosphotransferase, α-mannosidase (LAMAN), β-mannosidase, galactosylceramidase (GALC), saposin C, arylsulfatase A (ARSA), α-L-fucosidase (FUCA1), aspartylglucosaminidase, α-N-acetylgalactosaminidase, acidic sphingomyelinase (ASM), α-galactosidase A, β-glucuronidase (GUSB), heparan-N-sulfatase (SGSH), α-N-acetylglucosaminidase (NAGLU), acetyl CoA α-glucosaminide N-acetyltransferase, N-acetylglucosamine-6-sulfate sulfatase, acidic ceramidase (AC), amylo-1,6-glucosidase, sialidase, aspartylglucosaminidase, palmitoyl protein thioesterase-1 (PPT-1), tripeptidyl peptidase-1 (TPP-1), hyaluronidase-1, acidic α-glucosidase (GAA), CLN1, and CLN2. The same can also be said for the anti-hTfR heavy chain antibody variable region peptide.

An hTfR affinity peptide bound to α-L-iduronidase (IDUA) can be used as a therapeutic agent for Hurler syndrome or Hurler-Scheie syndrome, an hTfR affinity peptide bound to iduronate-2-sulfatase (IDS) can be used as a therapeutic agent for Hunter syndrome, an hTfR affinity peptide bound to glucocerebrosidase (GBA) can be used as a therapeutic agent for Gaucher disease, an hTfR affinity peptide bound to β-galactosidase can be used as a therapeutic agent for GM1-gangliosidosis types 1 to 3, an hTfR affinity peptide bound to GM2 activator protein can be used as a therapeutic agent for GM2-gangliosidosis AB variant, an hTfR affinity peptide bound to β-hexosaminidase A can be used as a therapeutic agent for Sandhoff disease and Tay-Sachs disease, an hTfR affinity peptide bound to β-hexosaminidase B can be used as a therapeutic agent for Sandhoff disease, an hTfR affinity peptide bound to N-acetylglucosamine-1-phosphotransferase can be used as a therapeutic agent for I-Cell disease, an hTfR affinity peptide bound to α-mannosidase (LAMAN) can be used as a therapeutic agent for α-mannosidosis, an hTfR affinity peptide bound to β-mannosidase can be used as a therapeutic agent for β-mannosidosis, an hTfR affinity peptide bound to galactosylceramidase (GALC) can be used as a therapeutic agent for Krabbe disease, an hTfR affinity peptide bound to saposin C can be used as a therapeutic agent for Gaucher-like storage disease, an hTfR affinity peptide bound to arylsulfatase A (ARSA) can be used as a therapeutic agent for metachromatic leukodystrophy, an hTfR affinity peptide bound to α-L-fucosidase (FUCA1) can be used as a therapeutic agent for fucosidosis, an hTfR affinity peptide bound to aspartylglucosaminidase can be used as a therapeutic agent for aspartylglucosaminuria, an hTfR affinity peptide bound to α-N-acetylgalactosaminidase can be used as a therapeutic agent for Schindler disease and Kawasaki disease, an hTfR affinity peptide bound to acidic sphingomyelinase (ASM) can be used as a therapeutic agent for Niemann-Pick disease, an hTfR affinity peptide bound to α-galactosidase A can be used as a therapeutic agent for Fabry disease, an hTfR affinity peptide bound to β-glucuronidase (GUSB) can be used as a therapeutic agent for Sly syndrome, an hTfR affinity peptide bound to heparan N-sulfatase (SGSH) can be used as a therapeutic agent for Sanfilippo syndrome A, an hTfR affinity peptide bound to α-N-acetylglucosaminidase (NAGLU) can be used as a therapeutic agent for Sanfilippo syndrome B, an hTfR affinity peptide bound to acetyl CoA α-glucosaminide N-acetyltransferase and an hTfR affinity peptide bound to N-acetylglucosamine-6-sulfate sulfatase can be used as therapeutic agents for Sanfilippo syndrome, an hTfR affinity peptide bound to acidic ceramidase (AC) can be used as a therapeutic agent for Farber disease, an hTfR affinity peptide bound to amylo-1,6-glucosidase can be used as a therapeutic agent for Cori disease (Forbes-Cori disease), an hTfR affinity peptide bound to sialidase can be used as a therapeutic agent for central nervous system disorders in sialidase deficiency, an hTfR affinity peptide bound to palmitoyl protein thioesterase-1 (PPT-1) can be used as a therapeutic agent for neuronal ceroid-lipofuscinosis or Santavuori-Haltia disease, an hTfR affinity peptide bound to tripeptidyl peptidase-1 (TPP-1) can be used as a therapeutic agent for neuronal ceroid-lipofuscinosis or Jansky-Bielschowsky disease, an hTfR affinity peptide bound to hyaluronidase-1 can be used as a therapeutic agent for hyaluronidase deficiency, an hTfR affinity peptide bound to acidic α-glucosidase (GAA) can be used as a therapeutic agent for Pompe disease, and hTfR affinity peptides bound to CLN1 and CLN2 can be each used as a therapeutic agent for Batten disease. hTfR affinity peptides bound to the above-described lysosomal enzymes can be utilized particularly as therapeutic agents for central nervous system disorders in lysosomal diseases. For example, an hTfR affinity peptide bound to α-L-iduronidase (IDUA) can be used as a therapeutic agent for central nervous system disorders in Hurler syndrome or Hurler-Scheie syndrome. Furthermore, the pharmaceutical use applications are not limited to these diseases.

In addition to those, examples of the protein that can be bound to an hTfR affinity peptide and exert drug efficacy include growth factors, antibody drugs, cytokines, nerve growth factor (NGF), brain-derived nerve growth factor (BDNF), ciliary neurotrophic factor (CNTF), glial cell line-derived neurotrophic factor (GDNF), neurotrophin 3, neurotrophin 4/5, neurotrophin 6, neuregulin 1, erythropoietin, darbepoetin, activin, basic fibroblast growth factor (bFGF), fibroblast growth factor 2 (FGF2), epidermal growth factor (EGF), vascular endothelial growth factor (VEGF), interferon α, interferon β, interferon γ, interleukin 6, granulocyte macrophage colony-stimulating factor (GM-CSF), granulocyte colony-stimulating factor (G-CSF), macrophage colony-stimulating factor (GM-CSF), granulocyte colony-stimulating factor (G-CSF), macrophage colony-stimulating factor (M-CSF), tumor necrosis factor α receptor (TNF-αR), PD-1 ligand, PD-L1, PD-L2, an enzyme having activity of degrading beta-amyloid, anti-beta amyloid antibody, anti-BACE antibody, anti-EGFR antibody, anti-PD-1 antibody, anti-PD-L1 antibody, anti-PD-L2 antibody, anti-HER2 antibody, anti-TNF-α antibody, and anti-CTLA-4 antibody.

An hTfR affinity peptide bound to nerve growth factor (NGF) can be used as a neurological disorder therapeutic agent for Alzheimer's disease, an hTfR affinity peptide bound to brain-derived growth factor (BDNF) can be used as a neurological disorder therapeutic agent for Huntington's disease, Alzheimer's disease, and the like, an hTfR affinity peptide bound to CNTF can be used as a therapeutic agent for amyotrophic lateral sclerosis, an hTfR affinity peptide bound to GDNF, neurotrophin 3, or neurotrophin 4/5 can be used as a therapeutic agent for cerebral ischemia, an hTfR affinity peptide bound to GDNF can be used as a therapeutic agent for Parkinson's disease, an hTfR affinity peptide bound to neuregulin 1 can be used as a therapeutic agent for schizophrenia, an hTfR affinity peptide bound to erythropoietin or darbepoietin can be used as a therapeutic agent for cerebral ischemia, an hTfR affinity peptide bound to bFGF or FGF2 can be used as a therapeutic agent for traumatic central nervous system disorders and for recovery after brain surgery or spinal surgery, an hTfR affinity peptide bound to an enzyme having an activity of degrading beta-amyloid, an anti-beta amyloid antibody, or an anti-BACE antibody can be used as a therapeutic agent for Alzheimer's disease, an hTfR affinity peptide bound to an anti-EGFR antibody, an anti-PD-1 antibody, an anti-PD-L1 antibody, an anti-PD-L2 antibody, an anti-HER2 antibody, or anti-CTLA-4 antibody can be used as a therapeutic agent for tumors in the central nervous system, including brain tumors, and an hTfR affinity peptide bound to TNFαR can be used as a therapeutic agent for cerebral ischemia and brain inflammatory diseases.

Therapeutic agents for diseases such as neurodegenerative diseases such as Alzheimer's disease, Parkinson's disease, and Huntington's disease, mental disorders such as schizophrenia and depression, multiple sclerosis, amyotrophic lateral sclerosis, tumors in the central nervous system including brain tumors, lysosomal diseases accompanied with brain damage, glycogen storage diseases, muscular dystrophy, cerebral ischemia, brain inflammatory diseases, prion diseases, and traumatic central nervous system disorders, may generally be the different protein (A) to be fused with the hTfR affinity peptide. Furthermore, therapeutic agents for viral and bacterial central nervous system disorders may also generally be the different protein (A) to be fused with the hTfR affinity peptide. In addition, drugs that can be used for recovery after brain surgery and spinal surgery may also generally be the different protein (A) to be fused with the hTfR affinity peptide.

Those used as therapeutic agents in the present invention can also be used to prevent the onset of diseases.

The proteins listed above as the different protein (A) to be bound to the hTfR affinity peptide are usually wild-type proteins. However, mutants in which one or a plurality of amino acids among the amino acids constituting these wild-type proteins have been substituted with other amino acids, deleted, or the like, are also included in these proteins as long as the mutants have the original physiological activity of the proteins. Here, a protein having its original physiological activity means that the protein has 20% or more of the physiological activity of the wild-type protein. The physiological activity of the protein with respect to the physiological activity of the wild-type protein is more preferably 40% or more, even more preferably 50% or more, still more preferably 80% or more, and even more preferably 90% or more.

The amino acid sequence of a mutant-type protein preferably has the amino acid sequence identity of 80% or more, more preferably has the amino acid sequence identity of 85% or more, even more preferably has the amino acid sequence identity of 90% or more, and still more preferably has the amino acid sequence identity of 95% or more, and for example, has the amino acid sequence identity of 98% or more.

In a case where the amino acid sequence of a wild-type different protein (A) is substituted with other amino acids to produce a mutant form, the number of amino acids to be substituted is preferably 1 to 10, more preferably 1 to 5, and even more preferably 1 to 3. In a case where amino acids are deleted, the number of amino acids to be deleted is preferably 1 to 10, more preferably 1 to 5, and even more preferably 1 to 3. Furthermore, a desired mutant form can be produced by combining these substitution and deletion of amino acids. Furthermore, products having one or a plurality of amino acids added in the amino acid sequence or at the N-terminus or C-terminus of the wild-type different protein (A) or a mutant thereof, are also included in these proteins as long as the products retain the functions of these proteins completely or partially. The number of amino acids to be added in this case is preferably 1 to 10, more preferably 1 to 5, and even more preferably 1 to 3. A desired mutant form can be produced by combining these addition, substitution, and deletion of amino acids.

In a case where mutation is applied in these different proteins (A) to add amino acids at the C-terminus or the N-terminus, and in a case where the added amino acids are located between these proteins and the hTfR affinity peptide when these proteins are fused with the hTfR affinity peptide, the added amino acids are considered to constitute a part of the different proteins (A).

When the different protein (A) is human IDS, a preferable fusion protein includes the following (1) to (4):
(1) a fusion protein in which the N-terminus of hIDS is bound to the C-terminus of an hTfR affinity peptide having the amino acid sequence of SEQ ID NO:3, SEQ ID NO:4, SEQ ID NO:5, SEQ ID NO:6, SEQ ID NO:7, or SEQ ID NO:55 via a peptide linker having the amino acid sequence of SEQ ID NO:59,
(2) a fusion protein having the amino acid sequence of SEQ ID NO:62, in which the N-terminus of hIDS is bound to the C-terminus of the hTfR affinity peptide 5 having the amino acid sequence of SEQ ID NO:7 via a peptide linker having the amino acid sequence of SEQ ID NO:59,
(3) a fusion protein having the amino acid sequence of SEQ ID NO:60, in which the N-terminus of hIDS is bound to the C-terminus of the hTfR affinity peptide 6 having the amino acid sequence of SEQ ID NO:55 via a peptide linker having the amino acid sequence of SEQ ID NO:59, and
(4) a fusion protein having the amino acid sequence of SEQ ID NO:64, in which the N-terminus of the hTfR affinity peptide 5 having the amino acid sequence of SEQ ID NO:7 is bound to the C-terminus of the hTfR affinity peptide 5 having the amino acid sequence of SEQ ID NO:7 via a peptide linker having the amino acid sequence of SEQ ID NO:59, and the N-terminus of hIDS is further bound to the C-terminus of the former hTfR affinity peptide 5 via a peptide linker having the amino acid sequence GS.

When the different protein (A) is human SGSH, a preferable fusion protein includes the following fusion proteins. That is,
(1) a fusion protein in which the N-terminus of hSGSH is bound to the C-terminus of an hTfR affinity peptide having the amino acid sequence of SEQ ID NO:3, SEQ ID NO:4, SEQ ID NO:5, SEQ ID NO:6, SEQ ID NO:7, or SEQ ID NO:55 via a peptide linker having the amino acid sequence of SEQ ID NO:59, and
(2) a fusion protein in which the N-terminus of hSGSH having the amino acid sequence of SEQ ID NO:66 is bound to the C-terminus of the hTfR affinity peptide 3 having the amino acid sequence of SEQ ID NO:5 via a peptide linker having the amino acid sequence of SEQ ID NO:59.

Trastuzumab is an antibody medicine exerting an anti-tumor effect by specifically binding to HER2, which is encoded by human oncogene HER2/neu(c-erbB-2). Trastuzumab is composed of heavy chains and light chains, and the respective amino acid sequences of the chains are set forth in SEQ ID NO:40 and SEQ ID NO:41, respectively.

One specific embodiment of the fusion protein of an hTfR affinity peptide and different protein (A) according to the present invention is a fusion protein of an hTfR affinity peptide and trastuzumab, in which the hTfR affinity peptide 1 is bound to the C-terminus of a heavy chain of trastuzumab via a linker. The fusion protein of the hTfR affinity peptide 1 and trastuzumab can be produced by culturing host cells into which an expression vector for a protein having the amino acid sequence of SEQ ID NO:42 (hTfR affinity peptide 1-trastuzumab heavy chain fusion protein), in which the hTfR affinity peptide 1 having the amino acid sequence of SEQ ID NO: 3 is bound to the C-terminus of a heavy chain of trastuzumab via a peptide linker having the amino acid sequence of SEQ ID NO:69, and an expression vector for the protein of the trastuzumab light chain have been introduced. In the other embodiments, fusion proteins of hTfR affinity peptides and trastuzumab of other embodiments can also be produced by using expression vectors for an hTfR affinity peptide 2-trastuzumab heavy chain fusion protein having the amino acid sequence of SEQ ID NO:43, an hTfR affinity peptide 3-trastuzumab heavy chain fusion protein having the amino acid sequence of SEQ ID NO:44, an hTfR affinity peptide 4-trastuzumab heavy chain fusion protein having the amino acid sequence of SEQ ID NO:45, and an hTfR affinity peptide 5-trastuzumab heavy chain fusion protein having the amino acid sequence of SEQ ID NO:46, instead of the expression vector of the expression vector for the hTfR affinity peptide 1-trastuzumab heavy chain fusion protein. An expression vector for a fusion protein of the hTfR affinity peptide 6 and the trastuzumab heavy chain can also be used.

Trastuzumab is mainly used as a therapeutic agent for breast cancer; however, since trastuzumab cannot pass through the BBB when intravenously injected, it cannot exert drug efficacy against cancers in the central nervous system. However, when trastuzumab is bound to an anti-hTfR heavy chain antibody variable region peptide, trastuzumab can pass through the BBB, and can therefore act even against cancers expressing HER2 in the central nervous system. Since breast cancer may metastasize to the central nervous system, the conjugate can be expected to be effective against such cancers in the central nervous system that have metastasized from breast cancer.

Antibody medicines other than trastuzumab can also be produced as fusion proteins with anti-hTfR heavy chain antibody variable region peptides, similarly to trastuzumab. In this case, the anti-hTfR heavy chain antibody variable region peptide may be bound to the C-terminus of a heavy chain of an antibody, may be bound to the N-terminus of a heavy chain of an antibody, may be bound to the C-terminus of a light chain of an antibody, or may be bound to the N-terminus of a light chain of an antibody.

The drug to be bound to the hTfR affinity peptide to form a drug complex may be a low molecular weight compound. Here, the low molecular weight compound is preferably a compound that should be allowed to pass through the BBB and act on the central nervous system; however, without being limited to this, the low molecular weight compound may be a contrast agent, a fluorescent dye, a phospholipid that may be a constituent component of liposomes, a functional lipid that may be a constituent component of lipid nanoparticles, or the like. The molecular weight of the low molecular weight compound is, for example, 1 to 50 kD, 0.5 to 20 kD, or 1 to 5 kD. A peptide composed of 5 to 20 amino acids, and a polysaccharide having a structure formed by dehydration condensation of 5 to 20 monosaccharides, are also included in the low molecular weight compound.

The hTfR affinity peptide and the low molecular weight compound are bound by covalent bonding directly or via a linker. The binding mode of the hTfR affinity peptide and the low molecular weight compound is not particularly limited as long as the binding affinity of the hTfR affinity peptide to hTfR is maintained, and the drug exerts drug efficacy in the central nervous system when an hTfR affinity peptide-drug complex is administered into the living body. As the linker, for example, a linker containing valine-citrulline or valine-alanine, which are cleaved by cathepsin B, or a linker containing cysteine-cysteine is suitably used. When such a linker is administered into a living body, since the linker can be cleaved by the metabolic mechanism in the living body, for example, by an enzyme, the low molecular weight compound can be separated from the hTfR affinity peptide and exert drug efficacy by itself. In the present specification, such a linker that can be cleaved in vivo is referred to as a biodegradable linker.

When an hTfR affinity peptide and a low molecular weight compound are bound by a biodegradable linker, it is possible to dispose a spacer between these in addition to the linker. By disposing a spacer, the accessibility of the enzyme that cleaves the linker to the complex is improved. Such a spacer is not particularly limited to these; however, a p-aminocarbamate group/carbamate group is suitably used.

The drug in the hTfR affinity peptide-drug complex may be a nucleic acid. Here, the nucleic acid may be any of single-stranded DNA, double-stranded DNA, single-stranded RNA, and double-stranded RNA. In order to prevent degradation of nucleic acids by nucleases in the living body, the nucleotides constituting these nucleic acids can be modified to make the nucleic acids resistant to nucleases. The chain length of the nucleic acid is not particularly limited; however, the chain length is, for example, 10 bp to 1000 bp, or 10 bp to 100 bp. In this complex, the hTfR affinity peptide and the nucleic acid are bound by covalent bonding directly or via a linker. The binding mode of an immunoglobulin single variable domain and a low molecular weight compound is not particularly limited as long as the binding affinity of the hTfR affinity peptide to hTfR is maintained, and the drug exerts drug efficacy in the central nervous system when the hTfR affinity peptide-nucleic acid complex is administered into the living body. As the linker, a biodegradable linker is preferably used.

When an hTfR affinity peptide and a nucleic acid are bound by a biodegradable linker, it is possible to dispose a spacer between these in addition to the linker. By disposing a spacer, accessibility of the nucleic acid that cleaves the linker to the complex is improved. Such a spacer is not particularly limited to these; however, a p-aminocarbamate group/carbamate group is suitably used.

The nucleic acid to be bound to the hTfR affinity peptide is, for example, a nucleic acid used for the treatment of central nervous system diseases. Such a nucleic acid is an antisense nucleic acid against mRNA whose expression in the central nervous system is abnormally increased. Such an antisense nucleic acid binds to an mRNA that is abnormally expressed, and exhibits an effect of inhibiting translation of the mRNA into a protein.

An hTfR affinity peptide-drug complex (including an hTfR affinity peptide-protein(A) fusion protein) can be supplied to medical institutions as a drug (pharmaceutical composition) containing the complex as an active ingredient, in the form of a freeze-dried product, an aqueous liquid preparation, or the like. In the case of an aqueous liquid preparation, the hTfR affinity peptide-drug complex can be preliminarily dissolved in a solution containing a stabilizer, a buffer agent, and a tonicity adjusting agent, and this solution can be supplied as a preparation enclosed in a vial or a syringe. A preparation enclosed in a syringe is generally referred to as a prefilled syringe preparation. When prepared into a prefilled syringe preparation, administration of a drug by a patient himself or herself can be made easier. Administration of the drug is performed by parenteral means, for example, intravenous injection. The administered drug can pass through the blood brain barrier and exert drug efficacy in the central nervous system, for example the drug can exert drug efficacy in cerebral cortex, cerebellum, hippocampus, midbrain, pons, medulla oblongata, caudate nucleus, putamen, globus pallidus, thalamus, and hypothalamus, particularly can exert drug efficacy in cerebral cortex, cerebellum, hippocampus, and midbrain.

The hTfR affinity peptide can be used in order to modify the surface of vesicles into which drugs can be enclosed. Examples of the vesicles include liposomes and lipid nanoparticles (LNP). A liposome refers to a spherical vesicle having a lipid bilayer, and generally includes phospholipids, particularly phosphatidylcholine, as a constituent component. Furthermore, the liposome may also include other lipids such as egg yolk phosphatidylethanolamine as constituent components. A lipid nanoparticle refers to a particle having a diameter of 10 nm to 1000 nm, typically less than about 200 nm, and containing lipids as a main component, and a lipid nanoparticle can enclose a hydrophobic (lipophilic) molecule and contains biocompatible lipids such as triglycerides, diglycerides, monoglycerides, fatty acids, and steroids, as main constituent components. These vesicles can have drugs enclosed therein.

When vesicles having a drug enclosed therein are administered into the living body, the membranes constituting the vesicles fuse with the cell membrane, and the drug is released into the cells. That is, these vesicles can be used as a carrier for a drug. When an hTfR affinity peptide is disposed on the surface of such vesicles, the vesicles bind, via the peptide, to hTfR on cerebrovascular endothelial cells constituting the blood brain barrier, and the drug enclosed in the vesicles can be released into brain parenchyma such as cerebellar brain parenchyma, as the vesicles fuse with the cell membrane of cerebrovascular endothelial cells, or as the vesicles are taken up into cells by endocytosis or the like.

The term vesicle as used in the present specification should be interpreted to include, in addition to the above-described liposomes and lipid nanoparticles, polymer nanoparticles, micelles, an emulsion, a nanoemulsion, microspheres, nanospheres, microcapsules, nanocapsules, a dendrimer, a nanogel, metal nanoparticles, and all other nano- and microparticles that can be used as drug delivery systems (DDS).

### Examples

Hereunder, the present invention will be described in more detail with reference to Examples; however, the present invention is not intended to be limited to the Examples.

### [Example 1] Construction of vector for hTfR expression

A gene fragment encoding human transferrin receptor (hTfR) was amplified by PCR using human spleen Quick Clone cDNA (Clontech Laboratories, Inc.) as a template and using primer hTfR5' (SEQ ID NO:49) and primer hTfR3' (SEQ ID NO:50). The amplified gene fragment encoding hTfR was digested with MluI and NotI and was inserted between the sites of Mlul and NotI of a pCI-neo vector (Promega Corporation). The obtained vector was named pCI-neo(hTfR). Next, this vector was digested with MluI and NotI to excise the gene fragment encoding hTfR, and the excised gene fragment was incorporated between the sites of MluI and NotI of pE-mIRES-GS-puro, which is an expression vector described in PCT International Publication (WO 2012/063799), to construct pE-mIRES-GS-puro(hTfR), which was a vector for hTfR expression.

### [Example 2] Production of recombinant hTfR

pE-mIRES-GS-puro(hTfR) was introduced into CHO-K1 cells by an electroporation method, subsequently the cells were subjected to selective culture using CD OptiCHO^{™} medium (Invitrogen, Inc.) containing methionine sulfoximine (MSX) and puromycin, and recombinant hTfR-expressing cells were obtained. These recombinant hTfR-expressing cells were cultured to prepare recombinant hTfR (rhTfR).

### [Example 3] Production of phage library

Alpacas were immunized using the rhTfR prepared in Example 2 as an antigen. Blood was collected from the immunized alpacas, and peripheral blood lymphocytes were isolated from the blood. RNA was extracted from peripheral blood lymphocytes, and this RNA was subjected to reverse transcription to obtain cDNA. A DNA fragment having a nucleotide sequence encoding the variable region of a heavy chain antibody was amplified by a PCR method using the obtained cDNA as a template. The amplified DNA fragment was incorporated into a phagemid such that when packaged into a phage, the variable region of the heavy chain antibody would be displayed on the surface of the phage, and this phagemid was introduced into Escherichia coli by an electroporation method. Escherichia coli into which the phagemid was introduced was further infected with a helper phage (M13K07), and then the Escherichia coli was cultured to release the phage into the culture fluid. After completion of culture, the culture medium was centrifuged to collect the culture supernatant containing the phage. This culture supernatant containing the collected phage was used as a phage library. The concentration of the phage library was 3.36×10¹³ PFU/mL.

### [Example 4] Isolation of anti-hTfR heavy chain antibody-expressing phage clones

Phages expressing the variable region of a heavy chain antibody that specifically binds to hTfR (anti-hTfR heavy chain antibody-expressing phage) were cloned from the phage library produced in Example 3 by a biopanning method. Cloning was carried out generally as follows. The solution containing rhTfR produced in Example 2 was added to each well of a microtiter plate, and rhTfR was immobilized on the plate. Next, each well was washed three times with a 0.05% PBST solution, and then a blocking agent was added to each well to block each well. Next, each well was washed three times with a 0.05% PBST solution, and the phage library diluted with the blocking agent was added thereto. The plate was left to stand to allow phages that carried the anti-hTfR heavy chain antibody on the surface to bind to rhTfR. Next, each well was washed five times with a 0.05% PBST solution to remove phages that did not bind to rhTfR. Next, phages bound to rhTfR were dissociated from rhTfR by adding an acidic eluent thereto and were collected as a solution.

The collected solution was diluted and added to a solution containing Escherichia coli, and the Escherichia coli was infected with the phages. The Escherichia coli infected with the phages was further infected with a helper phage (M13K07), and then the Escherichia coli was cultured to release the phages into the culture fluid. The phages were purified using a PEG solution.

Escherichia coli was infected with the purified phages and then seeded on an agar medium. Escherichia coli cells that formed a single colony on the medium were collected, further cultured in the medium, and then the Escherichia coli cells were infected with a helper phage (M13K07) to release phages into the culture fluid. The phages released into the culture fluid were collected, designated as anti-hTfR heavy chain antibody-expressing phage clones, in a culture supernatant obtained by centrifuging each culture fluid. Each phage clone contained in the culture supernatant was purified using a PEG solution.

### [Example 5] Determination of amino acid sequence of variable region of anti-hTfR heavy chain antibody expressed by each anti-hTfR heavy chain antibody-expressing phage clone.

Five clones were selected from the anti-hTfR heavy chain antibody-expressing phages isolated in Example 4 and were named clones 1 to 5, respectively. A phagemid was purified from each clone, the nucleotide sequence of the variable region of the anti-hTfR heavy chain antibody encoded by each purified phagemid was determined, and based on this, the amino acid sequence of the variable region of the anti-hTfR heavy chain antibody expressed by each clone was determined (Table 1)

**[Table 1]**

| Table 1 Amino acid sequences of variable regions of anti-hTfR heavy chain antibodies expressed by clones 1 to 5 (examples of the amino acid sequence of each CDR) | |
|---|---|
| Clone number | Amino acid sequence of variable region of anti-hTfR heavy chain antibody |
| 1 | SEQ ID NO:3 |
| 2 | SEQ ID NO:4 |
| 3 | SEQ ID NO: 5 |
| 4 | SEQ ID NO:6 |
| 5 | SEQ ID NO:7 |

Here, a protein having the amino acid sequence set forth in SEQ ID NO:3 was referred to as hTfR affinity peptide 1, a protein having the amino acid sequence set forth in SEQ ID NO:4 was referred to as hTfR affinity peptide 2, a protein having the amino acid sequence set forth in SEQ ID NO:5 was referred to as hTfR affinity peptide 3, a protein having the amino acid sequence set forth in SEQ ID NO:6 was referred to as hTfR affinity peptide 4, and a protein having the amino acid sequence set forth in SEQ ID NO:7 was referred to as hTfR affinity peptide 5. Each of the hTfR affinity peptides 1 to 5 contains CDR1 to CDR3. The amino acid sequence of each of the CDRs is shown in Table 2. The amino acid sequence of the CDR of the hTfR affinity peptide 5 exemplified in Table 2 is identical to the hTfR affinity peptide 3. In Table 2, the amino acid sequence of each CDR of the hTfR affinity peptide 6 that will be described below in Example 6 is also shown together.

**[Table 2]**

| Table 2 Sequence number of amino acid sequence contained in CDR1 to CDR3 of hTfR affinity peptides 1 to 6 (examples of amino acid sequence of each CDR) | | | |
|---|---|---|---|
| Name | Amino acid sequence | | |
| | CDR1 | CDR2 | CDR3 |
| hTfR affinity peptide 1 | 8, 9 | 10, 11 | 12, 13 |
| hTfR affinity peptide 2 | 14, 15 | 16, 17 | 18, 19 |
| hTfR affinity peptide 3 | 20, 21 | 22, 23 | 24, 25 |
| hTfR affinity peptide 4 | 26, 27 | 28, 29 | 30, 31 |
| hTfR affinity peptide 5 | 20, 21 | 22, 23 | 24, 25 |
| hTfR affinity peptide 6 | 20, 21 | 56, 57 | 24, 25 |

### [Example 6] Production of hTfR affinity peptides 1 to 6

A DNA fragment containing a gene encoding the hTfR affinity peptide 1 to which a peptide tag set forth in SEQ ID NO:54 was added at the N-terminus, was artificially synthesized, and this was incorporated between the Mini site and the NotI site of a pCI-neo Mammalian Expression Vector to obtain an hTfR affinity peptide 1 expression vector. This expression vector was introduced into CHO-S cells using an ExpiCHO Expression System (Thermo Fisher Scientific, Inc.) according to the attached protocol, and the hTfR affinity peptide 1 was expressed in the CHO-S cells. These cells express the hTfR affinity peptide 1 and secrete this peptide into the culture fluid. These cells were cultured in the presence of neomycin according to the attached protocol. Specifically, CHO-S cells were suspended in 15 mL of a culture medium so as to have a concentration of 6×10⁶ cells/mL, this suspension was transferred into an Erlenmeyer flask having a volume of 150 mL, and the cells were cultured for one day at 37°C in a humidified environment composed of 5% CO₂ and 95% air at an agitation speed of about 120 rpm. The Feed liquid and Enhancer liquid included in the ExpiCHO Expression System were added to the culture, and the cells were cultured for 9 days at 32°C in a humidified environment composed of 5% CO₂ and 95% air at an agitation speed of about 120 rpm, letting the hTfR affinity peptide 1 be expressed. After completion of culturing, a supernatant obtained by centrifuging the culture fluid (3000 g, for 5 minutes) was filtered through a 0.22-µm filter (Millipore Corporation), and this was collected as a culture supernatant. The hTfR affinity peptide 1 was purified from the collected culture supernatant by the following method, and a refined product was obtained.

The collected culture supernatant, 5 column volumes of 50 mM Tris-HCl buffer solution (pH 7.5) containing 150 mM NaCl and 1 mM CaCl₂ as an equilibration solution was passed through an Anti-FLAG M1 Agarose Affinity Gel column (Sigma-Aldrich Co., LLC). Next, the culture supernatant was loaded onto the column, and the hTfR affinity peptide 1 was adsorbed to the affinity column. Next, the column was washed with 5 column volumes of the equilibration solution. The hTfR affinity peptide 1 was eluted by passing an eluent (50 mM Tris-HCl buffer solution (pH 7.5) containing 150 mM NaCl) through the column. The eluted hTfR affinity peptide 1 was used as a purified product in the following experiments. For the hTfR affinity peptides 2 to 5, purified products were obtained in the same manner as in the case of the hTfR affinity peptide 1. Furthermore, an expression vector encoding the hTfR affinity peptide 6 having the amino acid sequence set forth in SEQ ID NO: 55, in which one amino acid of CDR2 of the hTfR affinity peptide 5 had been substituted, was produced by a standard method, and a purified product of the hTfR affinity peptide 6 was obtained using this expression vector in the same manner as in the case of the hTfR affinity peptides 1 to 5.

### [Example 7] Evaluation of binding activity of hTfR affinity peptides 1 to 6 toward human TfR and monkey TfR

Measurement of the binding activity of the hTfR affinity peptides 1 to 5 toward human TfR (hTfR) and monkey TfR was carried out using OctetRED96 (ForteBio, Inc., a division of Pall Corporation), which is a biomolecular interaction analysis system using BioLayer Interferometry (BLI). The basic principles of biolayer interferometry will be briefly described. When light of a specific wavelength is projected onto a layer of biomolecules fixed on the surface of a sensor chip, the light is reflected from two surfaces, that is from the surfaces of the biomolecular layer and the internal reference layer, and an interference wave of the light is created. As molecules in the measurement sample bind to the biomolecules on the surface of the sensor chip, the thickness of the layer at the tip of the sensor increases, resulting in a wavelength shift occuring in the interference wave. By measuring the change in this wavelength shift, quantification of the number of molecules that bind to the biomolecules immobilized on the sensor chip surface and kinetic analysis can be performed in real time. The measurement was generally carried out according to the operation manual attached to OctetRED96. As the human TfR, a recombinant hTfR having the amino acid sequence of the extracellular region of hTfR, from the 89^{th} cysteine residue from the N-terminus to phenylalanine at the C-terminus in the amino acid sequence set forth in SEQ ID NO:1, with a histidine tag added at the N-terminus, was used (rhTfR, Sino Biological, Inc.). As the monkey TfR, a recombinant monkey TfR (r-monkey TfR, Sino Biological, Inc.) having the amino acid sequence of the extracellular region of cynomolgus monkey TfR, from the 89^{th} cysteine residue from the N-terminus to phenylalanine at the C-terminus in the amino acid sequence set forth in SEQ ID NO:2, with a histidine tag added at the N-terminus, was used.

The purified products of the hTfR affinity peptides 1 to 6 obtained in Example 6 were each diluted with HBS-P+ (10 mM HEPES containing 150 mM NaCl, 1% BSA, 50 µM EDTA, and 0.05% Surfactant P20), and solutions with three kinds of concentrations of 10 to 40 nM were prepared. These solutions were used as sample solutions. The rhTfR and r-monkey TfR were each diluted with HBS-P+, and 15 µg/mL solutions were prepared, which were used as an hTfR-ECD(Histag) solution and a monkey TfR-ECD(Histag) solution, respectively.

Each of the above-described sample solutions was dispensed on a 96-well black plate (Greiner Bio-One GmbH) at 200 µL per well. Furthermore, the hTfR-ECD(Histag) solution and the monkey TfR-ECD(Histag) solution prepared as described above were each dispensed into predetermined wells at 200 µL/well. In the wells for baseline, dissociation, and washing, HBS-P+ was dispensed at 200 µL/well. In the wells for regeneration, 10 mM Glycine-HCl (pH 1.7) was dispensed at 200 µL/well. In the wells for activation, 0.5 mM NiCl₂ solution containing 1% BSA was dispensed at 200 µL/well. This plate and a biosensor (Biosensor/Ni-NTA; ForteBio, Inc., a division of Pall Corporation) were placed at predetermined positions of OctetRED96.

OctetRED96 was operated under the conditions shown in the following Table 3 to acquire data, subsequently the binding reaction curves were fitted to a 1:1 binding model or a 2:1 binding model using the analysis software attached to OctetRED96, the association rate constants (kon) and the dissociation rate constants (koff) of each of the hTfR affinity peptides 1 to 6 for hTfR and monkey TfR were measured to calculate the dissociation constants (K_{D}). The measurement was carried out at a temperature of 25°C to 30°C.

**[Table 3]**

| Table 3 Operation conditions for OctetRED96 | | | | |
|---|---|---|---|---|
| | Step | Contact time (sec) | Rate (rpm) | Threshold value |
| 1 | Regeneration | 5 | 1000 | - |
| 2 | Washing | 5 | 1000 | - |

| The above-described steps 1 and 2 are repeated six times. | | | | |
|---|---|---|---|---|
| 3 | Activation | 60 | 1000 | 1.0 |
| 4 | Baseline 1 | 120 | 1000 | - |
| 5 | Loading | 600 | 1000 | - |
| 6 | Baseline 2 | 150 | 1000 | - |
| 7 | Association | 120 | 1000 | - |
| 8 | Dissociation | 200 | 1000 | - |
| Until the measurement of all the samples is completed, steps 1 to 8 are repeatedly performed. | | | | |

Table 4 shows the measurement results of the association rate constants (kon), the dissociation rate constants (koff), and the dissociation constants (K_{D}) of the hTfR affinity peptides 1 to 6 for hTfR and monkey TfR. Regarding the hTfR affinity peptides 1 and 2, the KD values for the monkey TfR were not obtained. With regard to these, the K_{D} values were separately measured using the hTfR-ECD(Histag) solution and the monkey TfR-ECD(Histag) solution produced in Example 2 and using Biacore. As a result, the K_{D} values of the hTfR affinity peptide 1 for the monkey TfR and human TfR were measured to be 1.76×10⁻⁸ and 1.65×10⁻⁸, respectively. Furthermore, the KD values of the hTfR affinity peptide 2 for the monkey TfR and human TfR were measured to be 1.76×10⁻⁸ and 1.65×10⁻⁸, respectively. These results indicate that the KD value of each hTfR affinity peptide with the monkey TfR is approximately 1.0×10⁻⁸ to 5.0×10⁻⁵, and the KD value with the human TfR is approximately 1.0×10⁻⁸ to 1.0×10⁻⁷.

**[Table 4]**

| Table 4 Affinity of hTfR affinity peptides 1 to 6 for hTfR and monkey TfR | | | | |
|---|---|---|---|---|
| Name | Animal species | kon (1/Ms) | koff (1/s) | KD (M) |
| hTfR affinity peptide 1 | Monkey | N/A | N/A | N/A |
| | Human | 2.80×10⁵ | 2.71×10⁻² | 9.70×10⁻⁸ |
| hTfR affinity peptide 2 | Monkey | N/A | N/A | N/A |
| | Human | 2.37×10⁵ | 1.46×10⁻² | 6.14×10⁻⁸ |
| hTfR affinity peptide 3 | Monkey | 1.15×10⁶ | 1.23×10⁻² | 1.07×10⁻⁸ |
| | Human | 5.71×10⁵ | 6.00×10⁻³ | 1.05×10⁻⁸ |
| hTfR affinity peptide 4 | Monkey | 3.18×10⁵ | 3.16×10⁻³ | 9.92×10⁻⁹ |
| | Human | 2.27×10⁵ | 2.70×10⁻³ | 1.19×10⁻⁸ |
| hTfR affinity peptide 5 | Monkey | 3.96×10⁵ | 6.95×10⁻³ | 1.75×10⁻⁸ |
| | Human | 4.07×10⁵ | 5.62×10⁻³ | 1.38×10⁻⁸ |
| hTfR affinity peptide 6 | Monkey | N/A | N/A | N/A |
| | Human | 4.28×10⁵ | 5.86×10⁻² | 1.37×10⁻⁷ |

From the above-described results, the relative value of the dissociation constant with the monkey TfR in the case where the value of the dissociation constant of each hTfR affinity peptide for the hTfR was taken as 1, was calculated (Table 5). All the relative values of the hTfR affinity peptides were in the range of 0.8 to 2.5. These results indicate that the affinity for the hTfR approximates to the affinity for the monkey TfR. Particularly, for other hTfR affinity peptides except for the hTfR affinity peptide 2, the relative values were in the range of 0.8 to 1.3, and this indicates that the affinity for the hTfR very highly approximates to the affinity for the monkey TfR. Therefore, it is naturally predicted that the pharmacokinetics of all of these hTfR affinity peptides (particularly, hTfR affinity peptides 1 and 3 to 5) in the living bodies of monkeys when administered to monkeys, very highly approximate to the pharmacokinetics when administered to humans, and therefore, in a case where a medicine is developed by utilizing these, some of non-clinical studies such as pharmacokinetic tests of the medicine can be carried out using monkeys, which can significantly accelerate the development of the medicine.

**[Table 5]**

| Table 5 Relative values of hTfR affinity peptides 1 to 6 for monkey TfR | |
|---|---|
| Name | Relative value |
| hTfR affinity peptide 1 | 1.06 |
| hTfR affinity peptide 2 | 2.36 |
| hTfR affinity peptide 3 | 1.02 |
| hTfR affinity peptide 4 | 0.83 |
| hTfR affinity peptide 5 | 1.27 |
| hTfR affinity peptide 6 | N/A |

### [Example 8] Production of hTfR affinity peptide-trastuzumab expression vector

pCI-neo Mammalian Expression Vector (Promega Corporation) was digested with MluI and NotI. A DNA fragment having an Mini site on the 5' side and a NotI site on the 3' side of a gene having a nucleotide sequence set forth in SEQ ID NO:47 encoding a protein having the amino acid sequence set forth in SEQ ID NO:42, in which the hTfR affinity peptide 1 is bound to the C-terminus of the trastuzumab heavy chain having the amino acid sequence set forth in SEQ ID NO:38, via a peptide linker having the amino acid sequence set forth in SEQ ID NO:69, was artificially synthesized. This was digested with Mini and NotI and incorporated between the MluI site and the NotI site of pCI-neo Mammalian Expression Vector, and an expression vector of a protein in which the hTfR affinity peptide 1 and the trastuzumab heavy chain were bound was produced. This expression vector was named pCI-neo-Trastuzumab(HC)-VHH(1). Similarly, for the hTfR affinity peptides 2 to 5 as well, DNA fragments each containing a gene encoding a protein in which one of the peptides and the trastuzumab heavy chain were bound, were artificially synthesized. These DNA fragments were each digested with MluI and NotI and incorporated between the MluI site and NotI site of pCI-neo Mammalian Expression Vector, and expression vectors of proteins in which any one of the hTfR affinity peptides 2 to 5 and the trastuzumab heavy chain were bound were produced. The produced expression vectors were named pCI-neo-Trastuzumab(HC)-VHH(2) to pCI-neo-Trastuzumab(HC)-VHH(5), respectively.

Furthermore, a DNA fragment having an MluI site on the 5' side and a NotI site on the 3' side of a gene having a nucleotide sequence set forth in SEQ ID NO:48 encoding the trastuzumab light chain having the amino acid sequence set forth in SEQ ID NO:41, was artificially synthesized, this was digested with MluI and NotI and ligated to the previously prepared pCI-neo Mammalian Expression Vector (Promega Corporation) that had been digested with MluI and NotI, to construct an expression vector for the trastuzumab light chain. This expression vector was named pCI-neo-Trastuzumab(LC).

### [Example 9] Production of hTfR affinity peptide-trastuzumab

The pCI-neo-Trastuzumab(HC)-VHH(1) and pCI-neo-Trastuzumab(LC) produced in Example 8 were simultaneously introduced into CHO-S cells using an ExpiCHO Expression System (Thermo Fisher Scientific, Inc.) according to the attached protocol, and a protein in which the hTfR affinity peptide 1 and the trastuzumab heavy chain were bound, and the trastuzumab light chain were co-expressed in the CHO-S cells. These cells express the protein in which an hTfR affinity peptide and trastuzumab are fused, and secrete this protein into the culture fluid. Hereinafter, this fusion protein will be referred to as hTfR affinity peptide-trastuzumab 1. These cells were cultured in the presence of neomycin according to the attached protocol. More specifically, CHO-S cells were suspended in 15 mL of a culture medium so as to have a concentration of 6×10⁶ cells/mL, this suspension was transferred into an Erlenmeyer flask having a volume of 150 mL, and then the cells were cultured for one day at 37°C in a humidified environment composed of 5% CO₂ and 95% air at an agitation speed of about 120 rpm. The Feed liquid and Enhancer liquid included in the ExpiCHO Expression System were added to the culture, and the cells were cultured for 9 days at 32°C in a humidified environment composed of 5% CO₂ and 95% air at an agitation speed of about 120 rpm, and the hTfR affinity peptide-trastuzumab 1 was expressed. After culturing, a supernatant obtained by centrifuging the culture fluid (3000 g, for 5 minutes) was filtered through a 0.22-µm filter (Millipore Corporation), and this was collected as a culture supernatant.

An open column packed with 1 mL of MabSelect SuRe LX (Cytiva, Inc.) was equilibrated by passing 5 column volumes of 25 mM HEPES buffer solution (pH 7.5) containing 50 mM NaCl through the column. Next, the culture supernatant was loaded onto the column, and the hTfR affinity peptide-trastuzumab 1 was bound to the resin. The column was washed by passing 5 column volumes of 25 mM HEPES buffer solution (pH 7.5) containing 50 mM NaCl through the column, and then the hTfR affinity peptide-trastuzumab 1 was eluted by passing 150 mM Glycine (pH 3.0) containing 100 mM NaCl through the column. 5% equivalent amount of 1 M Tris-HCl (pH 8.0) was added to the eluate containing the hTfR affinity peptide-trastuzumab 1 to adjust the pH to neutrality. This was subjected to buffer exchange using a PD-10 column (Cytiva, Inc.) for replacing the solvent with PBS(-). The obtained solution was used in the following experiments as a purified product of the hTfR affinity peptide-trastuzumab 1.

Similarly to the pCI-neo-Trastuzumab(HC)-VHH(1), pCI-neo-Trastuzumab(HC)-VHH(2) to pCI-neo-Trastuzumab(HC)-VHH(5) were introduced into CHO-S cells simultaneously with pCI-neo-Trastuzumab(LC), and each of the proteins in which the hTfR affinity peptides 2 to 5 and the trastuzumab heavy chain were bound, and the trastuzumab light chain were co-expressed in the CHO-S cells. These cells express a protein in which any one of the hTfR affinity peptides 2 to 4 and trastuzumab are fused, and secrete this protein into the culture fluid. Hereinafter, the fusion proteins expressed by these cells will be referred to as hTfR affinity peptide-trastuzumabs 2 to 5. Furthermore, the hTfR affinity peptide-trastuzumabs 1 to 5 will be collectively referred to as hTfR affinity peptide-trastuzumab. For the hTfR affinity peptide-trastuzumabs 2 to 5 as well, purified products were obtained in the same manner as in the case of the hTfR affinity peptide-trastuzumab 1.

### [Example 10] Evaluation of binding activity of hTfR affinity peptide-trastuzumab toward human TfR and monkey TfR

The affinity of the hTfR affinity peptide-trastuzumabs 1 to 5 produced in Example 9 for hTfR and monkey TfR was investigated according to the method described in Example 7. Table 6 shows the measurement results of the association rate constants (kon), the dissociation rate constants (koff), and the dissociation constants (K_{D}) of the hTfR affinity peptide-trastuzumabs 1 to 5 for hTfR and monkey TfR.

**[Table 6]**

| Table 6 Affinity of hTfR affinity peptide-trastuzumabs 1 to 5 for hTfR and monkey TfR | | | | |
|---|---|---|---|---|
| Name | Animal species | kon (1/Ms) | koff (1/s) | KD (M) |
| hTfR affinity peptide-trastuzumab 1 | Monkey | 5.72×10⁶ | 4.92×10⁻⁴ | 8.61×10⁻¹⁰ |
| | Human | 4.73×10⁵ | 2.26×10⁻⁴ | 4.77×10⁻¹⁰ |
| hTfR affinity peptide-trastuzumab 2 | Monkey | 6.59×10⁵ | 1.61×10⁻³ | 2.45×10⁻⁹ |
| | Human | 4.74×10⁵ | 4.95×10⁻⁴ | 1.04×10⁻⁹ |
| hTfR affinity peptide-trastuzumab 3 | Monkey | 5.74×10⁵ | 1.24×10⁻⁴ | 2.15×10⁻¹⁰ |
| | Human | 5.25×10⁵ | 1.63×10⁻⁴ | 3.12×10⁻¹⁰ |
| hTfR affinity peptide-trastuzumab 4 | Monkey | 5.13×10⁵ | 8.45×10⁻⁵ | 1.65×10⁻¹⁰ |
| | Human | 4.99×10⁵ | 6.19×10⁻⁵ | 1.24×10⁻¹⁰ |
| hTfR affinity peptide-trastuzumab 5 | Monkey | 5.41×10⁵ | 1.24×10⁻⁴ | 2.29×10⁻¹⁰ |
| | Human | 4.78×10⁵ | 1.18×10⁻⁴ | 2.47×10⁻¹⁰ |

As a result of the measurement of the binding activity of the hTfR affinity peptide-trastuzumabs toward the hTfR, for all of the hTfR affinity peptide-trastuzumabs, the dissociation constant (K_{D}) with the hTfR was 1×10⁻⁹ M to 1× 10⁻¹⁰ M. Furthermore, the dissociation constant with the monkey TfR was also similarly 1×10⁻⁹ M to 1×10⁻¹⁰ M. These results show that these hTfR affinity peptide-trastuzumabs maintain the affinity for both hTfR and monkey TfR, even when fused with other proteins.

From the above-described results, the relative value of the dissociation constant with monkey TfR in the case where the dissociation constant of each hTfR affinity peptide-trastuzumab with hTfR was taken as 1, was calculated (Table 7). All the relative values of the hTfR affinity peptide-trastuzumabs were in the range of 0.9 to 2.5. These results indicate that the affinity of each hTfR affinity peptide-trastuzumab for the hTfR approximates to the affinity for the monkey TfR. Particularly, for each of hTfR affinity peptides other than the hTfR affinity peptide-trastuzumab 2, the relative values were in the range of 0.8 to 1.9, and this indicates that the affinity for the hTfR very highly approximates to the affinity for the monkey TfR. Therefore, it is naturally predicted that the pharmacokinetics of each of these hTfR affinity peptide-trastuzumabs (particularly, each of hTfR affinity peptide-trastuzumabs 1 and 3 to 5) in the living bodies of monkeys when administered to monkeys, very highly approximate to the pharmacokinetics when administered to humans, and therefore, in a case where a medicine is developed by utilizing these, some of non-clinical studies such as pharmacokinetic tests of the medicine can be carried out using monkeys, which can significantly accelerate the development of the medicine.

**[Table 7]**

| Relative values of affinity of hTfR affinity peptide-trastuzumabs 1 to 5 for monkey TfR | |
|---|---|
| Name | Relative value |
| hTfR affinity peptide-trastuzumab 1 | 1.81 |
| hTfR affinity peptide-trastuzumab 2 | 2.36 |
| hTfR affinity peptide-trastuzumab 3 | 0.69 |
| hTfR affinity peptide-trastuzumab 4 | 1.33 |
| hTfR affinity peptide-trastuzumab 5 | 0.93 |

### [Example 11] Evaluation 1 of migration of hTfR affinity peptide-trastuzumab to brain using hTfR knock-in mouse

Next, whether the hTfR affinity peptide-trastuzumabs 1 to 5 pass through the BBB and migrate into the brain when injected intravenously, was evaluated using hTfR knock-in mouse (hTfR-KI mouse) in which the gene encoding the extracellular region of mouse transferrin receptor had been replaced with a gene encoding the extracellular region of the human transferrin receptor gene. The hTfR-KI mouse was generally produced by the following method.

A DNA fragment having a nucleotide sequence set forth in SEQ ID NO:51, in which a neomycin resistance gene flanked by loxP sequences was disposed on the 3' side of a cDNA encoding a chimeric hTfR whose intracellular region had the amino acid sequence of mouse hTfR and whose extracellular region had the amino acid sequence of human hTfR, was chemically synthesized. This DNA fragment was incorporated into a targeting vector having a nucleotide sequence set forth in SEQ ID NO: 52 as a 5' arm sequence and a nucleotide sequence set forth in SEQ ID NO:53 as a 3' arm sequence, by a standard method, and this was introduced into mouse ES cells by an electroporation method. The mouse ES cells after gene transfection were subjected to selective culture in the presence of neomycin, and mouse ES cells in which the targeting vector had been incorporated into the chromosome by homologous recombination were selected. The obtained recombinant mouse ES cells were injected into 8-cell stage embryos (host embryos) of ICR mouse, and the embryos were transplanted into pseudo-pregnant mouse (recipient mouse) obtained by mating with vasectomized mouse. The obtained offsprings (chimeric mice) were judged for their hair color, and individuals in which the ES cells had contributed to the formation of the organism with high efficiency, that is, individuals having a high ratio of white hair with respect to the entire hair, were selected. These chimeric mice were crossed with an ICR mouse to obtain F1 mice. White F1 mice were selected, DNA extracted from the tail tissue was analyzed, and a mouse in which the mouse transferrin receptor gene had been replaced with chimeric hTfR on the chromosome was designated as an hTfR-KI mouse.

Each of purified products of the hTfR affinity peptide-trastuzumabs 1 to 5 obtained in Example 9 was intravenously administered to two male hTfR-KI mice at a dose of 5 mg/kg. Furthermore, as a negative control, trastuzumab was intravenously administered to two male hTfR-KI mice at a dose of 5 mg/kg. After 6 and 24 hours from the administration, the mice were euthanized and subjected to whole body perfusion with physiological saline, and the brains (portion including the cerebrum and the cerebellum) were collected. The weights (wet weight) of the excised brains were measured, subsequently each of the brains was divided in the sagittal plane, and one half of the brain was used for measuring the concentration of the hTfR affinity peptide-trastuzumabs 1 to 5 in the tissue, while the other half was subjected to immunohistochemical staining.

The brain tissue for concentration measurement was homogenized in T-PER (Thermo Fisher Scientific, Inc.) containing Protease Inhibitor Cocktail (Sigma-Aldrich Co., LLC). The obtained homogenate was centrifuged (3000 g, 5 min), and the supernatant was collected. The amounts of the hTfR affinity peptide-trastuzumab and trastumab contained in the homogenate supernatant were measured by the following method. First, equal amounts of SULFO-Goat Anti Human IgG (h+1) (Bethyl Laboratories, Inc.) (0.5 µg/mL) and Goat Anti Human Kappa Light chain-biotin (IBL) (0.5 µg/mL) were mixed, the mixture was dispensed on a sampling plate in 25-µL portions, and the sampling plate was shaken with a microplate mixer at room temperature for 1 hour to prepare reaction solutions. SuperBlock blocking buffer in PBS was added to a Streptavidin Gold plate (Meso Scale Diagnostics, LLC) in 150-µL portions, and the plate was blocked by shaking the plate with a microplate mixer at room temperature for 1 hour. The plate was washed with PBST, subsequently the reaction solution was added in 25-µL portions, and the plate was shaken with a microplate mixer at room temperature for 1 hour. Next, 150 µL each of Read buffer T (Meso Scale Diagnostics, LLC) was added to each well, and the amount of luminescence from each well was measured using Sector^{™} Imager 6000 reader. The amount of the hTfR affinity peptide-trastuzumab contained per gram weight (wet weight) of brain (concentration of hTfR affinity peptide-trastuzumab in brain tissue) was calculated by creating a calibration curve from the measured values of standard samples of the hTfR affinity peptide-trastuzumab at known concentrations, and interpolating the measured value of each specimen into this calibration curve. The results are shown in Table 8.

**[Table 8]**

| Table 8 Concentration of hTfR affinity peptide-trastuzumabs 1 to 5 in brain tissue | | |
|---|---|---|
| Name | Concentration in brain tissue (µg/g·tissue) | |
| | After 6 hours from administration | After 24 hours from administration |
| Trastuzumab | 0.07 | 0.04 |
| hTfR affinity peptide-trastuzumab 1 | 0.13 | 0.07 |
| hTfR affinity peptide-trastuzumab 2 | 0.23 | 0.17 |
| hTfR affinity peptide-trastuzumab 3 | 0.37 | 0.15 |
| hTfR affinity peptide-trastuzumab 4 | 0.42 | 0.33 |
| hTfR affinity peptide-trastuzumab 5 | 0.33 | 0.16 |

All of the hTfR affinity peptide-trastuzumabs showed higher values of the concentration in the brain tissue both after 6 hours and after 24 hours from administration, as compared with the negative control group. These results indicate that the hTfR affinity peptide-trastuzumabs have a property of accumulating the BBB in the brain tissue.

### [Example 12] Evaluation 2 of migration of hTfR affinity peptide-trastuzumab to brain using hTfR knock-in mouse

Immunohistochemical staining of the hTfR affinity peptide in the brain tissue was generally performed by the following procedure. The brain tissue collected for immunohistochemical staining was immersed in OCT Compound (Sakura Finetek Japan Co., Ltd.) and rapidly frozen to - 80°C using Histo-Tek PINO (Sakura Finetek Japan Co., Ltd.), and a frozen block of tissue was produced. This frozen block was sliced to a thickness of 4 µm, and the sliced tissue was stuck to a MAS-coated slide glass (Matsunami Glass Industry, Ltd.). The slide glass was immersed in 4% para-formaldehyde (Wako Pure Chemical Industries, Ltd.) at 4°C for 5 minutes, and the specimen was fixed. Next, the slide glass was immersed in PBS containing 1% BSAfor blocking. Next, appropriately diluted Goat Anti hIgG-heavy and light chain Antibody (Bethyl Laboratories, Inc.) was dropped onto the tissue slice and was allowed to react for 1 hour. Next, Fluorescein Amplification Working Solution included in TSA-Plus Fluorescence Kit (PerkinElmer, Inc.) was dropped onto the tissue slice and was allowed to react for 15 minutes in the dark. Next, Anti-Fluorescein-HRP included in CSA II Biotin-Free Tyramide Signal Amplification System (Dako A/S) was dropped onto the tissue slice and was allowed to react for 15 minutes. Next, the tissue slice was reacted with DAB substrate (3,3'-diaminobenzidine, Vector Laboratories, Inc.) to develop color. Next, the tissue slice was counterstained with Mayer's hematoxylin (Merck & Co., Inc.), dehydrated, made transparent, and then enclosed, and the resultant was observed under an optical microscope.

FIG. 1 is optical photographs of tissue slices stained 6 hours after the administration of the hTfR affinity peptide-trastuzumab, and FIG. 2 is optical photographs of tissue slices stained 24 hours after the administration of the hTfR affinity peptide-trastuzumab. In the cerebellar tissue of the mouse administered with trastuzumab as a negative control, no staining was recognized both 6 hours and 24 hours after the administration, which shows that intravenously injected trastuzumab did not reach the cerebellar brain parenchyma, and that there was almost no background staining (FIG. 1(a) and FIG. 2(a)). On the other hand, in the cerebellar tissue of the mouse administered with the hTfR affinity peptide-trastuzumabs 1 to 5, staining was recognized in the brain parenchyma both 6 hours and 24 hours after the administration, which shows that intravenously injected trastuzumab reached the cerebellar brain parenchyma (FIG. 1(b) to FIG. 1(f) and FIG. 2(b) to FIG. 2(f)). Particularly, in the cerebellar tissue of the mouse administered with the hTfR affinity peptide-trastuzumab 3, staining was recognized in the brain parenchyma both 6 hours and 24 hours after the administration, which shows that intravenously injected hTfR affinity peptide-trastuzumab 3 efficiently reached the cerebellar brain parenchyma (FIG. 1(e) and FIG. 2(e)).

### [Example 13] Evaluation of migration of hTfR affinity peptide-trastuzumab to brain using cynomolgus monkey

A purified product of hTfR affinity peptide-trastuzumab 5 obtained in Example 9 and trastumab were each administered once intravenously to one male cynomolgus monkey (Macaca fascicularis) at a dose of 5 mg/kg. Furthermore, as a control, the same volume of physiological saline was intravenously administered to one male cynomolgus monkey. Peripheral blood was collected 20 minutes, 1 hour, 2 hours, 4 ours, and 8 hours after the administration, the concentrations of the hTfR affinity peptide-trastuzumab 5 and trastuzumab contained in the peripheral blood were measured by the method described in Example 11, and Cmax (µg/mL), AUCO-8hr (µg/hr/mL), and t1/2β (hr) were calculated from the measured values. The Cmax (µg/mL), AUCO-8hr (µg/hr/mL), and t1/2β (hr) of the hTfR affinity peptide-trastuzumab 5 were 142, 816, and 25.1, respectively. On the other hand, the Cmax (µg/mL),AUCO-8hr (µg/hr/mL), and t1/2β (hr) of trastuzumab were 110, 509, and 2.27, respectively. These results indicate that the hTfR affinity peptide-trastuzumab 5 is relatively stable in the blood when administered intravenously to a cynomolgus monkey.

Eight hours after the administration, the cynomolgus monkeys were subjected to whole body perfusion with physiological saline. After the perfusion, brain tissue including the medulla oblongata and various organs were extracted. The concentrations of the hTfR affinity peptide-trastuzumab 5 and trastuzumab contained in these extracted brain tissue and various organs were measured by the method described in Example 11.

Regarding the brain tissue, this tissue was divided into cerebral cortex, cerebellum, hippocampus, midbrain, pons, medulla oblongata, caudate nucleus, putamen, globus pallidus, thalamus, hypothalamus, cervical spinal cord, thoracic spinal cord, and lumbar spinal cord, and measurement was performed on each of them. Furthermore, regarding the various organs, retina, liver, kidneys, heart (left ventricle), heart (aortic valve), lungs, bone marrow, spleen, thymus, testes, prostate, rectus femoris muscle, EDL, soleus muscle, triceps, and diaphragm were collected, and measurement was performed on each of them.

FIG. 3 shows the measurement results for the brain tissue. In cerebral cortex, cerebellum, hippocampus, midbrain, pons, medulla oblongata, caudate nucleus, putamen, globus pallidus, and thalamus, trastumab was hardly detected; however, the hTfR affinity peptide-trastuzumab 5 was detected at extremely high concentrations in these organs as compared with trastumab. Furthermore, in hypothalamus as well, the hTfR affinity peptide-trastuzumab 5 was detected at a concentration more than twice the concentration of trastumab. On the other hand, in cervical spinal cord, thoracic spinal cord, and lumbar spinal cord, the hTfR affinity peptide-trastuzumab 5 was detected at lower concentrations than trastumab. In retina, the hTfR affinity peptide-trastuzumab 5 and trastumab were detected at almost the same concentration. These results indicate that the hTfR affinity peptide-trastuzumab 5 administered intravenously to cynomolgus monkeys crosses the blood brain barrier by binding to transferrin present on cerebrovascular endothelial cells, and reaches the tissues in the central nervous system, such as cerebral cortex, cerebellum, hippocampus, midbrain, pons, medulla oblongata, caudate nucleus, putamen, globus pallidus, and thalamus.

FIG. 4 shows the measurement results for various organs. In the heart where HER2 protein, which is a target molecule of trastumab, is expressed, trastumab was detected at high concentrations, and in bone marrow where the expression level of transferrin receptor is high, the hTfR affinity peptide-trastuzumab 5 was detected at high concentrations. This indicates that the hTfR affinity peptide-trastuzumab 5 administered intravenously to cynomolgus monkeys reaches multiple organs, including liver, kidneys, heart, lungs, bone marrow, spleen, testes, and prostate.

### [Example 14] Production of hTfR affinity peptide-hIDS expression vector

pD2535nt-HDP_v2 (HORIZON Discovery Group plc) was digested with PacI and PmeI. A DNA fragment having a PacI site on the 5' side and a PmeI site on the 3' side of a gene having a nucleotide sequence set forth in SEQ ID NO:61 encoding a protein having the amino acid sequence set forth in SEQ ID NO:60, in which hTFR affinity peptide 6 having the amnio acid sequence set forth in SEQ ID NO:55 is bound to the N-terminus of hIDS having the amino acid sequence set forth in SEQ ID NO:58 via a peptide linker having the amino acid sequence set forth in SEQ ID NO:59, was artificially synthesized. This was digested with PacI and PmeI and incorporated between a PacI site and a PmeI site of pD2535nt-HDP_v2. The obtained vector was designated as VHH6-GS3-hIDS expression vector. Furthermore, a protein having the amino acid sequence set forth in SEQ ID NO:60, which is encoded by this expression vector, was designated as VHH6-GS3-hIDS.

pD2535nt-HDP_v2 (HORIZON Discovery Group plc) was digested with PacI and PmeI. A DNA fragment having a PacI site on the 5' side and a PmeI site on the 3' side of a gene having a nucleotide sequence set forth in SEQ ID NO:63 encoding a protein having the amino acid sequence set forth in SEQ ID NO:62, in which hTFR affinity peptide 5 having the amino acid sequence set forth in SEQ ID NO:7 is bound to the N-terminus of hIDS having the amino acid sequence set forth in SEQ ID NO:58 via a peptide linker having the amino acid sequence set forth in SEQ ID NO:59, was artificially synthesized. This was digested with PacI and PmeI and incorporated between a PacI site and a PmeI site of pD2535nt-HDP_v2. The obtained vector was designated as VHH5-GS3-hIDS expression vector. Furthermore, a protein having the amino acid sequence set forth in SEQ ID NO:62, which is encoded by this expression vector, was designated as VHH5-GS3-hIDS.

pD2535nt-HDP_v2 (HORIZON Discovery Group plc) was digested with PacI and PmeI. A DNA fragment having a PacI site on the 5' side and a PmeI site on the 3' side of a gene having a nucleotide sequence set forth in SEQ ID NO:65 encoding a protein having the amino acid sequence set forth in SEQ ID NO:64, in which hTFR affinity peptide 5 having the amino acid sequence set forth in SEQ ID NO:7 is bound to the N-terminus of hIDS having the amino acid sequence set forth in SEQ ID NO:58 via a peptide linker having the amino acid sequence set forth in SEQ ID NO:59, and hTFR affinity peptide 5 is further bound to the N-terminus of this hTFR affinity peptide 5 via a peptide linker having the amino acid sequence shown as GS, was artificially synthesized. This was digested with PacI and PmeI and incorporated between a PacI site and a PmeI site of pD2535nt-HDP_v2. The obtained vector was designated as VHH5-Tandem-GS3-hIDS expression vector. Furthermore, a protein having the amino acid sequence set forth in SEQ ID NO:64, which is encoded by this expression vector, was designated as VHH5-Tandem-GS3-hIDS.

### [Example 15] Production of hTfR affinity peptide-hIDS

GS knockout CHO cells (HD-BIOP3 cells) were transformed by the introduction of the VHH5-GS3-hIDS expression vector produced in Example 14 using a glutamine synthetase (GS) knockout CHO expression platform (HORIZON Discovery Group plc) according to the attached protocol, and VHH5-GS3-hIDS was expressed in HD-BIOP3 cells. pD2535nt-HDP_v2 incorporates GS gene as a selection marker, enabling to transfect a gene into HD-BIOP3 cells and subsequently select in a glutamine-free medium are enabled. Furthermore, it is also possible to provide higher selection pressure to cells by using a medium containing a low concentration (up to 50 µmol/L) of MSX. Cell transformation was generally carried out by the following method. HD-BIOP3 cells were suspended in 15 mL of Dynamis medium (Thermo Fisher Scientific, Inc.) supplemented with L-glutamine at 4 mM, to a concentration of 1×10⁶ cells/mL, and this suspension was transferred into an Erlenmeyer flask having a volume of 150 mL. A solution obtained by mixing 37.5 µL of FreeStyle MAX Reagent (Thermo Fisher Scientific, Inc.) with 600 µL of OptoPro SFM (Thermo Fisher Scientific, Inc.), was mixed with a solution obtained by dissolving 37.5 µg of VHH5-GS3-hIDS expression vector in 600 µL of OptoPro SFM, and the entire amount of this mixture was added to the cell suspension. The cells were cultured for 2 days at 37°C in a humidified environment composed of 5% CO₂ and 95% air at an agitation speed of about 120 rpm, and then the cells were subjected to selective culture in Dynamis medium containing MSX at 25 µM or 35 µM. HD-BIOP3 cells obtained by selective culture express VHH5-GS3-hIDS and secrete this into the culture fluid. These cells were suspended in 200 mL of Dynamis medium to a concentration of 3×10⁵ cells/mL, this suspension was transferred into an Erlenmeyer flask having a volume of 1000 mL, and the cells were cultured for 7 days at 37°C in a humidified environment composed of 5% CO₂ and 95% air at an agitation speed of about 120 rpm, to express VHH5-GS3-hIDS. On the third day and fifth day after the start of culture, 10 mL of EfficientFeed^{™} C+ 2X Supplement (Thermo Fisher Scientific, Inc.) was added. After culturing, a supernatant obtained by centrifuging the culture fluid (3000 g, for 5 minutes) was filtered through a 0.22-µm filter (Millipore Corporation), and this was collected as a culture supernatant.

An open column packed with 1 mL of MabSelect Xtra (Cytiva, Inc.) was equilibrated by passing 5 column volumes of 25 mM HEPES buffer solution (pH 7.5) containing 50 mM NaCl through the column. Next, the culture supernatant was loaded onto the column, and VHH5-GS3-hIDS was bound to the resin. The column was washed by passing 5 column volumes of 25 mM HEPES buffer solution (pH 7.5) containing 50 mM NaCl through the column, and then VHH5-GS3-hIDS was eluted by passing 150 mM Glycine (pH 3.0) containing 100 mM NaCl through the column. A 5% equivalent amount of 1M Tris-HCl (pH 8.0) was added to the eluate containing the VHH5-GS3-hIDS to adjust the pH to neutrality. Next, ultrafiltration was performed using an Ultracel-30 regenerated cellulose membrane (pore size: 30 kDa, Merck & Co., Inc.), and the filtrate was concentrated until the liquid volume became about 1 mL.

The above-described concentrated liquid was loaded onto a size-exclusion column, Superdex 200 increase 10/300 GL column (column volume: 24 mL, bed height: 30 cm, Cytiva, Inc.), which had been equilibrated with 1.5 column volumes of 20 mM Tris buffer solution (pH 7.4) containing 300 mM NaCl, at a constant flow rate of 0.5 mL/min, and the same buffer solution was further supplied at the same flow rate. At this time, an absorption spectrophotometer for continuously measuring the absorbance of the eluate was disposed in the flow path of the eluate from the size-exclusion column, the absorbance at 280 nm was monitored, and a fraction showing an absorption peak at 280 nm was collected as a fraction containing VHH5-GS3-hIDS. Next, the buffer was exchanged with 20 mM Tris buffer solution (pH 7.4) containing 50 mM NaCl using an Ultracel-30 regenerated cellulose membrane, and a concentrated liquid thus obtained was designated as a purified product of VHH5-GS3-hIDS.

For VHH6-GS3-hIDS as well, a purified product of VHH6-GS3-hIDS was obtained by a method similar to that for VHH5-GS3-hIDS, from a culture supernatant obtained by culturing HD-BIOP3 cells into which a VHH6-GS3-hIDS expression vector had been introduced. Furthermore, also for VHH5-Tandem-GS3-hIDS, a purified product of VHH5-Tandem-GS3-hIDS was obtained by a method similar to that for VHH5-GS3-hIDS, from a culture supernatant obtained by culturing HD-BIOP3 cells into which a VHH5-Tandem-GS3-hIDS expression vector had been introduced. Three kinds of proteins, namely, VHH5-GS3-hIDS, VHH6-GS3-hIDS, and VHH5-Tandem-GS3-hIDS, were collectively referred to as hTfR affinity peptide-hIDS.

### [Example 16] Evaluation of binding activity of hTfR affinity peptide-hIDS to human TfR and monkey TfR

With regard to the proteins of VHH5-GS3-hIDS, VHH6-GS3-hIDS, and VHH5-Tandem-GS3-hIDS obtained by standard methods using CHO cells, the binding activity to human transferrin receptor (hTfR) was measured by an ELISA method. The ELISA method was generally carried out by the following method. A recombinant hTfR containing the extracellular region of hTfR, which contained the amino acid sequence from the N-terminus to the 89^{th} residue in the amino acid sequence set forth in SEQ ID NO: 1, was diluted with a 0.05 M hydrogen bicarbonate buffer solution (pH 9.6) to a concentration of 10 µg/mL, 100 µL of the dilution was added to each well of a 96-well microtiter plate (Nunc A/S), and the plate was left to stand for at least one hour at room temperature to allow the antibody to adsorb to the plate. Next, 300 µL of TBS containing 1% BSA and 0.05% Tween 20 was added to each well, and the plate was left to stand for one hour at room temperature. Each well was washed three times with TBS containing 0.05% Tween 20 (TBS-T), subsequently VHH5-GS3-hIDS was diluted to 100, 33.33, 11.11, 3.71, 1.24, 0.41, and 0.14 nM, VHH6-GS3-hIDS was diluted to 333, 111, 37, 12.33, 4.11, 1.37, and 0.46, and VHH5-Tandem-GS3-hIDS was diluted to 1, 0.33, 0.11, 0.037, 0.012, 0.041, and 0.014 nM, using TBS containing 0.1% BSA and 0.05% Tween 20. 100 µL each of the dilutions were added to each well, and the plate was left to stand for at least one hour at room temperature. The plate was washed three times with TBS-T, subsequently 100 µL of an anti-hIDS monoclonal antibody diluted to 0.5 µg/mL with TBS containing 0.1% BSA and 0.05% Tween 20 was added to each well, and the plate was left to stand for at least one hour at room temperature. After washing three times with TBS-T, 100 µL of an HRP-labeled anti-mouse IgG polyclonal antibody (Bethyl Laboratories, Inc.) solution diluted to 50 ng/mL with TBS containing 0.1% BSA and 0.05% Tween 20, was added to each well, and the plate was left to stand for at least one hour at room temperature. Each well was washed three times with TBS-T, subsequently 50 µL of a substrate solution for color development, TMB Stabilized Substrate for Horseradish Peroxidase (Promega Corporation), was added to each well, and the plate was left to stand for 5 to 10 minutes at room temperature. Next, 100 µL of a stop solution (1 N hydrochloric acid) was added to each well, and then the absorbance at 450 nm of each well was measured using a plate reader (MOLECULAR DEVICES, LLC). EC₅₀ was calculated from the obtained measured values. As a result, the EC₅₀ values of VHH5-GS3-hIDS, VHH6-GS3-hIDS, and VHH5-Tandem-GS3-hIDS were 1.087, 22.67, and 0.077 nM, respectively. These results indicate that all of these three types of hTfR affinity peptide-hIDS have relatively high affinity for hTfR.

### [Example 17] Production of IDS-KO/hTfR-KI mouse

An IDS-KO/hTfR-KI mouse is a mouse that is hemi-deficient in the iduronate-2-sulfatase (IDS) gene and is heterozygous for the chimeric TfR gene. IDS-KO/hTfR-KI mouse was generally produced by the following method. A DNA fragment in which a neomycin resistance gene flanked by loxP sequences was disposed on the 3' side of a cDNA encoding a chimeric TfR whose intracellular region had the amino acid sequence of mouse TfR and whose extracellular region had the amino acid sequence of human TfR, was chemically synthesized. This DNA fragment was incorporated into a targeting vector having a 5' arm sequence and a 3' arm sequence by a standard method, and this was introduced into mouse ES cells by an electroporation method. The mouse ES cells after gene transfection were subjected to selective culture in the presence of neomycin, and mouse ES cells in which the targeting vector had been incorporated into the chromosome by homologous recombination were selected. The obtained recombinant mouse ES cells were injected into 8-cell stage embryos (host embryos) of ICR mouse, and the embryos were transplanted into pseudo-pregnant mouse (recipient mouse) obtained by mating with vasectomized mouse. The obtained offsprings (chimeric mice) were judged for their hair color, and individuals in which the ES cells had contributed to the formation of the organism with high efficiency, that is, individuals having a high ratio of white hair with respect to the entire hair, were selected. These chimeric mice were crossed with ICR mouse to obtain F1 mice. White F1 mice were selected, DNA extracted from the tail tissue was analyzed, and a mouse in which the mouse hTfR gene had been replaced with chimeric TfR on the chromosome was designated as an hTfR-KI mouse. Based on this mouse, a mouse that was hemi-deficient in the IDS gene and was heterozygous for the chimeric TfR gene (IDS-KO/hTfR-KI mouse), was produced. Production of the IDS-KO/hTfR-KI mouse was carried out according to the technique described in Patent Literature (WO 2016/208695).

### [Example 17] Evaluation of drug efficacy of each protein using IDS-KO/hTfR-KI mouse

The drug efficacy of hTfR affinity peptide-hIDS was evaluated by measuring the concentration of heparan sulfate (HS), which is known to accumulate in the organs of patients with Hunter syndrome, who genetically lack hIDS activity. The method will be described in detail below.

Purified products of the three kinds of proteins, VHH5-GS3-hIDS, VHH6-GS3-hIDS, and VHH5-Tandem-GS3-hIDS, obtained in Example 15 were diluted with physiological saline to produce solutions containing each of the proteins at a concentration of 0.4 mg/mL.

Diluted VHH5-GS3-hIDS, VHH6-GS3-hIDS, and VHH5-Tandem-GS3-hIDS were administered to 50-week-old IDS-KO/hTfR KI mice at a dose of 2 mg/kg through tail vein injection for a total of three times at a frequency of once a week. Furthermore, mice with wild-type IDS gene were used as wild-type controls, and hemi-IDS-KO mice were used as pathological controls. These control mice were administered with physiological saline for a total of three times at a frequency of once a week. The liquid volume administered to each group was adjusted to be almost the same volume. The genotypes of the mice used in this case are as shown in Table 10. The number of mouse individuals in each group is also as shown in Table 10.

**[Table 9]**

| (Table 9)Genotype of mouse used for evaluation of drug efficacy of hTfR affinity peptide-hIDS, and dosage regimen of hTfR affinity peptide-hIDS | | | | | | | |
|---|---|---|---|---|---|---|---|
| Group | Genotype (hTfR/ids) | Administered drug | Dose (mg/kg) | Administered liquid volume (mL/kg) | Administration schedule | Route of administration | Number of individuals |
| Wild-type control | Hetero/WT | Physiological saline | - | 5 | Once a week (total of three times) | Intravenous injection | 3 |
| Pathological control | Hetero/Hemi | Physiological saline | - | | | | 3 |
| VHH6-GS3-hIDS-administered | | VHH6-GS3-hIDS-administered | 2 | | | | 5 |
| VHH5-GS3-hIDS-administered | | VHH5-GS3-hIDS | 2 | | | | 5 |
| VHH5-tandem-GS3-hIDS-administered | | VHH5-tandem-GS3-hIDS | 2 | | | | 2 |

One week after the third administration, CSF was collected from the cisterna magna of each mice under a triple anesthesia regimen of Betorfal (Meiji Seika Pharma Co., Ltd.), Midazolam (Sandoz Group AG), and Domitor (Nippon Zenyaku Kogyo Co., Ltd.). After the CSF collection, the mice were euthanized by exsanguination. Next, the mouse brains were extracted and rapidly frozen.

### [Example 18] Quantification of heparan sulfate in brain tissue and CSF

Quantification of heparan sulfate (HS) in the brain was generally carried out by the following method. Incidentally, heparan sulfate is a substrate for hIDS.

Solutions (a) to (l) used in the test were prepared by the following procedure.
(a) MeCN/water:
   2 mL of water for injection (Otsuka Pharmaceutical Factory, Inc.) and 18 mL of acetonitrile (Fujifilm Wako Pure Chemical Corporation) were mixed, and this was used as MeCN/water.
(b) Deuterium-labeled solvent:
   In an ice bath, 240 µL of acetyl chloride (Sigma-Aldrich Co., LLC) was added dropwise to 1.5 mL of methanol-d₄ (Sigma-Aldrich Co., LLC), and this was used as a deuterium-labeled solvent.
(c) Mobile phase A:
   A mixture of 475 mL of water for injection and 25 mL of 1M aqueous solution of ammonium formate (Fujifilm Wako Pure Chemical Corporation) was used as mobile phase A.
(d) Mobile phase B:
   A mixture of acetonitrile and the mobile phase A at a ratio of 93:7 (v/v) was used as mobile phase B.
(e) Heparan sulfate standard stock solution (HS standard stock solution):
   Heparan sulphate (Iduron, Ltd.) was dissolved in water for injection to prepare a 5.0 mg/mL solution. This solution was used as an HS standard stock solution.
(f) Heparan sulfate internal standard solution (HS internal standard solution):
   40 µL of the HS standard stock solution was measured into a borosilicate screw-capped test tube, and the solvent was distilled off under a nitrogen gas stream. 400 µL of the deuterium-labeled solvent was added to the solid-dried product, and the mixture was stirred and then reacted at 65°C for 75 minutes to perform a deuterium methanolysis reaction. After the reaction, the solvent was distilled off under a nitrogen gas stream. 500 µL of MeCN/water was added to the solid-dried product, and the mixture was subjected to ultrasonication for 30 minutes. This solution was used as a heparan sulfate internal standard solution (HS internal standard solution).
(i) Internal standard solution:
   1 µL of the HS internal standard solution was added to 1 mL of methanol, and the mixture was stirred and then subjected to ultrasonication for 30 minutes. This solution was used as an internal standard solution.
(j) Calibration curve samples:
   980 µL of water for injection was measured, and the 10 µL of HS standard stock solution was added thereto to prepare a solution containing 50 µg/mL of heparan sulfate. This solution was diluted with water for injection, and a solution containing 5000 ng/mL of heparan sulfate was prepared. This solution was diluted stepwise with water for injection, and solutions containing heparan sulfate at concentrations of 25, 50, 100, 250, 500, 1000, 2500, and 5000 ng/mL were prepared. 20 µL of each of these solutions was measured and dispensed into a borosilicate screw-capped test tube. These solutions were used as calibration curve samples.
(k) Solution for tissue extraction
   1 mL of polyoxyethylene (10) octylphenyl ether was added to physiological saline, and the total amount was adjusted to 500 mL. This solution was used as a solution for tissue extraction.
(l) 10% ammonium carbonate solution
   5 g of ammonium carbonate was dissolved in 50 mL of water for injection. This solution was used as 10% ammonium carbonate solution.

20 µL of the CSF collected in Example 17 was measured and dispensed into a borosilicate screw-capped test tube. This was used as a CSF sample solution.

The brain tissue extracted in Example 17 was freeze-dried, and the dry weight was measured. The freeze-dried tissue was crushed in the solution for tissue extraction, and then a supernatant was centrifuged. 20 µL of this was measured and dispensed into a borosilicate screw-capped test tube. This was used as a sample solution.

The solvent in each sample solution and calibration curve sample prepared was distilled off under a nitrogen gas stream. To each solid-dried product, 20 µL of 2,2-dimethoxypropane (Tokyo Chemical Industry Co., Ltd.) and 200 µL of hydrogen chloride methanol (Sigma-Aldrich Co., LLC) with a hydrochloric acid concentration of 3 mol/L were added and stirred, and then a methanolysis reaction was carried out at a reaction temperature of 70°C for a reaction time of 90 minutes. After the reaction, the reaction was terminated by cooling on ice, and 200 µL of 10% ammonium carbonate solution and 50 µL of the internal standard solution were added thereto. The solvent was distilled off under a nitrogen gas stream, and then 250 µL of water for injection was added to the solid-dried product. This was loaded onto a solid phase cartridge (OASIS HLB (1 cc, 30 mg, Waters Corporation)) that had been conditioned in advance by passing methanol and water for injection therethrough, and after washing with water for injection, 500 µL of methanol was added thereto, and elution was performed by centrifuging. The solvent was distilled off under a nitrogen gas stream, subsequently 2 mL of water for injection and 18 mL of acetonitrile were added thereto, and the dried product was redissolved by ultrasonication. This was centrifuged, and then the supernatant was charged into an LC vial.

LC/MS/MS analysis was carried out using hydrophilic interaction ultrahigh performance liquid chromatography coupled with a tandem quadrupole mass spectrometer. As the mass spectrometer (MS/MS apparatus), QTRAP5500 (AB Sciex, LLC) was used, and Nexera X2 (Shimadzu Corporation) was added thereto as an HPLC apparatus. Furthermore, an Acquity UPLC^{™} BEH Amide 1.7 µm (2.1 × 150 mm, Waters Corporation) was used as an LC column. The mobile phase A and the mobile phase B were used as the mobile phase. The column temperature was set to 60°C.

The column was equilibrated with the mobile phase B, subsequently 5 µL of a sample was injected, and chromatography was carried out under the mobile phase gradient conditions shown in Table 11. The flow rate of the mobile phase was set to 0.4 mL/min.

**[Table 10]**

| (Table 10) Conditions for liquid chromatography | | |
|---|---|---|
| Elapsed time after sample injection (minutes) | Mobile phase A (% (v/v)) | Mobile phase B (% (v/v)) |
| 0 | 0 | 100 |
| 5.00 | 0 | 100 |
| 7.00 | 20 | 80 |
| 12.5 | 20 | 80 |
| 13.0 | 70 | 30 |
| 15.0 | 70 | 30 |
| 15.5 | 0 | 100 |
| 20.0 | 0 | 100 |

The ion source parameters of the MS/MS apparatus were set as shown in Table 12 according to the instruction manual of QTRAP5500 (AB Sciex, LLC).

**[Table 11]**

| (Table 11) Setting of various parameters for ion source of MS/MS apparatus | |
|---|---|
| Ion Source | ESI (Turbo Spray) |
| Polarity | Positive |
| Scan type | MRM |
| IonSpray viltage | 5500 V |
| Heater gas temperature | 425°C |
| Curtain gas (CUR) | 30.00 |
| Collision gas (CAD) | 8.00 |
| Ion Source Gas 1 (GS1) | 70.00 |
| Ion Source Gas 2 (GS2) | 70.00 |
| Entrance Potential | 10.00 |
| Duration | 15 .00min |

Table 13 shows the MS internal parameters.

**[Table 12]**

| (Table 12) MS internal parameters | | | | | | |
|---|---|---|---|---|---|---|
| | Precursor ion (m/z) Q1 | Product ion (m/z) Q3 | Time (msec) | DO (volts) | collision energy (volts) | CXP (volts) |
| Substance derived from HS | 384.100 | 162.100 | 100.00 | 45.00 | 21.00 | 15.00 |
| HS internal standard substance | 390.200 | 162.100 | 100.00 | 45.00 | 21.00 | 15.00 |
| Substance derived from DS | 426.100 | 236.100 | 100.00 | 45.00 | 12.00 | 17.00 |
| DS internal standard substance | 432.200 | 239.200 | 100.00 | 45.00 | 12.00 | 17.00 |

LC/MS/MS analysis was performed for each calibration curve sample, and the area of the peak on the chromatogram chart corresponding to the product ion derived from heparan sulfate in the calibration curve sample (HS detection peak area) was determined. Furthermore, the area of a detection peak corresponding to the product ion derived from the HS internal standard solution (HS-IS detection peak area) was determined.

The area of the detection peak derived from heparan sulfate with respect to the detection peak area derived from the HS internal standard solution (HS detection peak area / HS-IS detection peak area) in each calibration curve sample was plotted on the axis of ordinate, the heparan sulfate concentration in each calibration curve sample was plotted on the axis of abscissa, and a regression equation was obtained using quadratic discriminant analysis.

The brain tissue sample solution was subjected to LC/MS/MS analysis, and the heparan sulfate contained in the sample solution was quantified by interpolating in the regression equation.

FIG. 5 shows the measurement results for HS contained in CSF. In the groups administered with VHH5-GS3-hIDS, VHH6-GS3-hIDS, and VHH5-Tandem-GS3-hIDS, the concentration of HS contained in the CSF was reduced compared to the pathological control in all cases, and it is understood that all of these passed through the BBB, reached the central nervous system, and degraded the HS that had accumulated there. Regarding VHH5-Tandem-GS3-hIDS, the concentration of HS contained in the CSF was less than 40% compared to the pathological control, and from this, it is understood that linking two hTfR affinity peptides in tandem is an effective means to allow the fusion protein to pass through the BBB.

FIG. 6 shows the measurement results for HS contained in brain tissue. In the groups administered with VHH5-GS3-hIDS, VHH6-GS3-hIDS, and VHH5-Tandem-GS3-hIDS, the concentration of HS contained in the brain tissue was reduced compared to the pathological control in all cases, and it is understood that all of these passed through the BBB, reached the brain tissue, and degraded the HS that had accumulated there. Regarding VHH5-Tandem-GS3-hIDS, the concentration of HS contained in the brain tissue was reduced by almost half compared to the pathological control, and from this, it is understood that linking two hTfR affinity peptides in tandem is an effective means to allow the fusion protein to pass through the BBB. Furthermore, it can be predicted from these results that when hIDS fused to an hTfR affinity peptide is intravenously injected to a patient with Hunter syndrome, the fusion protein can pass through the BBB, reach the central nervous system of the patient, and exert drug efficacy by degrading the HS that has accumulated there.

### [Example 18] Production of hTfR affinity peptide-hSGSH expression vector

pCI-neo Mammalian Expression Vector (Promega Corporation) was digested with MluI and NotI. A DNA fragment having an Mini site on the 5' side and a NotI site on the 3' side of a gene having a nucleotide sequence set forth in SEQ ID NO:68 encoding a protein having the amino acid sequence set forth in SEQ ID NO:67, in which heparan N-sulfatase (hSGSH) having a DYKDDDDK tag added at the N-terminus set forth in SEQ ID NO:66 is bound to the C-terminus of the hTfR affinity peptide 3 having the amino acid sequence set forth in SEQ ID NO:5 via a peptide linker having the amino acid sequence set forth in SEQ ID NO:59, was artificially synthesized. This was digested with Mini and NotI and incorporated between an MluI site and a NotI site of pCI-neo Mammalian Expression Vector, and an expression vector for VHH3-GS3-hSGSH was produced. This expression vector was named pCI-neo-VHH3-GS3-hSGSH.

### [Example 19] Production of hTfR affinity peptide-hSGSH

The pCI-neo-VHH3-GS3-hSGSH produced in Example 18 was introduced into CHO-S cells using ExpiCHO Expression System (Thermo Fisher Scientific, Inc.) according to the attached protocol, and a protein (VHH3-GS3-hSGSH) in which the hTfR affinity peptide 3 and hSGSH were bound , was expressed in the CHO-S cells. These cells express VHH3-GS3-hSGSH and secrete this into the culture fluid. Here, the CHO-S cells into which pCI-neo-VHH3-GS3-hSGSH had been introduced were designated as pCI-neo-VHH3-GS3-hSGSH-expressing cells. Incidentally, a fusion protein of an hTfR affinity peptide and hSGSH will be referred to as hTfR affinity peptide-hSGSH.

The pCI-neo-VHH3-GS3-hSGSH-expressing cells were cultured according to the protocol attached to the ExpiCHO Expression System. Specifically, cells were suspended in 15 mL of a culture medium so as to have a concentration of 6×10⁶ cells/mL, this suspension was transferred into an Erlenmeyer flask having a volume of 150 mL, and the cells were cultured for one day at 37°C in a humidified environment composed of 5% CO₂ and 95% air at an agitation speed of about 120 rpm. The Feed liquid and Enhancer liquid included in the ExpiCHO Expression System were added to the culture, and the cells were cultured for 9 days at 32°C in a humidified environment composed of 5% CO₂ and 95% air at an agitation speed of about 120 rpm. After culturing, a supernatant obtained by centrifuging the culture fluid (3000 g, for 5 minutes) was filtered through a 0.22-µm filter (Millipore Corporation), and this was collected as a culture supernatant.

An open column packed with 1 mL of anti-FLAG M1 antibody-agarose-bound affinity gel (MERCK & Co., Inc.) was equilibrated by passing 5 column volumes of 50 mM Tris buffer solution (pH 7.5) containing 150 mM NaCl and 1 mM CaCl₂ through the column. Furthermore, the culture supernatant was prepared to be 1 mM CaCl₂ by adding 1 M CaCl₂ at a ratio of 1/1000. Next, the culture supernatant was loaded onto the column so as to let VHH3-GS3-hSGSH bound to the resin. The column was washed by passing 5 column volumes of 50 mM Tris buffer solution (pH 7.5) containing 150 mM NaCl and 1 mM CaCl₂ through the column, and then VHH3-GS3-hSGSH was eluted by passing 50 mM Tris buffer solution (pH 7.5) containing 150 mM NaCl and 2 mM EDTA through the column. This was subjected to buffer exchange using a PD-10 column (Cytiva, Inc.), for the solvent to be replaced with 20 mM phosphate buffer solution containing 7.5 mg/mL sucrose and 0.8 mg/mL NaCl. The obtained solution was used in the following experiments as a purified product of VHH3-GS3-hSGSH.

### [Example 20] Evaluation of binding activity of hTfR affinity peptide-hSGSH to human TfR and monkey TfR

The affinity of the VHH-GS3-hSGSH produced in Example 19 for hTfR and monkey TfR was examined according to the method described in Example 7. Table 13 shows the measurement results for the association rate constants (kon), the dissociation rate constants (koff) , and the dissociation constant (K_{D}) of VHH3-GS3-hSGSH for hTfR and monkey TfR (cynomolgus monkey TfR). Regarding the cynomolgus monkey TfR, a recombinant monkey TfR (r-monkey TfR, Sino Biological, Inc.) having the amino acid sequence of the extracellular region of cynomolgus monkey TfR, from the 89^{th} cysteine residue from the N-terminus to phenylalanine at the C-terminus in the amino acid sequence set forth in SEQ ID NO:2, with a histidine tag added at the N-terminus, was used as the monkey TfR.

**[Table 13]**

| (Table 13) Binding activity of hTfR affinity peptide-hSGSH toward human TfR and monkey TfR | | | | |
|---|---|---|---|---|
| | Animal species | kon (1/Ms) | koff (1/s) | k_{D} (M) |
| VHH3-GS3-hSGSH | Cynomolgus monkey | 4.17 × 10⁵ | 4.81 × 10⁻⁷ | 1.16 × 10⁻¹² |
| | Human | 4.23 × 10⁵ | < 1.0 × 10⁻⁷ | < 1.0 × 10⁻¹² |

### [Example 21] Evaluation of drug efficacy of hTfR affinity peptide-hSGSH

The drug efficacy of hTfR affinity peptide-hSGSH was evaluated by measuring the concentration of heparan sulfate (HS), which is known to accumulate in the organs of patients with Sanfilippo syndrome A who genetically lack the hSGSH activity. The method will be described in detail below.

An hTfR-KI mouse produced by the method described in Example 17 was crossed with an SGSH gene knockout mouse (SGSH-KO mouse), which is a model mouse for Sanfilippo syndrome A, to obtain an SGSH-KO/hTfR KI mouse.

The VHH3-GS3-hSGSH obtained in Example 21 was diluted with physiological saline, and solutions each containing each of the proteins at a concentration of 0.4 mg/mL were produced.

Diluted VHH3-GS3-hSGSH was administered to 16- to 19-week-old SGSH-KO/hTfR KI mice at a dose of 2 mg/kg through tail vein injection for a total of four times at a frequency of once a week. Furthermore, mice with wild-type SGSH gene were used as wild-type controls, and homozygous SGSH-KO mice were used as pathological controls. These control mice were administered with physiological saline for a total of four times at a frequency of once a week. The liquid volume administered to each group was adjusted to be almost the same volume. The genotypes of the SGSH-KO/hTfR KI mouse used in this case are as shown in Table 12. The number of mice in each group is also as shown in Table 14.

**[Table 14]**

| (Table 14) Genotype of mouse used for evaluation of drug efficacy of hTfR affinity peptide-hSGSH, and dosage regimen of hTfR affinity peptide-hSGSH | | | | | | | |
|---|---|---|---|---|---|---|---|
| Group | Genotype (hTfR/sgsh) | Administered drug | Dose (mg/kg) | Administered liquid volume (mL/kg) | Administration schedule | Route of administration | Number of individuals |
| Wild-type control | WT/WT | Physiological saline | - | 5 | Once a week (total of four times) | Intravenous injection | 3 |
| Pathological control | Hemi/KO | Physiological saline | - | | | | 3 |
| VHH3-GS3-hSGSH-administered | | VHH3-GS3-hSGSH | 2 | | | | 3 |

One week after the fourth administration, CSF was collected from the cisterna magna of each mice under a triple anesthesia regimen of Betorfal (Meiji Seika Pharma Co., Ltd.), Midazolam (Sandoz Group AG), and Domitor (Nippon Zenyaku Kogyo Co., Ltd.). After the CSF collection, the mice were euthanized by exsanguination. Next, the mouse brains were extracted and rapidly frozen. The amounts of HS contained in the CSF and the brain tissue were measured by the method described in Example 18.

FIG. 7 shows the measurement results for HS contained in brain tissue and CSF. In the VHH3-GS3-hSGSH-administered group, the concentration of HS in the brain tissue was reduced by about 37% as compared to the pathological control, and it is understood that VHH3-GS3-hSGSH passed through the BBB, reached the central nervous system, and degraded the HS that had accumulated there (FIG. 7(a)). Furthermore, in the VHH3-GS3-hSGSH-administered group, the concentration of HS contained in the CSF was reduced by about 13% as compared to the pathological control, and it is understood that VHH3-GS3-hSGSH passed through the BBB, reached the central nervous system, and degraded the HS that had accumulated there (FIG. 7(b)). From these results, it can be predicted that when hSGSH fused to an hTfR affinity peptide is intravenously injected to a patient with Sanfilippo syndrome A, the fusion protein can pass through the BBB, reach the central nervous system of the patient, and exert drug efficacy by degrading the HS that has accumulated there.

### Industrial Applicability

According to an embodiment of the present invention, there can be provided a drug in which a human transferrin receptor affinity peptide and a pharmacologically active substance are bound, and which can pass through the blood brain barrier and exert its efficacy in the central nervous system.

### Reference Signs List

1: blood vessel, 2: brain parenchyma.

### Sequence Listing Free Text

SEQ ID NO:1: Amino acid sequence of human transferrin receptor
SEQ ID NO:2: Amino acid sequence of cynomolgus monkey transferrin receptor
SEQ ID NO:3: Amino acid sequence of hTfR affinity peptide 1
SEQ ID NO:4: Amino acid sequence of hTfR affinity peptide 2
SEQ ID NO:5: Amino acid sequence of hTfR affinity peptide 3
SEQ ID NO:6: Amino acid sequence of hTfR affinity peptide 4
SEQ ID NO:7: Amino acid sequence of hTfR affinity peptide 5
SEQ ID NO:8: Amino acid sequence 1 of CDR1 of hTfR affinity peptide 1
SEQ ID NO:9: Amino acid sequence 2 of CDR1 of hTfR affinity peptide 1
SEQ ID NO:10: Amino acid sequence 1 of CDR2 of hTfR affinity peptide 1
SEQ ID NO:11: Amino acid sequence 2 of CDR2 of hTfR affinity peptide 1
SEQ ID NO:12: Amino acid sequence 1 of CDR3 of hTfR affinity peptide 1
SEQ ID NO:13: Amino acid sequence 2 of CDR3 of hTfR affinity peptide 1
SEQ ID NO: 14: Amino acid sequence 1 of CDR1 of hTfR affinity peptide 2
SEQ ID NO: 15: Amino acid sequence 2 of CDR1 of hTfR affinity peptide 2
SEQ ID NO: 16: Amino acid sequence 1 of CDR2 of hTfR affinity peptide 2
SEQ ID NO: 17: Amino acid sequence 2 of CDR2 of hTfR affinity peptide 2
SEQ ID NO: 18: Amino acid sequence 1 of CDR3 of hTfR affinity peptide 2
SEQ ID NO: 19: Amino acid sequence 2 of CDR3 of hTfR affinity peptide 2
SEQ ID NO:20: Amino acid sequence 1 of CDR1 of hTfR affinity peptide 3
SEQ ID NO:21: Amino acid sequence 2 of CDR1 of hTfR affinity peptide 3
SEQ ID NO:22: Amino acid sequence 1 of CDR2 of hTfR affinity peptide 3
SEQ ID NO:23: Amino acid sequence 2 of CDR2 of hTfR affinity peptide 3
SEQ ID NO:24: Amino acid sequence 1 of CDR3 of hTfR affinity peptide 3
SEQ ID NO:25: Amino acid sequence 2 of CDR3 of hTfR affinity peptide 3
SEQ ID NO:26: Amino acid sequence 1 of CDR1 of hTfR affinity peptide 4
SEQ ID NO:27: Amino acid sequence 2 of CDR1 of hTfR affinity peptide 4
SEQ ID NO:28: Amino acid sequence 1 of CDR2 of hTfR affinity peptide 4
SEQ ID NO:29: Amino acid sequence 2 of CDR2 of hTfR affinity peptide 4
SEQ ID NO:30: Amino acid sequence 1 of CDR3 of hTfR affinity peptide 4
SEQ ID NO:31: Amino acid sequence 2 of CDR3 of hTfR affinity peptide 4
SEQ ID NO:32: Amino acid sequence of peptide linker 1
SEQ ID NO:33: Amino acid sequence of peptide linker 2
SEQ ID NO:34: Amino acid sequence of peptide linker 3
SEQ ID NO:35: Amino acid sequence of peptide linker 4
SEQ ID NO:36: Amino acid sequence of peptide linker 5
SEQ ID NO: 37: One example of amino acid sequence of Fc region of human IgG
SEQ ID NOG 8: Amino acid sequence of human serum albumin
SEQ ID NO:39: Albumin-binding domain of protein derived from Streptococcus strain G418
SEQ ID NO:40: Amino acid sequence of trastuzumab heavy chain
SEQ ID NO:41: Amino acid sequence of trastuzumab light chain
SEQ ID NO:42: Amino acid sequence of fusion protein of hTfR affinity peptide 1 and trastuzumab heavy chain
SEQ ID NO:43: Amino acid sequence of fusion protein of hTfR affinity peptide 2 and trastuzumab heavy chain
SEQ ID NO:44: Amino acid sequence of fusion protein of hTfR affinity peptide 3 and trastuzumab heavy chain
SEQ ID NO:45: Amino acid sequence of fusion protein of hTfR affinity peptide 4 and trastuzumab heavy chain
SEQ ID NO:46: Amino acid sequence of fusion protein of hTfR affinity peptide 5 and trastuzumab heavy chain
SEQ ID NO:47: Nucleotide sequence of DNA encoding fusion protein of hTfR affinity peptide 1 and trastuzumab heavy chain, synthesized sequence
SEQ ID NO:48: Nucleotide sequence of DNA encoding trastuzumab light chain, synthesized sequence
SEQ ID NO:49: Nucleotide sequence of primer hTfR5', synthesized sequence
SEQ ID NO:50: Nucleotide sequence of primer hTfR3', synthesized sequence
SEQ ID NO: 51: Nucleotide sequence of DNA in which neomycin resistance gene flanked by loxP sequences is disposed on 3' side of cDNA encoding chimeric hTfR, synthesized sequence
SEQ ID NO: 52: Nucleotide sequence of 5' arm of targeting vector, synthesized sequence
SEQ ID NO:53: Nucleotide sequence of 3' arm of targeting vector, synthesized sequence
SEQ ID NO:54: Peptide tag
SEQ ID NO:55: Amino acid sequence of hTfR affinity peptide 6
SEQ ID NO:56: Amino acid sequence 1 of CDR2 of hTfR affinity peptide 6
SEQ ID NO:57: Amino acid sequence 2 of CDR2 of hTfR affinity peptide 6
SEQ ID NO: 58: Amino acid sequence of peptide linker 6
SEQ ID NO:59: Amino acid sequence of human I2S
SEQ ID NO:60: Amino acid sequence of VHH6-GS3-hI2S
SEQ ID NO:61: Nucleotide sequence of VHH6-GS3-hI2S
SEQ ID NO:62: Amino acid sequence of VHH5-GS3-hI2S
SEQ ID NO:63: Nucleotide sequence of VHH5-GS3-hI2S
SEQ ID NO:64: Amino acid sequence of VHH5-Tandem-GS3-hI2S
SEQ ID NO:65: Nucleotide sequence of VHH5-Tandem-GS3-hI2S
SEQ ID NO:66: Amino acid sequence of human SGSH
SEQ ID NO:67: Amino acid sequence of VHH3-GS3-hSGSH
SEQ ID NO:68: Nucleotide sequence of VHH3-GS3-hSGSH
SEQ ID NO:69: Amino acid sequence of peptide linker 7

## Claims

1. A peptide having affinity for human transferrin receptor (human transferrin receptor affinity peptide), the peptide comprising a variable region of a heavy chain antibody having three complementarity-determining regions, CDR1, CDR2, and CDR3, and being selected from the group consisting of the following (1) to (5):
(1) a peptide in which the amino acid sequence of CDR1 is the amino acid sequence of SEQ ID NO:8 or SEQ ID NO:9, the amino acid sequence of CDR2 is the amino acid sequence of SEQ ID NO: 10 or SEQ ID NO: 11, and the amino acid sequence of CDR3 is the amino acid sequence of SEQ ID NO: 12 or SEQ ID NO: 13;
(2) a peptide in which the amino acid sequence of CDR1 is the amino acid sequence of SEQ ID NO: 14 or SEQ ID NO: 15, the amino acid sequence of CDR2 is the amino acid sequence of SEQ ID NO: 16 or SEQ ID NO: 17, and the amino acid sequence of CDR3 is the amino acid sequence of SEQ ID NO: 18 or SEQ ID NO: 19;
(3) a peptide in which the amino acid sequence of CDR1 is the amino acid sequence of SEQ ID NO:20 or SEQ ID NO:21, the amino acid sequence of CDR2 is the amino acid sequence of SEQ ID NO:22 or SEQ ID NO:23, and the amino acid sequence of CDR3 is the amino acid sequence of SEQ ID NO:24 or SEQ ID NO:25;
(4) a peptide in which the amino acid sequence of CDR1 is the amino acid sequence of SEQ ID NO:26 or SEQ ID NO:27, the amino acid sequence of CDR2 is the amino acid sequence of SEQ ID NO:28 or SEQ ID NO:29, and the amino acid sequence of CDR3 is the amino acid sequence of SEQ ID NO:30 or SEQ ID NO:31; and
(5) a peptide in which the amino acid sequence of CDR1 is the amino acid sequence of SEQ ID NO:20 or SEQ ID NO:21, the amino acid sequence of CDR2 is the amino acid sequence of SEQ ID NO:56 or SEQ ID NO:57, and the amino acid sequence of CDR3 is the amino acid sequence of SEQ ID NO:24 or SEQ ID NO:25.

2. A human transferrin receptor affinity peptide selected from the group consisting of the following (1) to (6):
(1) a peptide having the amino acid sequence identity of 80% or higher to the amino acid sequence of SEQ ID NO:3 and having three complementarity-determining regions, CDR1, CDR2, and CDR3, having the amino acid sequences shown in (1) of claim 1;
(2) a peptide having the amino acid sequence identity of 80% or higher to the amino acid sequence of SEQ ID NO:4 and having three complementarity-determining regions, CDR1, CDR2, and CDR3, having the amino acid sequences shown in (2) of claim 1;
(3) a peptide having the amino acid sequence identity of 80% or higher to the amino acid sequence of SEQ ID NO:5 and having three complementarity-determining regions, CDR1, CDR2, and CDR3, having the amino acid sequences shown in (3) of claim 1;
(4) a peptide having the amino acid sequence identity of 80% or higher to the amino acid sequence of SEQ ID NO:6 and having three complementarity-determining regions, CDR1, CDR2, and CDR3, having the amino acid sequences shown in (4) of claim 1;
(5) a peptide having the amino acid sequence identity of 80% or higher to the amino acid sequence of SEQ ID NO:7 and having three complementarity-determining regions, CDR1, CDR2, and CDR3, having the amino acid sequences shown in (3) of claim 1; and
(6) a peptide having the amino acid sequence identity of 80% or higher to the amino acid sequence of SEQ ID NO:55 and having three complementarity-determining regions, CDR1, CDR2, and CDR3, having the amino acid sequences shown in (5) of claim 1.

3. The human transferrin receptor affinity peptide according to claim 2, wherein the amino acid sequence identity is 85% or higher.

4. The human transferrin receptor affinity peptide according to claim 2, wherein the amino acid sequence identity is 90% or higher.

5. The human transferrin receptor affinity peptide according to claim 2, wherein the amino acid sequence identity is 95% or higher.

6. The human transferrin receptor affinity peptide according to claim 1, wherein the human transferrin receptor affinity peptide contains the amino acid sequence selected from the group consisting of the following (1) to (6):
(1) the amino acid sequence in which 1 to 10 amino acids are substituted, deleted, or added relative to the amino acid sequence of SEQ ID NO:3, and the amino acid sequence of CDR1 is the amino acid sequence of SEQ ID NO:8 or SEQ ID NO:9, the amino acid sequence of CDR2 is the amino acid sequence of SEQ ID NO: 10 or SEQ ID NO: 11, and the amino acid sequence of CDR3 is the amino acid sequence of SEQ ID NO: 12 or SEQ ID NO: 13;
(2) the amino acid sequence in which 1 to 10 amino acids are substituted, deleted, or added relative to the amino acid sequence of SEQ ID NO:4, and the amino acid sequence of CDR1 is the amino acid sequence of SEQ ID NO: 14 or SEQ ID NO: 15, the amino acid sequence of CDR2 is the amino acid sequence of SEQ ID NO: 16 or SEQ ID NO: 17, and the amino acid sequence of CDR3 is the amino acid sequence of SEQ ID NO: 18 or SEQ ID NO: 19;
(3) the amino acid sequence in which 1 to 10 amino acids are substituted, deleted, or added relative to the amino acid sequence of SEQ ID NO:5, and the amino acid sequence of CDR1 is the amino acid sequence of SEQ ID NO:20 or SEQ ID NO:21, the amino acid sequence of CDR2 is the amino acid sequence of SEQ ID NO:22 or SEQ ID NO:23, and the amino acid sequence of CDR3 is the amino acid sequence of SEQ ID NO:24 or SEQ ID NO:25;
(4) the amino acid sequence in which 1 to 10 amino acids are substituted, deleted, or added relative to the amino acid sequence of SEQ ID NO:6, and the amino acid sequence of CDR1 is the amino acid sequence of SEQ ID NO:26 or SEQ ID NO:27, the amino acid sequence of CDR2 is the amino acid sequence of SEQ ID NO:28 or SEQ ID NO:29, and the amino acid sequence of CDR3 is the amino acid sequence of SEQ ID NO:30 or SEQ ID NO:31;
(5) the amino acid sequence in which 1 to 10 amino acids are substituted, deleted, or added relative to the amino acid sequence of SEQ ID NO:7, and the amino acid sequence of CDR1 is the amino acid sequence of SEQ ID NO:20 or SEQ ID NO:21, the amino acid sequence of CDR2 is the amino acid sequence of SEQ ID NO:22 or SEQ ID NO:23, and the amino acid sequence of CDR3 is the amino acid sequence of SEQ ID NO:24 or SEQ ID NO:25; and
(6) the amino acid sequence in which 1 to 10 amino acids are substituted, deleted, or added relative to the amino acid sequence of SEQ ID NO:55, and the amino acid sequence of CDR1 is the amino acid sequence of SEQ ID NO:20 or SEQ ID NO:21, the amino acid sequence of CDR2 is the amino acid sequence of SEQ ID NO:55 or SEQ ID NO:56, and the amino acid sequence of CDR3 is the amino acid sequence of SEQ ID NO:24 or SEQ ID NO:25.

7. The human transferrin receptor affinity peptide according to claim 5, wherein the number of substituted, deleted, or added amino acids is 1 to 5.

8. The human transferrin receptor affinity peptide according to claim 5, wherein the number of substituted, deleted, or added amino acids is 1 to 3.

9. The human transferrin receptor affinity peptide according to any one of claims 1 to 8, wherein the human transferrin receptor affinity peptide has affinity for both an extracellular region of human transferrin receptor and an extracellular region of monkey transferrin receptor.

10. The human transferrin receptor affinity peptide according to claim 9, wherein a dissociation constant with the extracellular region of human transferrin receptor is 5×10⁻⁹ to 1×10⁻⁷.

11. The human transferrin receptor affinity peptide according to claim 9, wherein a dissociation constant with the extracellular region of human transferrin receptor is 8×10⁻⁹ to 2×10⁻⁸.

12. The human transferrin receptor affinity peptide according to claim 9, wherein when the value of the dissociation constant with the extracellular region of human transferrin receptor is taken as 1, the value of a dissociation constant with monkey transferrin receptor is 0.5 to 2.5.

13. The human transferrin receptor affinity peptide according to claim 9, wherein when the value of the dissociation constant with the extracellular region of human transferrin receptor is taken as 1, the value of the dissociation constant with the monkey transferrin receptor is 0.7 to 1.2.

14. A heavy chain antibody variable region peptide in which a plurality of the human transferrin receptor affinity peptide according to any one of claims 1 to 13 are linked, the heavy chain antibody variable region peptide being selected from the group consisting of the following (1) to (3):
(1) a heavy chain antibody variable region peptide in which 2 to 10 of the human transferrin receptor affinity peptide according to claims 1 to 13 are linked directly or via a linker;
(2) a heavy chain antibody variable region peptide in which 2 or 3 of the human transferrin receptor affinity peptide according to claims 1 to 13 are linked directly or via a linker; and
(3) a heavy chain antibody variable region peptide in which two of the human transferrin receptor affinity peptide according to claims 1 to 13 are linked directly or via a linker.

15. The human transferrin receptor affinity peptide according to claim 14, wherein the linker for linking a plurality of the human transferrin receptor affinity peptide is a peptide linker consisting of 1 to 50 amino acids.

16. The human transferrin receptor affinity peptide according to claim 14, wherein the amino acid sequence of the peptide linker for linking a plurality of the human transferrin receptor affinity peptide is selected from the group consisting of a single amino acid, the amino acid sequence Gly-Ser, the amino acid sequence Ser-Ser, the amino acid sequence Gly-Gly-Ser, the amino acid sequence Gly-Gly-Gly, and the amino acid sequences of SEQ ID NO:32, SEQ ID NO:33, SEQ ID NO:34, SEQ ID NO:35, SEQ ID NO:36, SEQ ID NO:59, and SEQ ID NO:69.

17. The human transferrin receptor affinity peptide according to claim 14, wherein the amino acid sequence of the peptide linker for linking the heavy chain antibody variable region peptides is the amino acid sequence in which 2 to 10 of the amino acid sequences selected from the group consisting of a single amino acid, the amino acid sequence Gly-Ser, the amino acid sequence Ser-Ser, the amino acid sequence Gly-Gly-Ser, the amino acid sequence Gly-Gly-Gly, and SEQ ID NO:32, SEQ ID NO:33, SEQ ID NO:34, SEQ ID NO:59, and SEQ ID NO:69, are linked in tandem.

18. A human transferrin receptor affinity peptide-drug complex, wherein the human transferrin receptor affinity peptide according to any one of claims 1 to 17 is linked to a drug.

19. The complex according to claim 18, wherein the drug is any one of a different protein (A), a nucleic acid, or a low molecular weight compound.

20. A fusion protein comprising:
the human transferrin receptor affinity peptide according to any one of claims 1 to 17 and
a different protein (A).

21. The fusion protein according to claim 20, wherein the human transferrin receptor affinity peptide is bound to the C-terminus of the different protein (A) directly or via a linker.

22. The fusion protein according to claim 20, wherein the human transferrin receptor affinity peptide is bound to the N-terminus of the different protein (A) directly or via a linker.

23. The fusion protein according to claim 21 or 22, wherein the linker is a peptide linker consisting of 1 to 50 amino acids.

24. The fusion protein according to claim 23, wherein the amino acid sequence of the peptide linker is selected from the group consisting of a single amino acid, the amino acid sequence Gly-Ser, the amino acid sequence Ser-Ser, the amino acid sequence Gly-Gly-Ser, the amino acid sequence Gly-Gly-Gly, and the amino acid sequences of SEQ ID NO:32, SEQ ID NO:33, SEQ ID NO:34, SEQ ID NO:35, SEQ ID NO:36, SEQ ID NO:59, and SEQ ID NO:69.

25. The fusion protein according to claim 23, wherein the amino acid sequence of the peptide linker is the amino acid sequence in which 2 to 10 of the amino acid sequences selected from the group consisting of a single amino acid, the amino acid sequence Gly-Ser, the amino acid sequence Ser-Ser, the amino acid sequence Gly-Gly-Ser, the amino acid sequence Gly-Gly-Gly, each of the amino acid sequences of SEQ ID NO:32, SEQ ID NO:33, and SEQ ID NO:34, and the amino acid sequences of SEQ ID NO:59 and SEQ ID NO:69, are linked in tandem.

26. The fusion protein according to any one of claims 20 to 25, wherein the different protein (A) is a protein originating from human.

27. The fusion protein according to any one of claims 20 to 26, wherein the different protein (A) is a cytokine, a growth factor, or an antibody medicine.

28. The fusion protein according to any one of claims 20 to 26, wherein the different protein (A) is selected from the group consisting of a brain-derived neurotrophic factor (BDNF), a nerve growth factor (NGF), a lysosomal enzyme, a ciliary neurotrophic factor (CNTF), a glial cell-derived neurotrophic factor (GDNF), a neurotrophin 3, a neurotrophin 4/5, a neurotrophin 6, a neuregulin 1, an erythropoietin, a darbepoietin, an activin, a basic fibroblast growth factor (bFGF), a fibroblast growth factor 2 (FGF2), an epidermal growth factor (EGF), a vascular endothelial growth factor (VEGF), an interferon α, an interferon β, an interferon γ, an interleukin 6, a granulocyte-macrophage colony-stimulating factor (GM-CSF), a granulocyte-colony-stimulating factor (G-CSF), a macrophage colony-stimulating factor (M-CSF), a tumor necrosis factor-a receptor (TNF-α receptor), a PD-1 ligand, a PD-L1, a PD-L2, an enzyme having an activity of degrading beta-amyloid, an anti-beta-amyloid antibody, an anti-BACE antibody, an anti-EGFR antibody, an anti-PD-1 antibody, an anti-PD-L1 antibody, an anti-PD-L2 antibody, an anti-HER2 antibody, an anti-TNF-α antibody, and an anti-CTLA-4 antibody.

29. The fusion protein according to any one of claims 20 to 26, wherein the different protein (A) is a lysosomal enzyme, and the lysosomal enzyme is selected from the group consisting of α-L-iduronidase, iduronate-2-sulfatase, acidic α-glucosidase, glucocerebrosidase, β-galactosidase, GM2 activator protein, β-hexosaminidase A, β-hexosaminidase B, N-acetylglucosamine-1-phosphotransferase, α-mannosidase, β-mannosidase, galactosylceramidase, saposin C, allylsulfatase A, α-L-fucosidase, aspartylglucosaminidase, α-N-acetylgalactosaminidase, acidic sphingomyelinase, α-galactosidase A, β-glucuronidase, heparan-N-sulfatase, α-N-acetylglucosaminidase, acetyl CoA α-glucosaminide N-acetyltransferase, N-acetylglucosamine-6-sulfate sulfatase, acidic ceramidase, amylo-1,6-glucosidase, sialidase, palmitoyl protein thioesterase-1, tripeptidyl peptidase-1, hyaluronidase-1, CLN1, and CLN2.

30. The fusion protein according to any one of claims 20 to 29, wherein serum albumin is bound to the fusion protein.

31. The fusion protein according to any one of claims 20 to 29, wherein a human IgG Fc region or a portion thereof is bound to the fusion protein.

32. A nucleic acid encoding the human transferrin receptor affinity peptide according to any one of claims 1 to 17.

33. A nucleic acid encoding the fusion protein according to any one of claims 20 to 31.

34. An expression vector comprising the nucleic acid according to claim 32 or 33 incorporated therein.

35. A cell transformed with the expression vector according to claim 34.

36. The cell according to claim 35, wherein the cell is derived from a mammal.
